(19) Europäisches Patentamt
European Patent Office
Office européen des brevets

(11) Numéro de publication: **0 122 818**
**B1**

(12) # FASCICULE DE BREVET EUROPEEN

(45) Date de publication du fascicule du brevet: **07.10.87**

(21) Numéro de dépôt: **84400343.4**

(22) Date de dépôt: **20.02.84**

(51) Int. Cl.$^4$: **C 07 K 1/02,** A 61 K 37/02

(54) **Synthèse du hpGRF (Somatocrinine) en phase liquide et peptides intermédiaires.**

(30) Priorité: **21.02.83 FR 8302781**
**29.11.83 FR 8319058**

(43) Date de publication de la demande:
**24.10.84 Bulletin 84/43**

(45) Mention de la délivrance du brevet:
**07.10.87 Bulletin 87/41**

(84) Etats contractants désignés:
**AT BE CH DE FR GB IT LI LU NL SE**

(56) Documents cités:
**CHEMICAL ABSTRACTS, vol. 98, no. 13, 28 mars 1983, page 102, no. 101517c, Columbus, Ohio, US; R. GUILLEMIN et al.: "Growth hormone-releasing factor from a human pancreatic tumor that caused acromegaly"**
**NATURE, vol. 300, no. 5889, 18 novembre 1982, pages 276-278, Macmillan Journals Ltd., Basingstoke, GB; J. RIVIER et al.: "Characterization of a growth hormone-releasing factor from a human pancreatic islet tumour"**

(73) Titulaire: **SANOFI, société anonyme**
**40, Avenue George V**
**F-75008 Paris (FR)**

(72) Inventeur: **Diaz, J.**
**19, rue du Pradas**
**F-34470 Perols (FR)**
Inventeur: **Demarne, Henri**
**Avenue Major Flandre Le Florence**
**F-34000 Montpellier (FR)**
Inventeur: **Roncucci, Roméo**
**20, rue Tournefort**
**F-75005 Paris (FR)**
Inventeur: **Schmelck, Paul-Henry**
**117, rue de Courcelles**
**F-75017 Paris (FR)**

(74) Mandataire: **Gillard, Marie-Louise et al**
**Cabinet Beau de Loménie 55, Rue d'Amsterdam**
**F-75008 Paris (FR)**

(56) Documents cités:
**CHEMICAL AND PHARMACEUTICAL ABSTRACTS BULLETIN, vol. 31, no. 5, mai 1983, pages 1800-1802, Tokyo, JP; H. YAJIMA et al.: ""Solution synthesis of a tetratetracontapeptide amide with growth hormone releasing activity"**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 122 818 B1

**Description**

Le hpGRF (Human Pancreatic Growth Hormone releasing Factor) ou Somatocrinine est un peptide constitué par l'enchaînement de 44 amino acides. Sa séquence est la suivante:

1 5 10
H-Tyr-Ala-Asp-Ala-Ile-Phe-Thr-Asn-Ser-Tyr-Arg-

15 20
Lys-Val-Leu-Gly-Gln-Leu-Ser-Ala-Arg-Lys-Leu-Leu-

25 30 35
Gln-Asp-Ile-Met-Ser-Arg-Gln-Gln-Gly-Glu-Ser-Asn-

40 44
Gln-Glu-Arg-Gly-Ala-Arg-Ala-Arg-Leu-$NH_2$

Il a été récemment découvert par A. Guillemin et coll. (Science, *218*, 585—587 (1982)) à partir d'extraits d'une tumeur pancréatique humaine.

Ce peptide est particulièrement actif sur la stimulation de la libération de l'hormone de croissance (GH) aussi bien in vitro qu'in vivo. In vitro, en particulier son efficacité se manifeste à des doses de quelques femtomoles/ml. ($ED_{50}$=15 femtomoles/ml). L'intérêt thérapeutique en médecine humaine de cette substance va donc se situer au niveau du traitement du nanisme et des retards à la croissance en pédiatrie. D'autres applications sont possibles dans les cas de déficience anabolique protéique (ulcères de stress, réparation de fractures ou d'atteintes du cartilage, brûlures étendues (pendant la phase anabolique), réparations cutanées, ostéoporoses.

Dans le domaine vétérinaire, ce composé présente un intérêt évident au niveau de la croissance pondérale des animaux d'élevage (bovins, ovins, porcins, poulets, etc.) et de l'augmentation de la lactation (bovins, ovins, etc.).

Le développement industriel de ce composé polypeptidique nécessite la synthèse de quantités importantes de cette substance. Les procédés classiques de la synthèse en phase solide permettent la préparation en des temps courts de faibles quantités de ce principe actif (Science, *218*, 585—587 (1982)) à des coûts très élevés et incompatibles avec un développement pharmaceutique à grande échelle.

On a maintenant trouvé un procédé de synthèse en phase liquide pouvant être transposé à l'échelle industrielle permettant un accès au principe actif avec un rendement et un taux de pureté excellents. Ce procédé est basé sur le principe d'une synthèse par fragments.

Le procédé de la présente invention est caractérisé en outre en ce que l'on couple, l'un après l'autre et dans l'ordre de la séquence, les fragments dans lesquels:

a) les fonctions acides latérales des acides aspartique et glutamique et la fonction amine latérale de la lysine sont protégées par des groupes protecteurs stables dans les conditions de déprotection du groupement Boc (tertiobutoxycarbonyle),

b) la fonction guanidine de l'arginine est protégée par protonation, et

c) l'acide aminé N-terminal est protégé sur l'amine par le groupement Boc en éliminant sélectivement le groupement Boc de l'amine N-terminale du peptide en phase d'élongation par hydrolyse à l'acide trifluoroacétique, ledit couplage étant effectué dans un solvant polaire aprotique, et on élimine en fin de séquence tous les groupes protecteurs par hydrolyse à l'aide d'une solution 0,1 à 1 M d'acide méthanesulfonique ou trifluorométhanesulfonique dans l'acide trifluoroacétique.

Le procédé de la présente invention peut aussi s'appliquer à la synthèse du hpGRF 1—40, produit naturel isolé également par R. Guillemin, presque aussi actif que le hpGRF 1—44 et utilisable dans les mêmes indications thérapeutiques.

Le procédé de synthèse du GRF se caractérise en outre par les aspects suivants:

— application du principe de la protection minimale au niveau des chaînes latérales fonctionnalisées
— protection temporaire de la fonction guanidine latérale de l'arginine par le groupement nitro. L'arginine est introduite en séquence sous forme nitro guanidine. La fonction nitro est ensuite éliminée le plus tôt possible par hydrogénation catalytique à l'aide de Pd/charbon, ou bien en utilisant à la place de l'hydrogène gaz un générateur d'hydrogène comme l'acide formique ou le formiate d'ammonium. Ainsi, tous les fragments synthétisés possédant de l'arginine dans leur séquence ont en fin de synthèse les fonctions guanidine, simplement protégées par protonation à l'aide d'un acide fort (acide chlorhydrique par exemple)
— les fonctions acides carboxyliques des chaînes latérales des acides glutamique et aspartique sont protégées par des groupements clivables par hydrogénation catalytique ($H_2$/Pd/charbon) ou bien en milieu acide fort comme les mélanges acide méthanesulfonique (0,5 M)—acide trifluoroacétique, ou acide trifluorométhanesulfonique (0,5 M)—acide trifluoroacétique. Nous préconisons comme protecteurs l'ester benzylique (0 Bzl) ou 2,6-dichlorobenzylique. Ces groupements protecteurs sont

stables dans les conditions des déprotections intermittentes des amines en α (élimination des groupements t-butyloxy-carbonyle:Boc par l'acide trifluoroacétique)

— les fonctions amines des chaînes latérales des lysines sont protégées par des groupements clivables dans les mêmes conditions que précédemment. Nous préconisons les groupments benzyloxycarbonyle (Z), 2-chloro ou 2-bromo-benzyloxycarbonyle (2-Cl ou 2-Br Z) stables dans les conditions de déprotection intermittente par l'acide trifluoroacétique.

Les fonctions hydroxyle présentes dans la thréonine, la sérine et la tyrosine ne sont pas protégées.

L'élongation du peptide à partir des fragments synthétisés est réalisée en utilisant comme agent de couplage l'hexafluorophosphate de benzotriazolyl-oxyphosphonium (BOP), ou la dicyclohexylcarbodiimide en présence d'hydroxy-1 benzotriazole, ou selon la méthode aux carboxyazides (Curtius) dans un solvant approprié comme le diméthylformamide ou le diméthylsulfoxyde. L'isolement du produit du milieu réactionnel est effectué par introduction d'un tiers solvant insolubilisant (éther, acétate d'éthyle . . . ) qui précipite le peptide.

On se limite pendant les étapes de couplage à des purifications sommaires du type lavages en phase solide à l'aide de solvants appropriés afin d'éliminer le léger excès du dernier fragment couplé, ainsi que les impuretés apportées par les agents de couplage.

Chaque opération de couplage d'un fragment est suivie d'une phase de déprotection intermittente du groupement protecteur Boc (tertio-butyloxycarbonyle) au niveau de l'amine sur laquelle va être effectué le couplage suivant. Cette déprotection est assurée par l'acide trifluoroacétique dans le chlorure de méthylène (50/50 en volume).

Les déprotections des chaînes latérales en fin d'élongation du peptide peuvent être effectuées par hydrogénation en présence d'un catalyseur (comme le Pd/C) à l'aide d'hydrogène gazeux sous légère pression (1 à 5 kg) ou bien avec un générateur d'hydrogène comme l'acide formique ou le formiate d'ammonium. Il est également possible d'éliminer ce type de groupements protecteurs par un acide fort comme les mélanges d'acide méthanesulfonique dans l'acide trifluoroacétique (0,5 M) ou bien d'acide trifluorométhanesulfonique dans l'acide trifluoroacétique (0,5 M).

Le produit, après les déprotections terminales, est purifié par filtration sur gel de Sephadex G 50 à l'aide de l'acide acétique à 30%. Les fractions enrichies en GRF 1—44 sont réunies et soumises à une chromatographie sur échangeurs d'ions (cations) du type carboxy et à l'aide d'un gradient à force ionique croissante adapté au type de résine échangeuse d'ions utilisée. Les fractions de plus haute pureté sont réunies et purifiées soit par chromatographie de partition sur un support approprié du type Sephadex G 50 ou Biogel P 10, soit par la distribution à contre-courant.

Les fractions dont le titre de pureté est jugé satisfaisant par HPLC analytique sont réunies. Les autres sont recyclées.

Une variante du procédé consiste à remplacer la chromatographie de partition ou la distribution à contre-courant par la HPLC préparative.

Les fragments utilisés pour la synthèse de hpGRF 1—44 sont déterminés sur la base de considérations chimiques en tenant compte en particulier des risques minimaux de racémisation pendant les phases de couplage des fragments.

Chacun des fragments est alors synthétisé selon une stratégie adaptée à chaque cas selon un procédé "Step Wise" en phase liquide.

Le schéma I suivant représente l'une des stratégies de synthèse utilisable pour la synthèse de hpGRF 1—44.

Les flèches verticales délimitent les fragments utilisés dans la synthèse et chacun d'eux est désigné par une lettre:

| | |
|---|---|
| A: | fragment 40—44 |
| B: | fragment 33—39 |
| C: | fragment 28—32 |
| D: | fragment 25—27 |
| E: | fragment 21—24 |
| F: | fragment 16—20 |
| G: | fragment 12—15 |
| H: | fragment 5—11 |
| I: | fragment 1—4 |

En outre, les protections des chaînes latérales ont été symbolisées par:
$X_1$=ester de type benzyle (O Bzl)
$X_2$=protection carbamate du type benzyloxycarbonyle (Z)
Un trouvera ensuite dans les tableaux I à IX le procédé de synthèse utilisé pour chacun des fragments A à I.

G R F—Strategie de synthese

Schema I

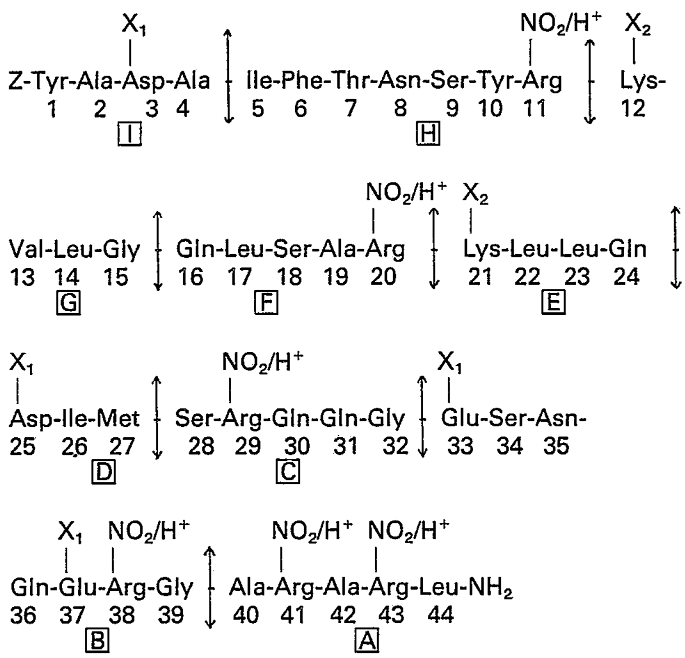

TABLEAU I (fragment A)

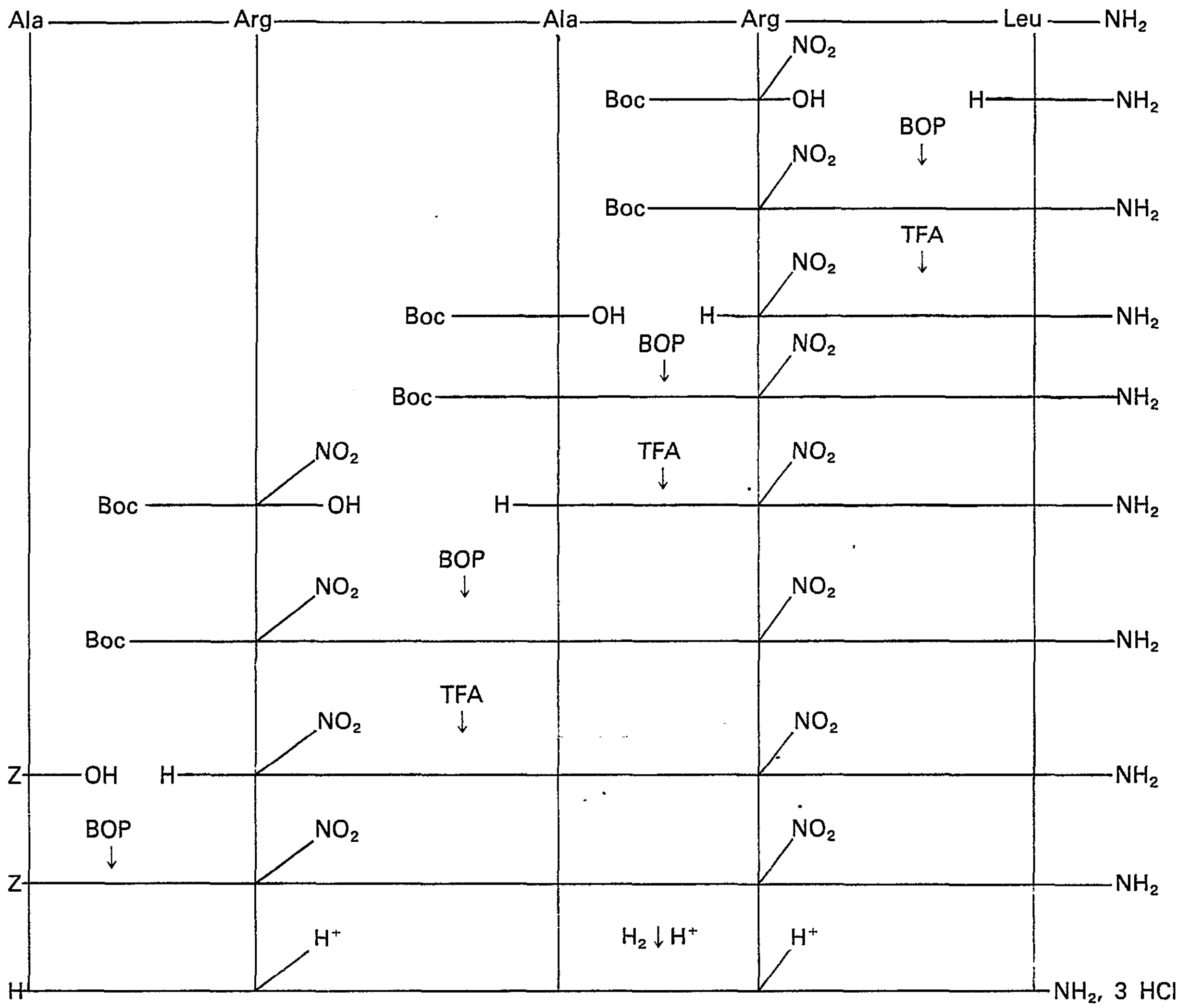

TABLEAU II (fragment B)

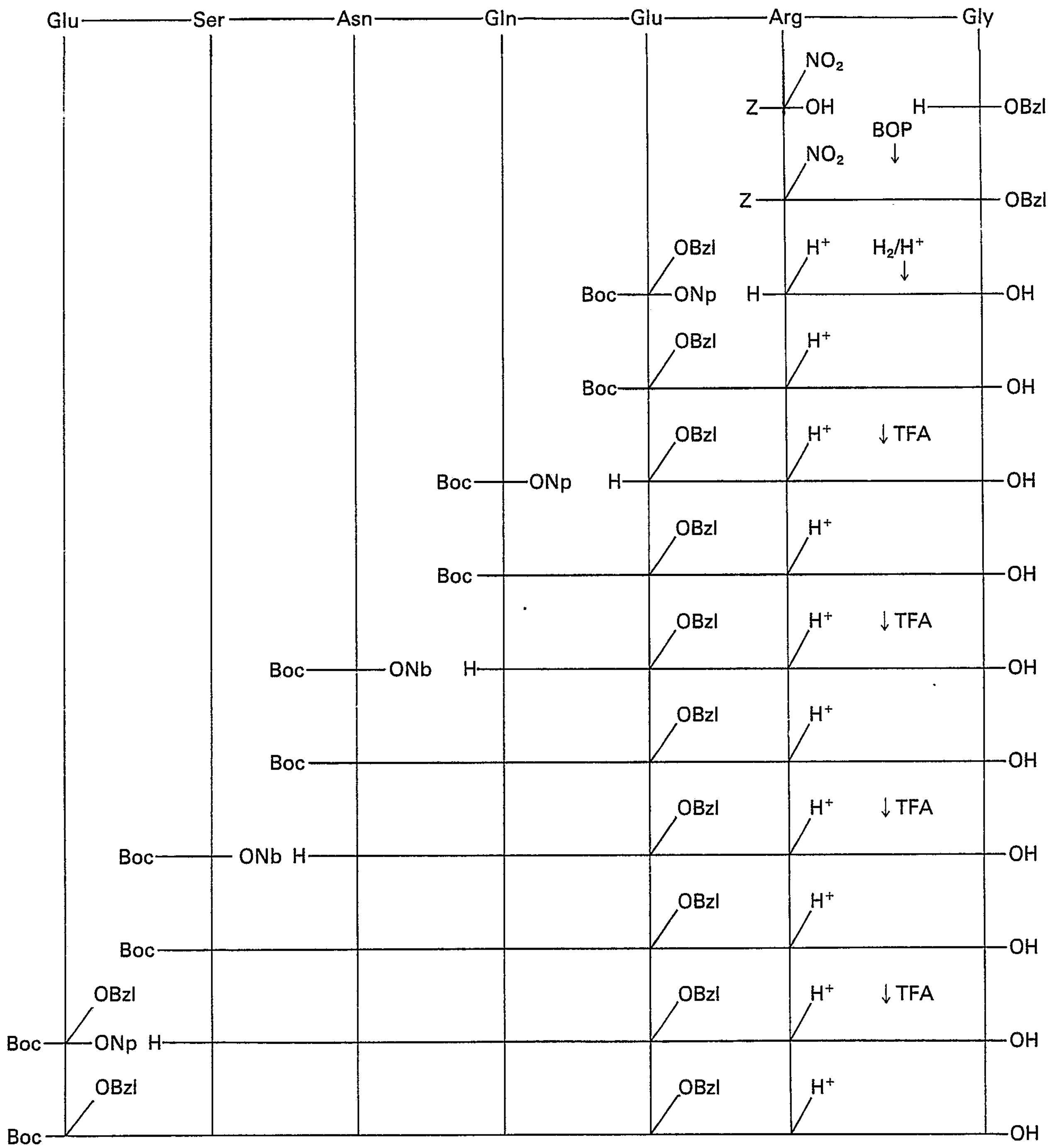

TABLEAU III (fragment C)

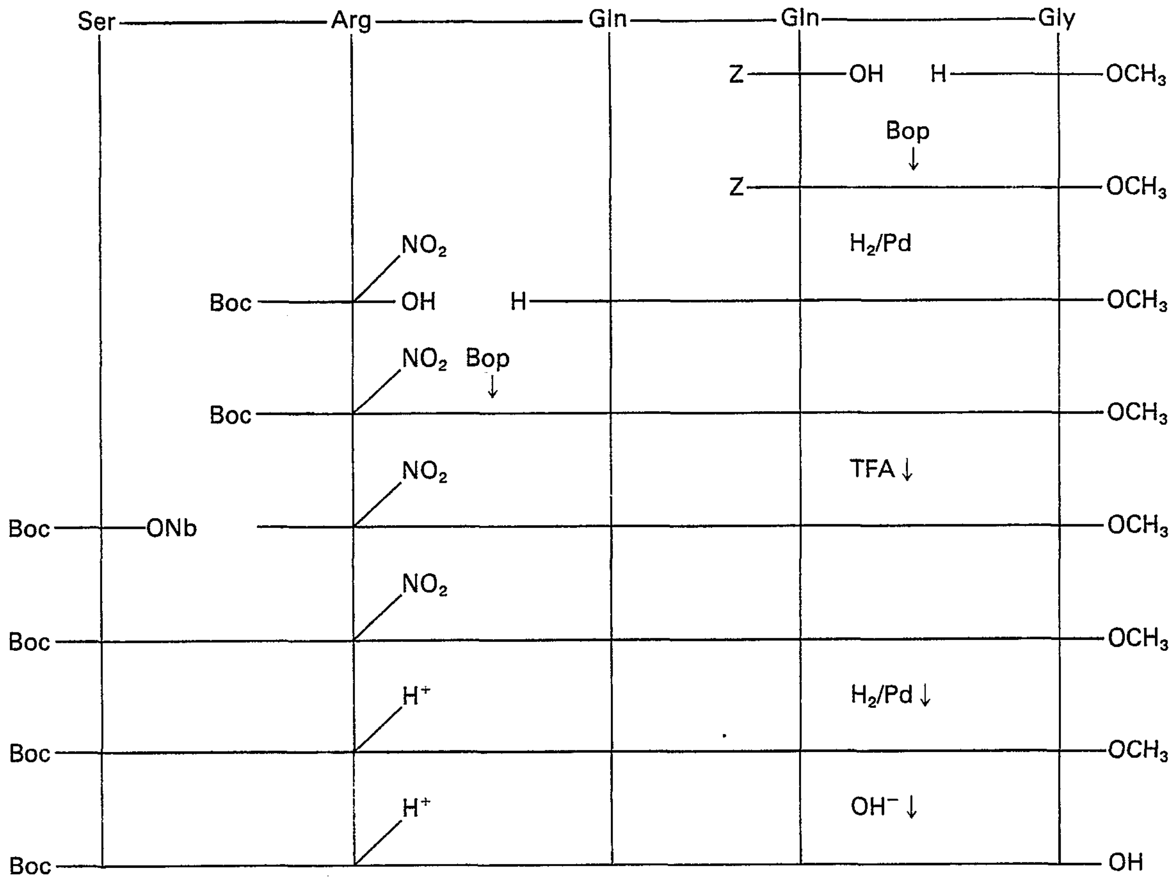


TABLEAU IV (fragment D)

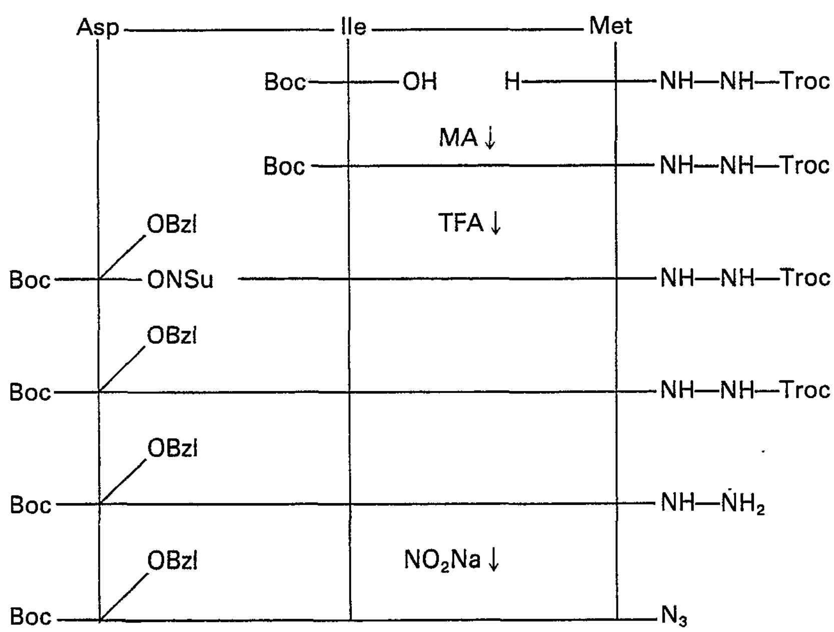

TABLEAU V (fragment E)

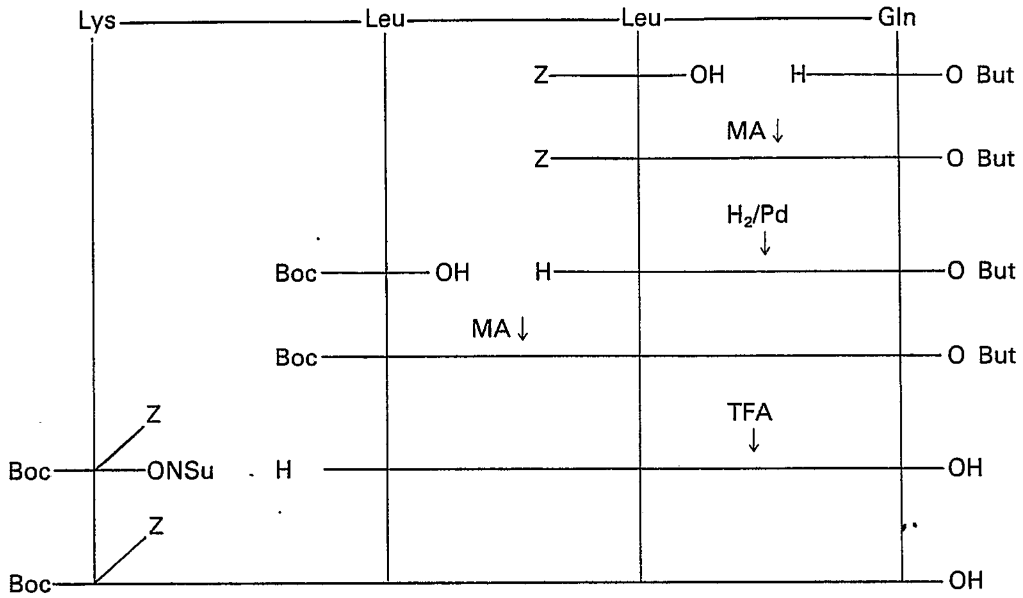


TABLEAU VI (fragment F)

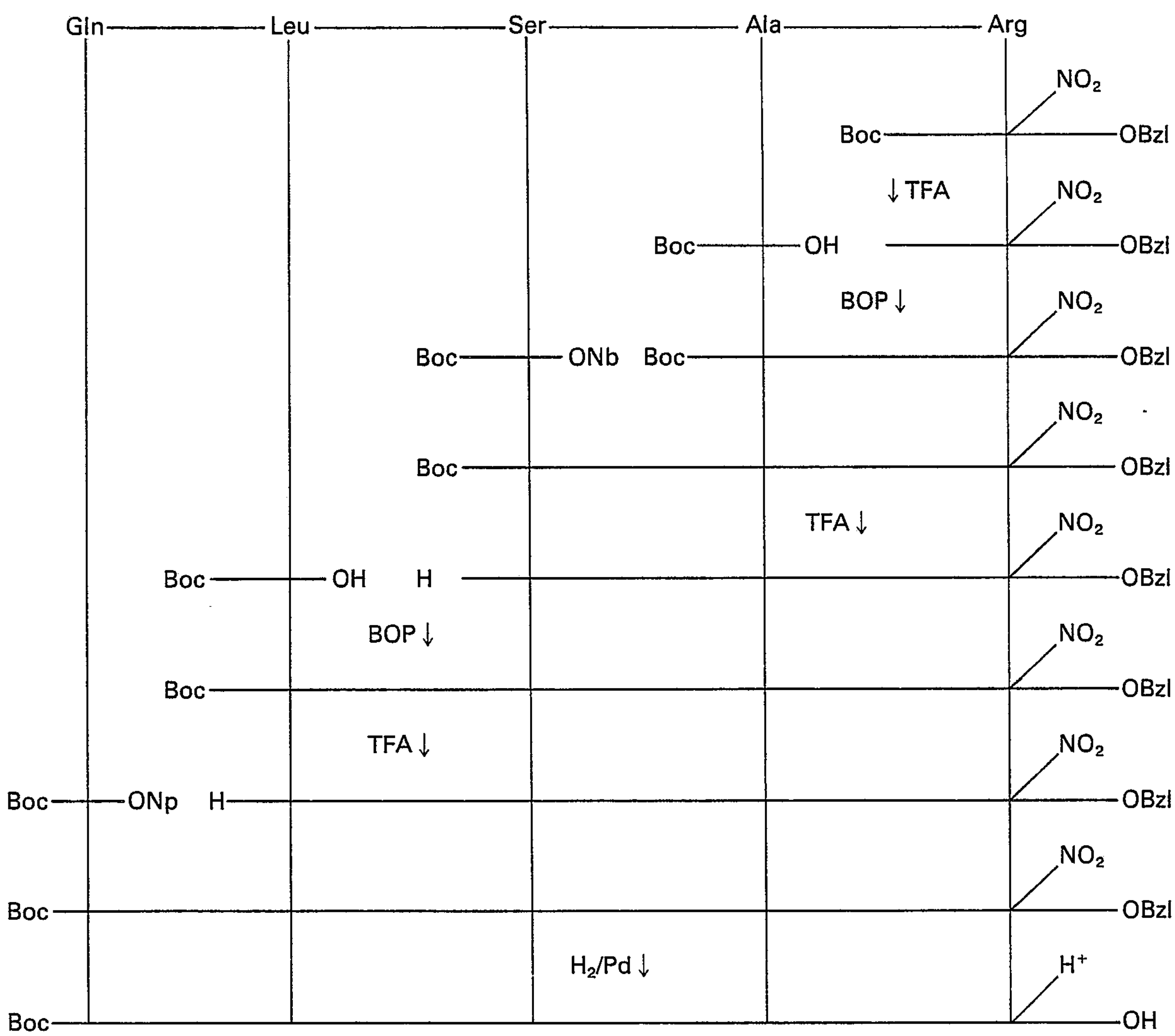

TABLEAU VII (fragment G)

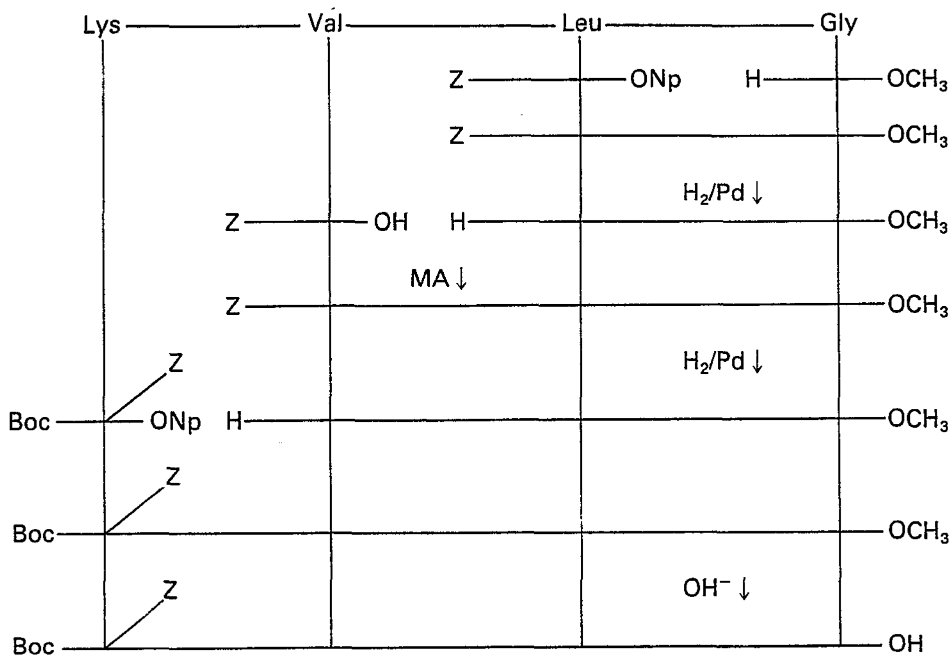

TABLEAU VIII (fragment H)

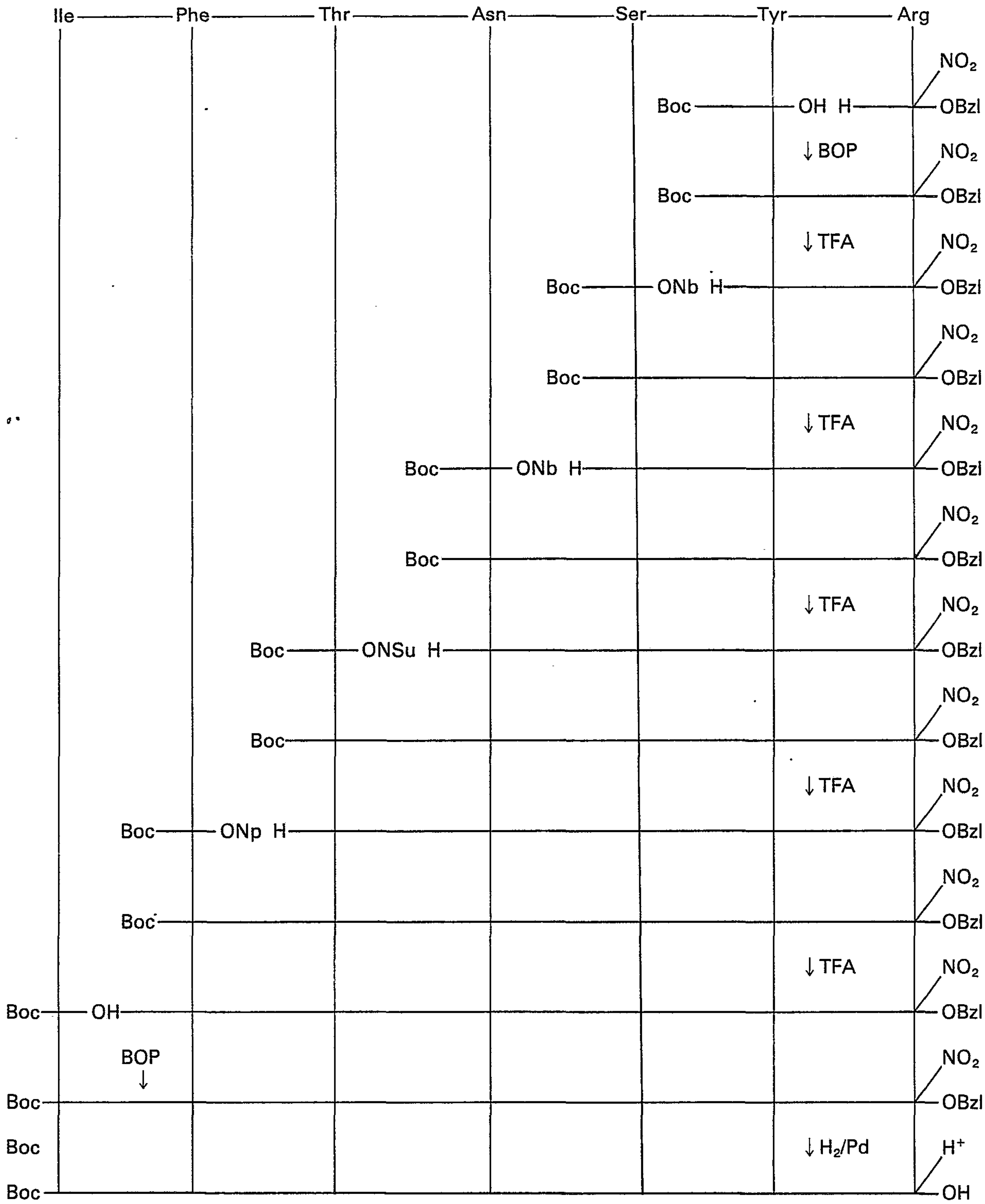

TABLEAU IX (fragment I)

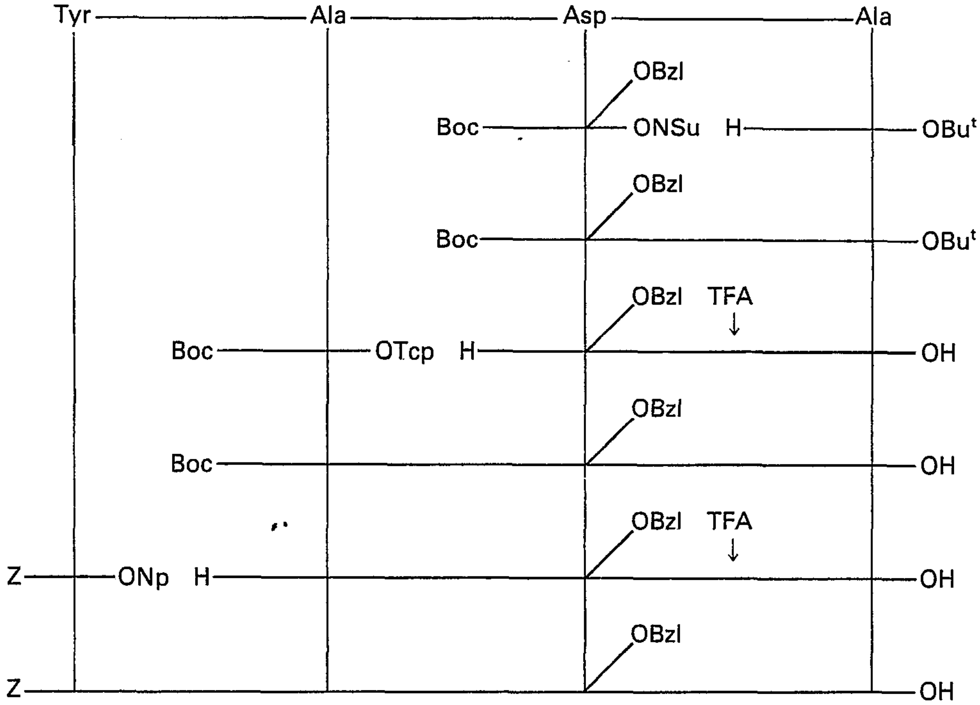


On peut également préparer le hpGRF 1—44 selon un procédé faisant intervenir les fragments:

| | |
|---|---|
| 20—44 | Peptide K |
| 5—19 | Peptide J |
| 1—4 | Peptide I |

que l'on couple dans l'ordre:

| | |
|---|---|
| 5—19+20—44 | 5—44 |
| 1—4 + 5—44 | 1—44 |

Le peptide j et le peptide K sont à leur tour synthétisés par un procédé par fragments.

Le peptide 5—19 est synthétisé à partir de 3 fragments et le peptide 20—44 à partir de 7 fragments.

Les schémas II et III ci-après représentent les stratégies utilisées pour préparer les peptides J et K, le peptide I ayant déjà été préparé dans le schéma I.

Le schéma IV représente le couplage des 3 peptides I, J et K pour aboutir au hpGRF 1—44.

Les tableaux X à XV représentent les schémas de synthèse des fragments non décrits en suivant le schéma I.

Enfin, dans tous les cas, pour obtenir hpGRF 1—40, il suffit de réduire le fragment A à l'alaninamide.

Synthese du fragment 20—44 (25 aa): peptide K

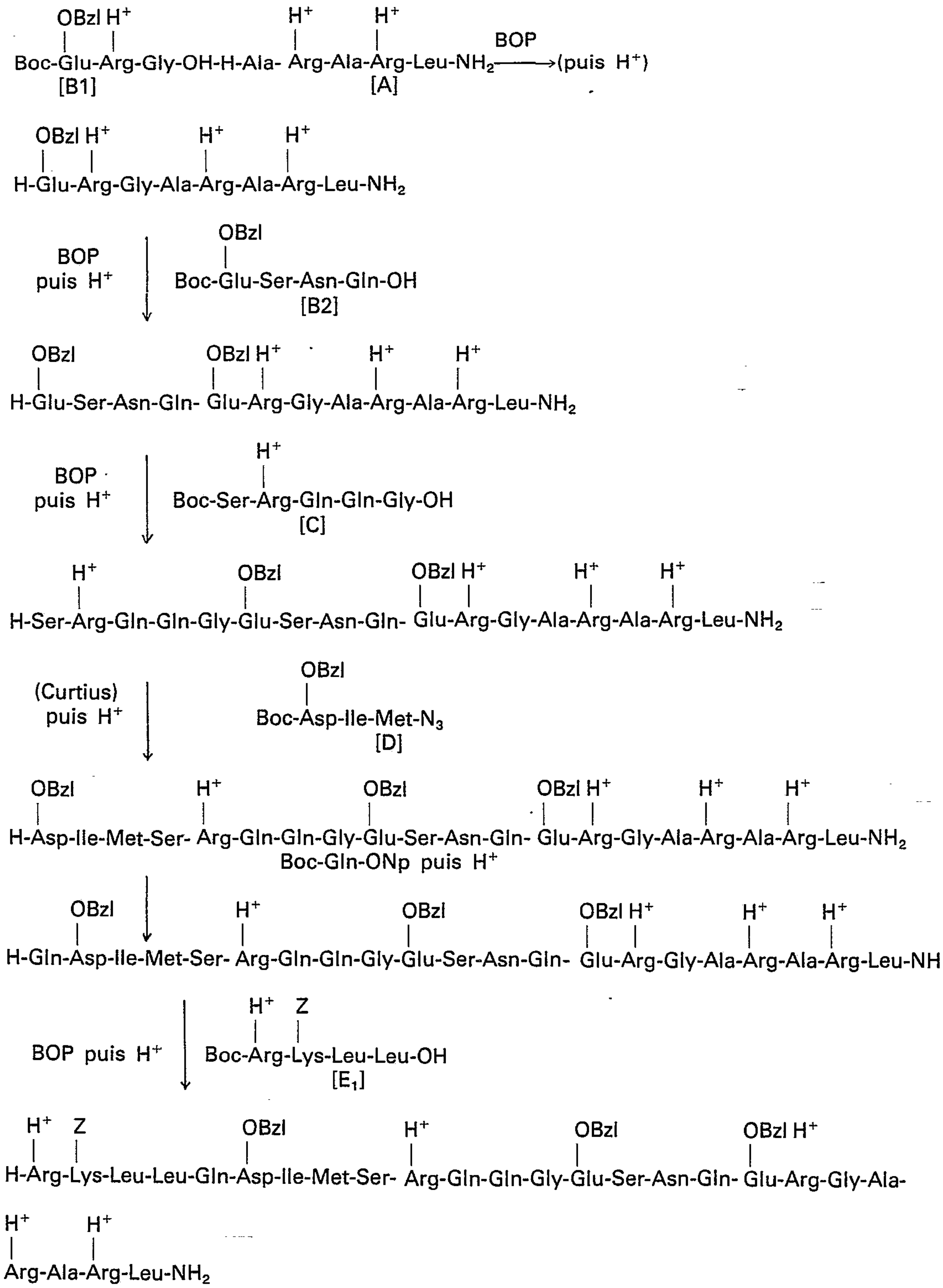


Schéma II

Synthese du fragment 5—19 (15 acides α aminés): peptide J

```
        H⁺  Z
        |   |
Boc-Tyr-Arg-Lys-Val-Leu-Gly-OH+ H-Gln-Leu-Ser-Ala-OCH3
           [G1]  |                      [F1]
      BOP puis H⁺|
                 ↓
    H⁺  Z
    |   |
H-Tyr-Arg-Lys-Val-Leu-Gly-Gln-Leu-Ser-Ala-OCH3

    Curtius      |     Boc-Ile-Phe-Thr-Asn-Ser-NH-NH2
    puis OH⁻     |
                 |             [H1]
                 ↓
                            H⁺  Z
                            |   |
Boc-Ile-Phe-Thr-Asn-Ser-Tyr-Arg-Lys-Val-Leu-Gly-Gln-Leu-Ser-Ala-OH
```

Schéma III

Passage a la sequence du GRF 1—44

```
                                     Peptide J+peptide K
                                             |
          BOP                                ↓
          puis H⁺  H⁺  Z                                  H⁺  Z          Bzl
                   |   |                                  |   |          |
H-Ile-Phe-Thr-Asn-Ser-Tyr-Arg-Lys-Val-Leu-Gly-Gln-Leu-Ser-Ala-Arg-Lys-Leu-Leu-Gln-Asp-Ile-

        H⁺              Bzl             Bzl H⁺          H⁺     H⁺
        |               |               |   |           |      |
Met-Ser-Arg-Gln-Gln-Gly-Glu-Ser-Asn-Gln-Glu-Arg-Gly-Ala-Arg-Ala-Arg-Leu-NH2

                                    |               OBzl
                                    |               |
          BOP puis H⁺ fort          ↓    Z-Tyr-Ala-Asp-Ala-OH
                                GRF-1—44     (peptide I)
```

Schéma IV

TABLEAU X—(sous-fragment $B_1$)

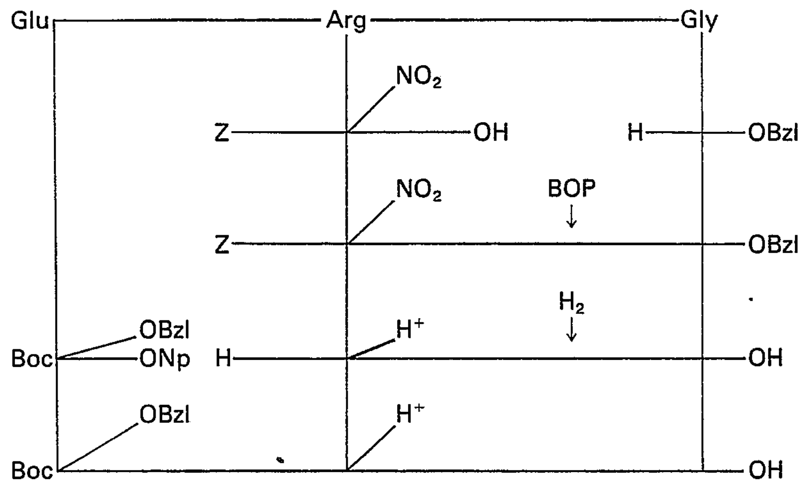


TABLEAU XI—(sous fragment $B_2$)

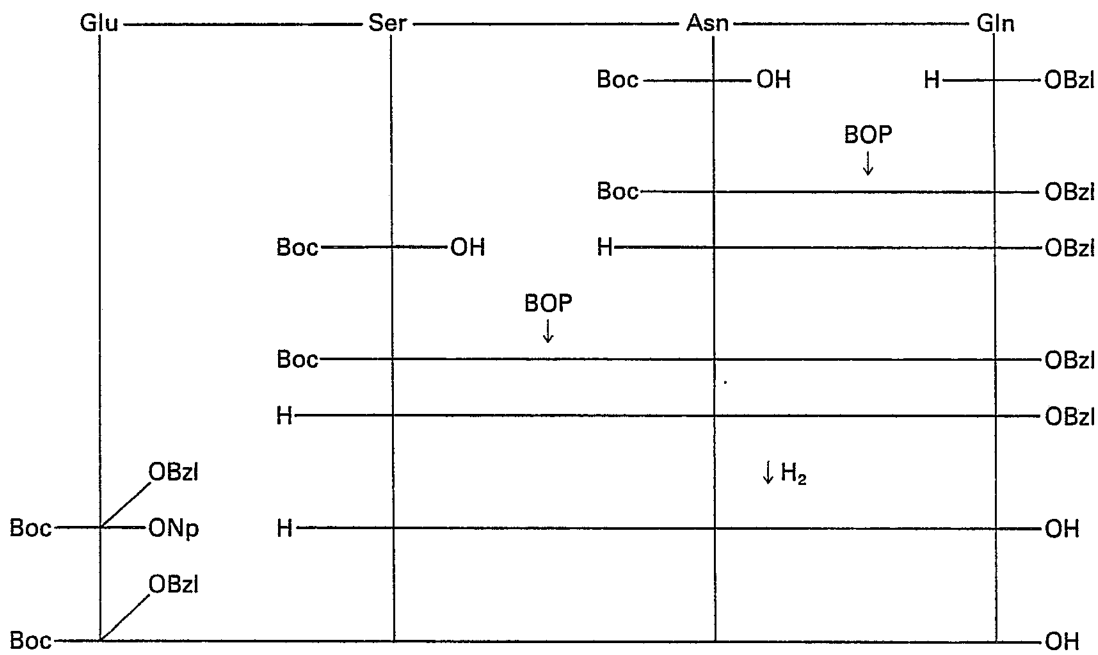

TABLEAU XII—(fragment $E_1$)

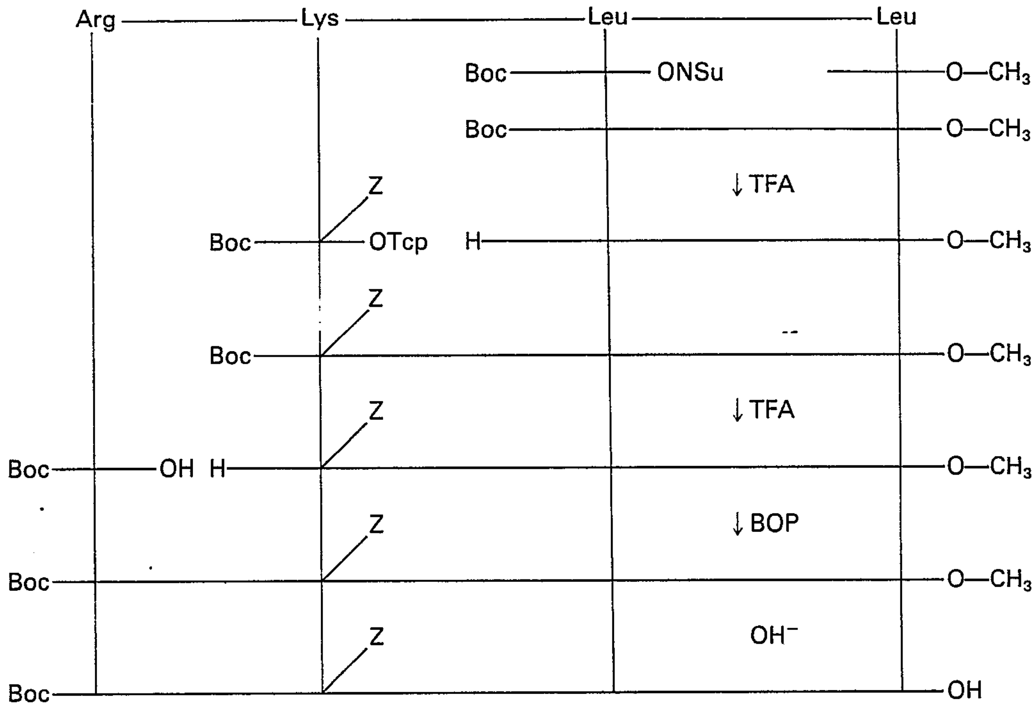


TABLEAU XIII—(fragment $F_1$)

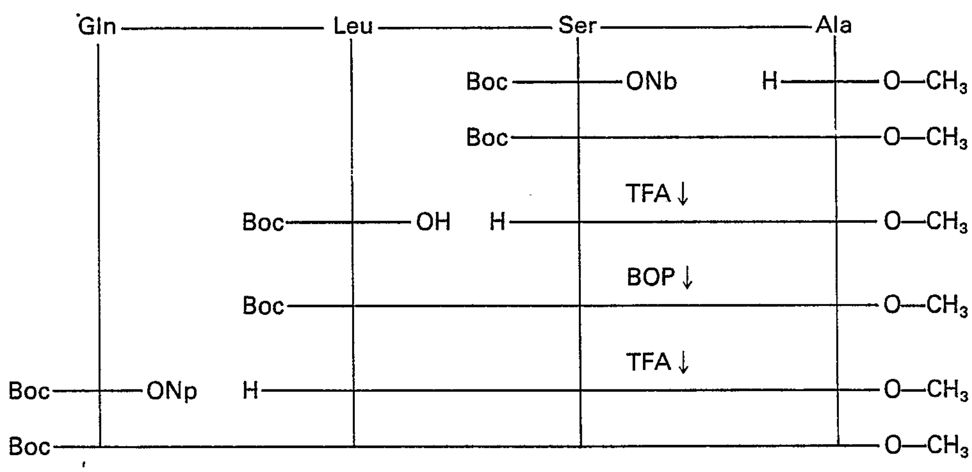

TABLEAU XIV—(fragment $G_1$)

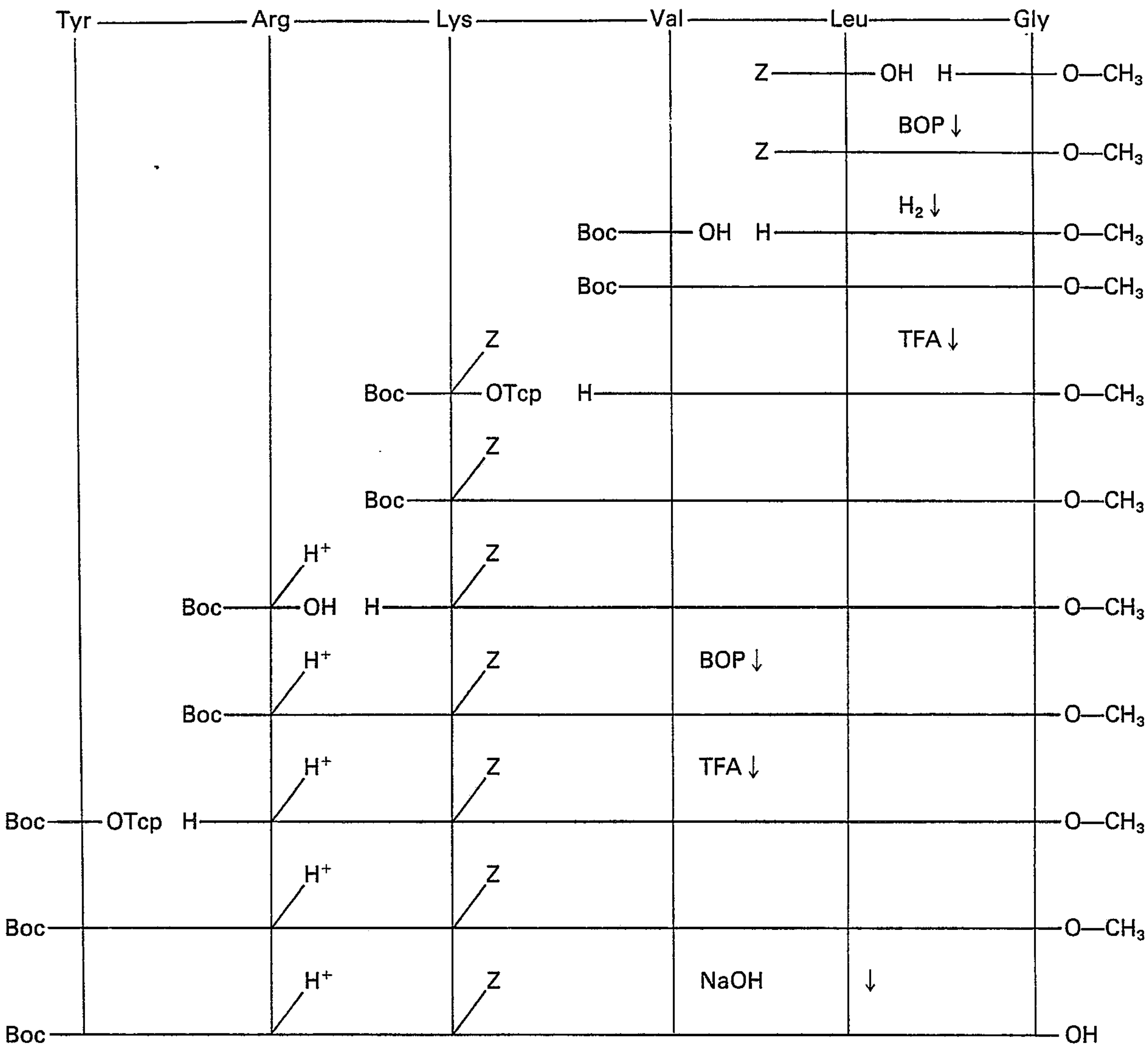

TABLEAU XV—(fragment $H_1$)

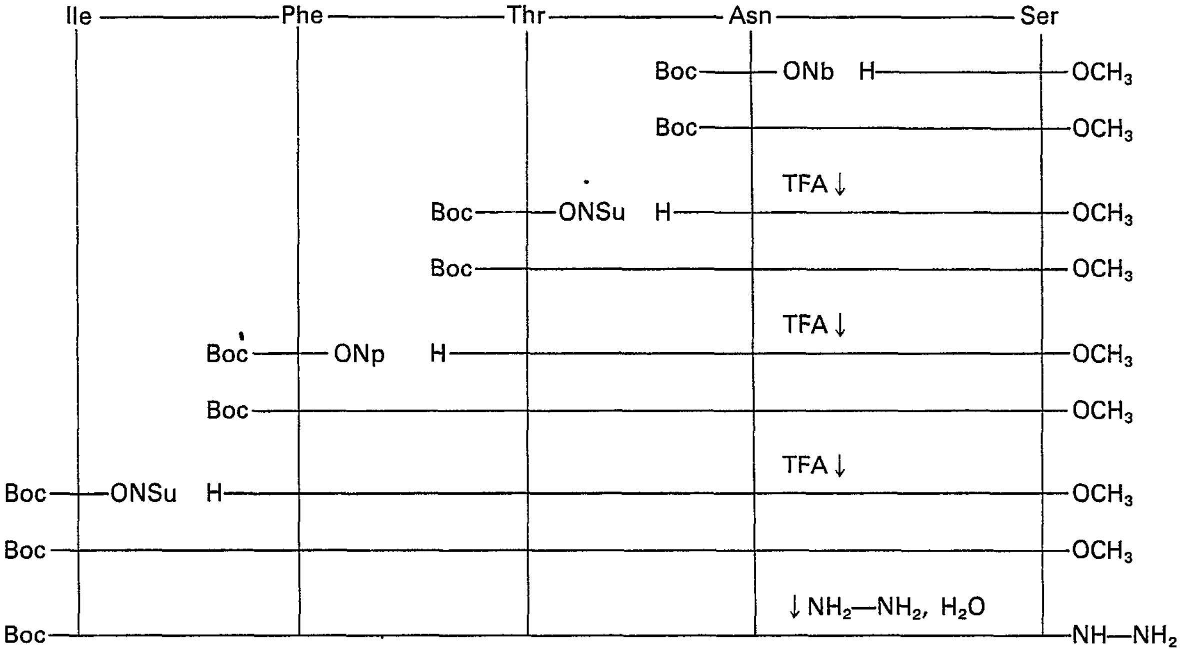


Les exemples suivants permettront de mieux comprendre la portée de l'invention.

Les abréviations suivantes seront utilisées.

Les acides aminés sont représentés par les symboles recommandés par la commission de nomenclature de l'IUPAC-IUB section biochimie.

Ala: Alanine
Arg: Arginine
Asn: Asparagine
Asp: Acide aspartique
Gln: Glutamine
Glu: Acide glutamique
Gly: Glycine
Ile: Isoleucine
Leu: Leucine
Lys: Lysine
Met: Méthionine
Phe: Phénylalanine
Ser: Sérine
Thr: Thréonine
Tyr: Tyrosine
Val: Valine

A l'exception de la glycine, ils ont tous la configuration L.

HPLC: chromatographie liquide haute performance

CCM: chromatographie sur couches minces

OBzl: ester benzylique 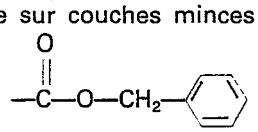

Z: benzyloxycarbonyle (carbamate) 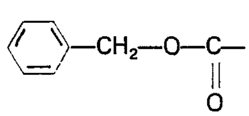

Boc: tertiobutyloxycarbonyl (carbamate) 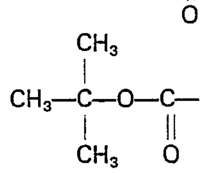

DMF: diméthylformamide
NEM: N-éthylmorpholine
TFA: acide trifluoroacétique
MA: méthode de couplage aux anhydrides mixtes

ONSu: ester activé avec la N-hydroxysuccinimide 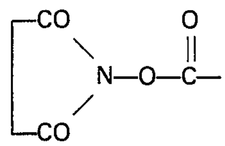

ONp: ester activé avec l'ortho nitrophénol 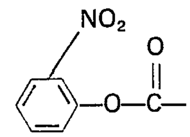

Troc: trichloroéthoxycarbonyle (carbamate) 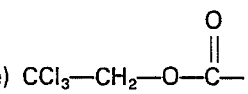

OBu$^t$: ester avec le tertiobutanol 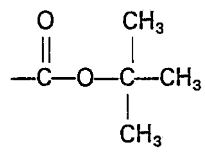

OTcp: ester activé avec le 2,3,5-trichlorophénol
OHBT: N-hydroxybenzotriazole

ONb: ester activé avec le N-hydroxy norbornène-5 dicarboximide-2,3 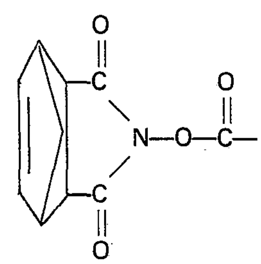

BOP: hexafluorophosphate de benzotriazolyloxyphosphonium 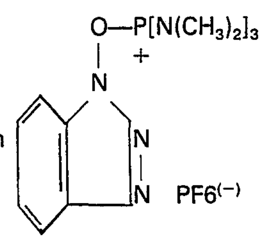

DCHa: dicyclohexylamine
DCU: dicyclohexylurée
DCC ou DCCI: dicyclohexylcarbodiimide
TA: température ambiante
DIPEA: diisopropyléthylamine
EPP: pureté polypeptidique
TFMSA: acide trifluorométhane sulfonique
AAA: analyse d'amino acides
Milieux de chromatographie exprimés en volumes:
BEW$_1$: butanol, AcOH, $H_2O$ 72/7/21
BEW$_2$: butanol, AcOH, $H_2O$ 67/10/23
BPEW$_1$: butanol, pyridine, AcOH, $H_2O$ 50/12/12/25
EPAW: AcOEt, pyridine, $HCO_2H$, $H_2O$ 63/21/10/6
BPEW$_2$: butanol, pyridine, AcOH, $H_2O$ 42/24/4/30

Exemple I

Synthèse de H-Ala-Arg-Ala-Arg-Leu-$NH_2$ (fragment A)

$NO_2$

|

1. H-Arg-Leu-$NH_2$

Dissoudre à la température ambiante 130 g de leucine amide (H-Leu-$NH_2$) dans 1,5 l de DMF. Ajouter 333 g de Boc-Arg ($NO_2$)—OH puis 500 g de BOP. Ajuster le pH à 7 au papier pH (sur de petits prélèvements dilués à l'eau) et à l'aide de N-éthylmorpholine (NEM). Le milieu est agité et on suit l'évolution de la réaction par CCM. La réaction est terminée au bout de 4 h. Le milieu est évaporé à sec sous vide à 25°C. Le résidu est repris par 1 litre d'eau et on obtient un solide qui est lavé à l'eau, puis avec une solution aqueuse de $NaHCO_3$ à 5% à l'eau, à l'acétate d'éthyle et, finalement, on sèche le solide à l'air. On contrôle par CCM.

Le solide précédent est introduit dans 2 l d'un mélange 50—50 en volume d'acide trifluoroacétique-chlorure de méthylène. Le milieu est agité 10 min à la température ambiante et évaporé à sec sous vide à la température ambiante. Le résidu d'évaporation est repris dans l'éther, essoré, séché et contrôlé par CCM et RMN. Rendement: 339 g (90%) en trifluoroacétate d'un solide blanc.

2. H-Ala-Arg($NO_2$)Leu-$NH_2$

443 g de trifluoroacétate de H-Arg($NO_2$)Leu-$NH_2$ sont dissous dans 2 l de DMF. On ajoute 200 g de Boc-Ala-OH et ensuite 500 g de BOP. On ajuste le pH à 7 au papier pH (sur de petits prélèvements du milieu réactionnel) à l'aide de NEM. Le milieu est agité et l'évolution de la réaction est suivie par CCM. La réaction est terminée au bout de 4 h. Le milieu est évaporé à sec sous vide à 25°C. Le résidu est repris par 2 l d'eau et 2 l d'acétate d'éthyle. La phase organique est lavée par une solution aqueuse de $NaHCO_3$ à 5%, à l'eau, séchée et évaporée. Le tripeptide est recristallisé dans l'acétate d'éthyléther, et finalement séché sous vide. Il est contrôlé par CCM et RMN.

Le précédent produit séché est traité par un mélange 50—50 (volume) de TFA-$CH_2Cl_2$ dans les conditions de l'exemple précédent (I-1). L'isolement est effectué également dans les mêmes conditions.

Rendement: 412 g (80%) exprimé en trifluoroacétate d'un produit pulvérulent blanc (contrôlé en CCM et RMN).

3. Arg($NO_2$)-Ala-Arg($NO_2$)-Leu-$NH_2$

A partir de 515 g de H-Ala-Arg($NO_2$)Leu-$NH_2$ dans 2,5 l de DMF et 333 g de Boc-Arg($NO_2$)-OH et 500 g de BOP, on obtient en utilisant les conditions opératoires décrites dans l'exemple I,1, après traitement au mélange TFA-$CH_2Cl_2$, 607 g (85%) d'un solide blanc contrôlé par CCM et RMN.

4. Z-Ala-Arg($NO_2$)-Ala-Arg-Leu-$NH_2$

A partir de 715 g de trifluoroacétate de H-Arg($NO_2$)Ala-Arg($NO_2$)-Leu-$NH_2$ en solution dans 4 l de DMF et 233 g de Z-Ala-OH et 500 g de BOP en utilisant la même technique que celle décrite en I-2 (et sans traitement dans ce cas par TFA-$CH_2Cl_2$), on obtient après recristallisation dans le mélange DMF-éther, 612 g de Z-Ala-Arg($NO_2$)-Ala-Arg($NO_2$)Leu-$NH_2$ (76%) sous forme d'un solide pulvérulent blanc, contrôlé par CCM et RMN.

5. Ala-Arg-Ala-Arg-Leu-$NH_2$, 3HCl

200 g de Z-Ala-Arg($NO_2$)Ala-Arg($NO_2$)-Leu-$NH_2$ (0,25 mole) sont mis en suspension dans 2 l de méthanol contenant 0,8 mole de HCl. On ajoute 40 g de Pd/C à 10% de Pd, et le milieu est agité sous atmosphère d'hydrogène sous 1,2 bar de pression, pendant 24 h. Après cet intervalle de temps, on contrôle la fin de la réaction par CCM. Le catalyseur est éliminé par filtration et le solvant évaporé sous vide à la température ambiante.

Le résidu solide est purifié par chromatographie sur gel de silice en utilisant comme milieu d'élution le mélange butanol, pyridine, $HO_2CCH_3$,$OH_2$ (50-12-12-25 en volume).

Les fractions contenant le produit pur sont réunies, évaporées et lyophilisées.

Rendement: 119 g (69%) d'un solide pulvérulent blanc.

Contrôles: RMN, CCM.

Analyse d'aminoacides: Leu 1,03 (1)
Arg 1,92 (2)
Ala 1,95 (2).

Exemple II: synthèse de Boc-Glu(OBzl)-Arg-Gly-OH, HCl (fragment $B_1$)

1°) Z-Arg($NO_2$)-Gly-OBzl

On dissout 3,37 g (0,01 mole) de tosylate de H-Gly-OBzl dans 15 ml de DMF, puis on ajoute 1 équivalent de NEM (1,3 ml). Cette solution est ajoutée à une solution de 3,53 g (0,01 mole) de Z-Arg($NO_2$)-OH dans 15 ml de DMF contenant un équivalent de NEM (1,3 ml). On ajoute alors 4,5 g (0,01 mole) de BOP au mélange réactionnel dont le pH est ensuite amené à 7 par addition de NEM et qui est agité à TA pendant 2 h 30 (la fin de la réaction est déterminée par CCM:chloroforme-méthanol (3/1). Le mélange réactionnel est évaporé à sec sous pression réduite (0,1 mm de mercure) à température inférieure à 30°C. Le résidu est repris dans 100 ml d'acétate d'éthyle. Cette solution est versée dans 120 ml d'eau glacée viroureusement agitée. Après

quelques instants d'agitation, un précipité se forme qui est abandonné une nuit au réfrigérateur. Le solide est essoré et lavé successivement à l'état solide avec:—

2×100 ml d'une solution aqueuse de bicarbonate de soude
2×100 ml d'une solution aqueuse de $SO_4HK$—$SO_4K_2$ à 5%
2×100 ml d'eau
2×100 ml d'éther

puis séché sous vide (0,1 mm de mercure) jusqu'à poids constant.

Rendement: 4 g (80%).
Point de fusion Koffler: 149°C.
CCM: chloroforme-méthanol (3/1)
Rf: 0,73
Contrôle: RMN.

2°) HCl-H-Arg-Gly-OH

On met en suspension 100 g (0,2 mole) de Z-Arg($NO_2$)-Gly-OBzl dans un mélange de 600 ml d'eau, 600 ml d'acide chlorhydrique N et 100 ml de tétrahydrofuranne. On ajoute 60 g de Pd/C à 10% contenant 50% d'humidité. Ce mélange est hydrogéné pendant 48 h à température ambiante et sous une pression de 15 mm de mercure. Le catalyseur est ensuite filtré et la solution aqueuse est amenée à pH 6,5 par addition de résine Amberlite IR 45 (forme OH). La résine est essorée. La solution est ensuite amenée à pH 8,5 par addition d'une nouvelle quantité de résine et l'ensemble est agité pendant 20 min au rotavapor sous vide (25 mm de mercure). On essore la résine et on vérifie l'absence de chlorure d'ammonium dans la solution aqueuse par le test de Nesler, puis on évapore à sec cette solution sous pression réduite (0,1 mm de mercure) à température inférieure à 30°C. On obtient un résidue gommeux qui est séché une nuit dans un dessiccateur avec de l'anhydride phosphorique.

Rendement: 43,19 g (80,6%).
CCM: acétate d'éthyle-pyridine-acide formique-eau 40-21-10-6.
Rf: 0,1 test de SaKaguchi positif (spot rouge).
Contrôle RMN.

3°) Boc-Glu(OBzl)-Arg(HCl)-Gly-OH

On dissout 41,5 g de HCl-H-Arg-Gly-OH (0,155 mole) dans un mélange de 400 ml de DMF et de 130 ml d'eau. Le pH est ajusté à 7 par addition d'acide chlohydrique N. A cette solution, on ajoute simultanément:

— une solution de 68,8 g de Boc-Glu(OBzl)-ONP (0,150 ml) dans un mélange de 100 ml de DMF et de 40 ml d'eau
— une solution de 22,7 g d'hydroxybenzothiazole (0,150 mole) dans un mélange de 100 ml de DMF et de 40 ml d'eau
— une solution de 37,8 ml de NEM (2×0,150 mole) dans 100 ml de DMF.

Le mélange réactionnel est agité à TA et la réaction est suivie en CCM (chloroforme-méthanol-acide acétique 95-5-9 et pyridine-acétate d'éthyle-acide formique-eau 21-40-10-6).

Au bout de 1 h de réaction, on rajoute 13,76 g de Boc-Glu(OBzl)-ONP.
Au bout de 2 h de réaction, on rajoute 13,76 g de Boc-Glu(OBzl)-ONP.
Au bout de 3 h de réaction, on rajoute 7 g de Boc-Glu(OBzl)-ONP et ou poursuit la réaction pendant 1 h.

Le mélange réactionnel est alors évaporé à sec sous pression réduite (0,1 mm de mercure) et à température inférieure à 30°C. On obtient une huile épaisse qui est dissoute dans 500 ml d'acétate d'éthyle. Par addition de 1 litre d'éther, une huile épaisse se forme. On abandonne au repos 24 h au réfrigérateur, puis on décante la phase liquide. L'huile résiduelle est reprise dans 500 ml de méthanol. A cette solution on ajoute 500 g de silice (70—230 mesh) et on évapore le solvant au rotavapor. La poudre obtenue est mélangée avec un mélange chloroforme-méthanol 80—20 et le gel formé est introduit au sommet d'une colonne de gel de silice (hauteur 200 cm, diamètre 85 mm) montée dans le mélange chloroforme-méthanol 80—20. Le produit est élué avec:

— mélange de chloroforme-méthanol 80—20: 25 litres
— mélange de chloroforme-méthanol (50—50): 10 litres
— méthanol: 30 litres.

La purification est suivie par CCM (pyridine-acétate d'éthyle-acide formique-eau 21-40-10-6). On recueille 2 fractions:

— fraction A 29,80 g.
CCM: pyridine-acétate d'éthyle-acide formique-eau 21-40-10-6
Rf: 0,75
Contrôle: RMN
HPLC-EPP: 96,98%
AAA: Glu 1,01-Gly 1,01-Arg 0,98

— fraction B 36,82 g
CCM: pyridine-acétate d'éthyle-acide formique-eau 21-40-10-6
Rf: 0,75
1H RMN spectre conforme
Contrôle: HPLC-EPP: 99,61%
AAA: Glu 0,93-Gly 0,99-Arg 1,08.

Exemple III: synthèse de Boc-Glu(OBzl)-Ser-Asn-Gln-OH (fragment $B_2$)

1°) Boc-Asn-Gln-OBzl

On dissout TFA, H-Gln-OBzl (0,445 mole) dans 500 ml de DMF. A cette solution, on ajoute successivement:

— 103,3 g de Boc-Asn-OH (0,445 mole)
— 62,3 ml de NEM
— 217,8 g de BOP (0,5 mole)

puis le pH de la solution est ramené à 7 par addition de NEM. Le mélange est agité à TA, on maintient le pH à 7 par addition de NEM si nécessaire. La réaction est suivie en CCM (chloroforme-méthanol 3-1). Au bout de 1 h, la réaction est terminée et on fait précipiter le produit par addition de 1,5 l d'acétate d'éthyle. On laisse 1 h sous agitation puis on abandonne une nuit au réfrigérateur. Le solide est essoré, lavé à l'acétate d'éthyle (3×500 ml), à l'éther (1 litre), puis séche.
Rendement: 108,13 g (53,9%)
CCM: chloroforme-méthanol 3-1
Rf: 0,63.

2°) TFA, H-Asn-Gln-OBzl

On dissout 102 g de Boc-Asn-Gln-OBzl (0,226 mole) dans 500 ml de TFA refroidis dans un bain de glace. On enlève le bain de glace et on poursuit l'agitation à TA pendant 15 min. On essore un léger insoluble et on poursuit l'agitation pendant 15 min. Le milieu réactionnel est concentré au 1,4 sous pression réduite (25 mm de mercure) et on reprend dans 300 ml d'éther. Cette opération est répétée 2 fois (2×300 ml d'éther) et le solvant restant éliminé sous vide 25 mm de mercure). On obtient un solide blanc hydroscopique.
Rendement: 107 g
CCM: chloroforme-méthanol 3-1
Rf: 0,08.

3°) Boc-Ser-Asn-Gln-OBzl

On dissout 0,226 mole de TFA, H-Asn-Gln-OBzl dans 1,5 l de DMF. A cette solution on ajoute successivement:

— 48,4 g de Boc-Ser-OH, 1/2 $H_2O$ (0,226 mole)
— 34,8 ml de NEM (0,27 mole)
— 111 g de BOP (0,25 mole)

puis le pH de la solution est ramené à 7 par addition de NEM. Le mélange est agité à TA en maintenant le pH à 7 par addition de NEM si nécessaire. L'évolution de la réaction est suivie en CCM (chloroforme-méthanol-acide acétique 9-2-0,5). Au bout de 2 h, la réaction est terminée. On filtre un léger insoluble et le milieu réactionnel est évaporé à sec sous pression réduite (0,1 mm de mercure) à température inférieure ou égale à 30°C. L'huile résiduelle est reprise dans 800 ml d'acétate d'éthyle. On laisse 1 h sous agitation puis on abandonne une nuit au repos au réfrigérateur. Le solide est essoré, lavé à l'acétate d'éthyle (2×200 ml) puis à l'éther (200 ml) et séché sous vide.
Rendement: 108,32 g (89%)
CCM: chloroforme-méthanol-acide acétique 9-2-0,5
Rf: 0,42.

4°) TFA, H-Ser-Asn-Gln-OBzl

On dissout 108 g de Boc-Ser-Asn-Gln-OBzl (0,2 mole) dans 520 ml de TFA refroidis dans un bain de glace. On enlève le bain de glace et on agite 30 min à TA. On filtre un léger insoluble, puis on évapore à sec sous pression réduite à température inférieure à 30°C. Le résidu est repris dans l'éther, le solide essoré, lavé plusieurs fois à l'éther et séché.
Rendement: 139 g
CCM: tétrahydrofuranne-pyridine-acide formique-eau 60-20-10-6
Rf: 0,81.

5°) TFA, H-Ser-Asn-Gln-OH

On dissout 0,2 mole de TFA, H-Ser-Asn-Gln-OBzl dans 1 litre d'eau. On ajoute à la solution 22,4 g de

Pd/C à 10% contenant 50% d'humidité. Le mélange est hydrogéné à TA et sous pression de 15 mm de mercure pendant 24 h sous agitation. On filtre le catalyseur puis on évapore à sec sous pression réduite (0,1 mm de mercure) et à température inférieure à 30°C. Le résidu est mis à sécher une nuit dans un dessiccateur avec de l'anhydride phosphorique.

Rendement: 93,66 g
CCM: tétrahydrofuranne-pyridine-acide formique-eau 60-20-10-6
Rf: 0,45.

6°) Boc-Glu(OBzl)-Ser-Asn-Gln-OH

On dissout 0,2 mole de TFA, H-Ser-Asn-Gln-OH dans un mélange de 100 ml d'eau et de 600 ml de DMF. On amène le pH de la solution à 7 par addition de NEM et ajoute une solution de 87,11 g de Boc-Glu(OBzl)-ONP (0,19 mole) dans 600 ml de DMF puis 29,5 g de HOBT (0,19 mole). Le pH du mélange est ramené à 7 par addition de NEM et on agite à TA. Au bout de 1 h d'agitation on rajoute 2,7 g de Boc-Glu(OBzl)-ONP. La réaction est suivie en CCM (tétrahydrofuranne-pyridine-acide formique-eau 60-20-10-6 et chloroforme-méthanol-acide acétique 95-5-3). Une 1/2 h après la dernière addition, on filtre un léger insoluble, puis on évapore à sec sous pression réduite (0,1 mm de mercure) à température inférieure à 30°C. L'huile obtenue est reprise dans 800 ml d'acétate d'éthyle, un précipité légèrement gélatineux se forme. Il est essoré et lavé à l'acétate d'éthyle (2×400 ml), puis à l'éther et séché.

Rendement: 114,3 g (90%).

Ce produite doit être relavé par de l'acétate d'éthyle (1,2 l) sous agitation pendant 4 h à TA. Le solide est essoré, lavé à l'éther, séché.

Rendement: 95,85 g (71,9%)
CCM: tétrahydrofuranne-pyridine-acide formique-eau 60-20-10-6
Rf: 0,62
: n-butanol-pyridine-acide acétique-eau 50-12-12-25
Rf: 0,46
1H RMN conforme
HPLC-EPP 87,81
AAA: Asn(asp): 1,01-Ser: 0,89-Glu(gln): 2,05
Point de fusion Koffler: 134°C (décomposition).

Exemple IV: synthèse de Boc-Ser-Arg-Gln-Gln-Gly-OH, HCl (fragment C)

1°) Z-Gln-Gly-OMe

Une suspension refroidie sur bain de glace de 75,36 g de HCl, H-Gly-OMe (0,6 M) dans 1 litre de DMF est additionnée de 168,2 g de Z-Gln-OH (0,6 M), de 256,41 g de BOP (0,7 M) et de 165,4 ml de N-éthylmorpholine (1,3 M). Après 20 h de réaction à TA, la solution est concentrée sous pression réduite et le résidu repris avec de l'acétate d'éthyle, La solution organique est lavée par:

— une solution de bicarbonate de sodium
— une solution de chlorure de sodium
— une solution de $KHSO_4/K_2SO_4$
— une solution de chlorure de sodium.

La solution organique est séchée sur $MgSO_4$ et filtrée. Après 20 h de repos, il y a cristallisation dans chacune des solutions. Après filtration, les fractions provenant d'une cristallisation en milieu aqueux sont regroupées et lavées dans AcOEt saturé en eau pour donner après filtration et séchage sous vide 7,78 g de produit (F. 158—159°C). La fraction provenant de la cristallisation dans AcOEt est lavée dans AcOEt saturé en eau pour donner, après filtration et séchage sous vide 61,8 g de produit (F. 150—154°C). La phase aqueuse et la phase organique donnent une cristallisation supplémentaire. Ces deux derniers produits sont regroupés et lavés comme précédemment pour fournir 38,05 g supplémentaires de qualité comparable.

Rendement global: 107,63 g
Contrôles RMN et CCM.

2°) HCl, H-Gln-Gly-OMe

Une solution de 61,8 g de Z-Gln-Gly-OMe (175,9 mM) dans 704 ml de DMF, 880 ml de méthanol est additionnée sous refroidissement par bain de glace de 176 ml d'une solution d'HCl N, puis de 3,1 g de Pd/C à 10%. Après 3 h 30 d'hydrogénation sous surpression de 35 cm de mercure, le catalyseur est filtré sur célite et la solution est concentrée sous pression réduite. Le résidue est utilisé directement dans l'étape suivante.

3°) Boc-Gln-Gln-Gly-OMe

Une solution du résidu obtenu à l'étape précédente (théoriquement 175,9 mM) dans 500 ml de DMF, refroidie sur bain de glace est additionnée de 77,6 g de Boc-Gln-ONp (211,1 mM), 26,4 g d'hydroxybenzotiazole (211,1 mM) et de N-éthylmorpholine jusqu'à obtenir un pH de 7. Après 20 h de

réaction à TA, la solution est prise en masse. Par addition d'acétate d'éthyle, on obtient un précipité qui est essoré, lavé à AcOEt et séché sous vide. On obtient 64,27 g de produit, soit un rendement de 82%.
Contrôles RMN et CCM.

4°) TFA, H-Gln-Gln-Gly-OMe

Une suspension refroidie sur bain de glace de 98,83 g de Boc-Gln-Gln-Gly-OMe (221,8 mM) dans 300 ml de dichlorométhane est additionnée de 400 ml de TFA. Après 30 min de réaction sur bain de glace et 1 h à TA, la solution est concentrée sous pression réduite jusqu'à la moitié de son volume initial et le résidu est versé sur de l'éther en agitation. Après filtration, lavage et séchage sous vide, on obtient 119 g de produit.

5°) Boc-Arg($NO_2$)-Gln-Gln-Gly-OMe

Une solution des 119 g du produit précédent dans 1190 ml de DMF refroidie sur bain de glace est additionnée de 77,91 g de Boc-Arg($NO_2$)-OH (244 mM), de 97,51 g de BOP (266,2 mM) et de N-éthylmorpholine jusqu'à obtention de pH 7. Après 20 h de réaction à TA, la solution est versée sur 8 l d'acétate d'éthyle. Le précipité obtenu est filtré, lavé à AcOEt, puis séché sous vide.
On obtient 153,7 g de produit.
Contrôles RMN et CCM.

6°) TFA, H-Arg($NO_2$)-Gln-Gln-Gly-OMe

Une suspension refroidie sur bain de glace de 153,7 g de Boc-Arg($NO_2$)-Gln-Gln-Gly-OMe (théoriquement 221,8 mM) dans 600 ml de dichlorométhane est additionnée de 750 ml de TFA. Après 1 h à TA, on ajoute encore 250 ml de TFA et, 30 min plus tard, 250 ml de TFA. Après 1 h supplémentaire de réaction, on concentre la solution jusqu'au tiers du volume initial et verse le résidu sur 3 l d'éther en agitation. Le précipité formé est essoré, lavé à l'éther et séché sous vide. On obtient 167 g de produit.

7°) Boc-Ser-Arg($NO_2$)-Gln-Gln-Gly-OMe

Une solution refroidie sur bain de glace de 167 g de TFA, H-Arg($NO_2$)-Gln-Gln-Gly-OMe (théoriquement 221,8 mM) dans 1,5 l de DMF est additionnée de 97,52 g de Boc-Ser-ONb (266,2 mM), 33,31 g d'hydroxybenzotriazole (266,2 mM) et de N-éthylmorpholine jusqu'à obtenir un pH de 7. Après 3 h de réaction à TA, on évapore une partie du DMF et l'on verse la solution résiduelle sur de l'acétate d'éthyle sous agitation. Le précipité est filtré, lavé à l'acétate d'éthyle et séché sous vide.
On obtient 153,3 g de produit, soit un rendement de 94,2% sur 4 étapes.
Contrôles RMN et CCM.

8°) Boc-Ser-Arg-Gln-Gln-Gly-OMe, AcOH

Une solution de 153,3 g de Boc-Ser-Arg($NO_2$)-Gln-Gln-Gly-OMe (208,9 mM) dans 1 litre de méthanol, 1 litre d'eau et 500 ml d'acide acétique est hydrogénée pendant 20 h en présence de 10 g de Pd/C à 10%. Après filtration du catalyseur et concentration de la solution, le résidu repris à l'eau est lyophilisé.
On obtient 153 g de produit, soit 97,8% de rendement.

9°) Boc-Ser-Arg-Gln-Gln-Gly-OH, HCl

Une solution de 104,4 g de Boc-Ser-Arg-Gln-Gln-Gly-OMe (139,4 mM) dans 2 l de DMF est additionnée sous refroidissement par bain de glace de 1 litre d'eau, puis de 27,88 g de NaOH (697 mM) dans 50 ml d'eau. Après 15 min de réaction à température voisine de 15°C, on neutralise par une solution d'acide chlorhydrique N jusqu'à obtenir un Ph de 6,5. Après évaporation, on triture le résidu dans le l'acétate d'éthyle, Le précipité formé est essoré, lavé à AcOEt et séche sous vide, puis à l'air.
On obtient 132,7 g de produit.

Purification: 109 g de Boc-Ser-Arg-Gln-Gln-Gly-OH, HCl sont purifiés par distribution à contre-courant dans le mélange n-butanol-méthanol-eau (4-1-5).

Après 700 transferts, on fractionne en trois parties qui, après évaporation et lyophilisation, donnent:

— une fraction de 27,5 g et
— deux fractions à repurifier de 23,79 g et 16,90 g

Contrôles RMN et CCM

| | | | |
|---|---|---|---|
| AAA: | Ser | : | 0,91 |
| | Arg | : | ,97 |
| | Gln(Glu) | : | 2,05 |
| | Gly | : | 1,01. |

Exemple V: Synthèse de Boc-Asp (OBzl)-Ile-Met-NH-$NH_2$ (fragment D)

1°) Boc-Met-NH-NH-Troc

4,31 g (10 mM) de Boc-Met-OH, DCHa en solution dans 50 ml acétate d'éthyle sont traités en présence de 20 ml d'eau par une solution saturée de bisulfate de potassium jusqu'à pH=3; la phase aqueuse est extraite plusieurs fois à l'acétate d'éthyle et les extraits séchés sur sulfate de magnésium. A cette solution, on ajoute 2,28 g (11 mM) de $H_2N$-NH-Troc (Troc=trichloro-2,2,2, éthoxycarbonyle, ce réactif étant préparé selon YAJIMA, Chem. Pharm. Bull. 1971, 19, 420).

Après refroidissement au bain de glace, on ajoute 2,37 g (11 mM) de DCC à 97% en solution dans 10 ml d'acétate d'éthyle. Après une nuit pendant laquelle le produit revient progressivement à TA, la dicyclohexylurée est filtrée et séchée (2,04 g) et la solution organique est lavée successivement par les solutions aqueuses suivantes: sulfate-bisulfate 5% 2 fois ClNa 2 M 2×bicarbonate de sodium 5%, 2 fois ClNa M 2 fois et enfin à l'eau 2 fois. Après séchage sur sulfate de magnésium et concentration du solvant presque à sec, on ajoute de l'hexane jusqu'à début de trouble et garde à +4° une nuit après quoi le précipité forme est filtre, lavé par un mélange d'hexane et d'acétate d'éthyle 4/1 et séché sou svide, on obtient ainsi 3,46 g (79%) Fc=92-4° alpha D 25°=−32°—C=1, dioxane.

CCM dans chloroforme-méthanol-AcOH 95/5/3—Rf=0,45

2°) TFA, H-Met-NH-NH-Troc

43,9 g (0,1 M) de Boc-Met-NH-NH-Troc en solution dans 200 ml de dichlorométhane et 20 ml d'éthanedithiol sont traités au bain de glace sous azote et avec agitation par 200 ml d'acide trifluoroacétique, on ôte le bain froid et laisse sous agitation pendant une heure. Le produit est isolé par élimination des réactifs volatils d'abord sous 20 mm puis 0,1 mm de pression, l'huile résiduelle est reprise 2 fois par 75 ml d'isopropanol en évaporant sous vide puis lavée 2 fois par 75 ml d'hexane par décantation après quoi elle est séchée sous vide en présence de potasse une nuit, après quoi elle commence à se solidifier et est utilisée telle quelle dans l'opération suivante.

3°) Boc-Ile-Met-NH-NH-Troc

L'huile ci-dessus (environ 0,1 M) de TFA, H-Met-NH-NH-Troc en solution dans 6500 ml d'acétate d'éthyle et refroidie au bain de glace est traitée par 12,7 ml (0,1 M) de N-éthylmorpholine puis 14,85 g (0,1 M) d'HOBt, 1 h 20 puis par 34,35 g (0,08 M) de Boc-Ile-OSu suivis de 12,7 ml (0,1 M) de N-éthylmorpholine (NEM). Après 1/2 h, le bain froid est ôté et par la suite de la NEM est ajoutée périodiquement de manière à maintenir le pH apparent vers 7.

Après 20 h, la réaction est complète et l'isolement se fait par lavages successifs par les solutions aqueuses suivantes: sulfate-bisulfate de potassium 5% 3 fois, de l'eau 3 fois, du bicarbonate de sodium 5% 3 fois et enfin, à l'eau jusqu'à neutralité.

Après séchage sur sulfate de magnésium et évaporation du solvant sous vide, on obtient 64 g de gomme qui est chromatographiée sur une colonne de silice avec du dichlorométhane contenant de 0 à 1,5% de méthanol. On obtient ainsi 30 g (68%) de produit ayant une pureté HPLC de 98,9% et un spectre RMN en accord avec la structure attendue. Fc: 88—92°.

4°) TFA, H-Ile-Met-NH-NH-Troc

27,6 g (50 mM) de Boc-Ile-Met-NH-NH-Troc en solution dans 140 ml de dichlorométhane et 14 ml d'éthanedithiol sont refroidis au bain de glace et agités sous azote puis on leur ajoute en 5—6 min, 140 ml d'acide trifluoroacétique. Le bain froid est ôté et, après 45 min, le produit est isolé par évaporation au maximum des réactifs, reprise de l'huile résiduelle par 2×60 ml d'isopropanol suivie d'évaporation et enfin par 2 lavages par 60 ml de pentane. Après séchage une nuit sur potasse sous vide, on obtient une huile d'aspect vitreux qui est utilisée dans l'opération suivante (30 g).

5°) Boc-Asp(OBzl)-Ile-Met-NH-NH-Troc

Les 30 g d'huile précédente en solution dans 250 ml de THF sont traités par 6,4 ml (50 mM) de NEM et 18,9 (45 mM) de Boc-Asp (OBzl)O Su. Le pH apparent est ajusté entre 6 et 7 par additions successives de NEM. Après 4 h, le produit est isolé par évaporation du THF, reprise dans 400 ml d'acétate d'éthyle, suivie des mêmes lavages que l'homologue précédent (3). Le résidu, après séchage et évaporation (36,5 g), est purifié par chromatographie sur une colonne de silice avec du dichlorométhane comme solvant contenant de 0 à 1,5% de méthanol. On obtient ainsi 25 g (66%) de produit présentant un spectre RMN conforme à la structure attendue. Fc=96—100°.

6°) Boc-Asp(OBzl)-Ile-Met-NH-$NH_2$

7,57 g (10 mM) de Boc-Asp(OBzl)-Ile-Met-NH-NH-Troc en solution dans 80 ml du mélange DMF-AcOH 80/20 sont traités à TA par 6,54 g de zinc en poudre fine sous agitation pendant 30 min. Le produit est isolé par filtration du zinc, traitement du filtrat par 800 g de glace, puis filtration après fusion de la glace suivie de lavages à l'eau répétés. Le solide blanc est d'abord séché à l'air puis sous vide poussé en présence de potasse et d'anhydride phosphorique. On obtient ainsi 5,60 g (96%) de produit à environ 95% de pureté d'après la CCM et la RMN. Fc=180—185°.—Contrôles CCM et HPLC—

Exemple VI: Boc-Arg-Lys(Z)-Leu-Leu-OH (fragment $E_1$)

1°) Boc-Leu-Leu-OMe

Dans 500 ml de dioxane contenant 18,4 g de NEM (0,16 M), on ajoute successivement 9,05 g de H-Leu-OMe, HCl (0,05 M), 7,4 g de HOBt (0,055 M) et 18 g de Boc-Leu-ONSu (0,055 M). Agiter à TA durant 18 h et ajuster le pH à 6—7 par NEM, au papier pH, si nécessaire en cours de la réaction. Evaporer le solvant, dissoudre le résidu dans AcOEt et laver la solution organique successivement 2 fois par $KHSO_4$—$K_2SO_4$ 5%, ClNa/eau, $NaHCO_3$ 5%, ClNa/eau. Sécher sur $MgSO_4$ et évaporer le solvant. Dissoudre le résidue dans le minimum d'éther et précipiter le produit par le pentane. Essorer. Sécher.

Rendement: 15 g (83%)

CCM chloroforme-méthanol-acide acétique 95-5-3

AcOET-hexane 25/75 -Fc: 131—136.

Possibilité de purification sur gel de silice 60 Merck (70—230 mesh) dans le chloroforme, avec élution par le même solvant.

2°) H-Leu-Leu-OMe, TFA

27,5 g de Boc-Leu-Leu-OMe (0,076 M) sont recouverts de 180 ml de TFA à TA: un léger échauffement se produit. Après 30 min, évaporer le solvant, reprendre l'huile résiduelle dans l'éther et gratter: un précipité blanc se forme; ajouter de pentane, essorer, sécher.

Rendement: 28,2 g (100%)

CCM: chloroforme-méthanol-acide acétique 95/5/3

chloroforme-méthanol-acide acétique 80/15/3.

3°) Boc-Lys(Z)-Leu-Leu-OMe

Dans 600 ml AcOEt refroidis au bain de glace, on ajoute successivement:

— 28,2 g de H-Leu-Leu-OMe, TFA (0,076 M)
— 26,22 g de NEM (0,0228 M)
— 11,23 g HOBt (0,083 M)
— 46,4 g de Boc-Lys(Z)OTcp (0,083 M).

Agiter, puis retirer le bain froid. Ajuster le pH à 6—7 par NEM si nécessaire. Après 18 h, laver la solution organique successivement par $KHSO_4$—$MgSO_4$ 5%, ClNa/eau, NaH $CO_3$, 5%, ClNa/eau. Sécher sur $MgSO_4$. Evaporer le solvant. Dissoudre l'huile résiduelle dans le chloroforme et déposer en tête d'une colonne (L: 90 cm-diamètre 5 cm) de gel de silice 60 Merck (70—230 mesh) dans le chloroforme. Eluer avec du chloroforme, fractionner. Evaporer les fractions contenant le produit pur; redissoudre dans l'éther la mousse obtenue et évaporer à sec. Triturer le résidu dans le pentane, essorer et sécher.

Rendement: 42,2 g (91%)

CCM AcOEt/hexane 1/1

chloroforme-MeOH-AcOH 95/5/3

Contrôle RMN-HPLC-EPP: 98,7%.

4°) Boc-Arg-Lys(Z)Leu-Leu-OMe

15,5 g de Boc-Lys(Z)Leu-Leu-OMe (0,025 M) sont recouverts de 100 ml de TFA à TA. Après 30 min avec agitation occasionnelle, évaporer le solvant à fond. Dissoudre l'huile résiduelle dans 150 ml de dioxane et amener à pH 6—7 par DIPEA, au papier pH. Refroidir au bain de glace et ajouter 7,21 g de Boc-Arg-OH, $H_2O$, HCl (0,025 M), 13,44 g de BOP (0,03 M) et 10,32 g de DIPEA (0,08 M). Agiter, puis retirer le bain froid et ajuster le pH à 6—7 par DIPEA si nécessaire. Après 18 h, évaporer le dioxane, dissoudre l'huile résiduelle dans AcOEt et laver successivement par $NaHCO_3$ 5%, ClNa/eau. Sécher sur $MgSO_4$. Evaporer le solvant et dissoudre l'huile dans MeOH-chloroforme 5—95, saturé d'eau, puis déposer en tête d'une colonne (L: 85 cm-diamètre: 4 cm) de gel de silice 60 Merck (70—230 mesh) dans le même mélange. Eluer avec:

— 1,5 l de MeOH-chloroforme 5—95 saturé d'eau
— 1 l de MeOH-chloroforme 7,5—92,5 saturé d'eau
— 2,5 l de MeOH-chloroforme.

Fractionner et évaporer les fractions pures. Reprendre dans l'éther, évaporer et tirer à fond sous vide.

Rendement: 16,8 g (87%)

CCM chloroforme-MeOH-AcOH 95/5/3

chloroforme-MeOH-AcOH 80/15/5

Contrôle RMN-HPLC-EPP 91,8%.

5°) Boc-Arg-Lys(Z)-Leu-Leu-OH

13,98 g de Boc-Arg-Lys(Z)Leu-Leu-OMe (0,018 M sont solubilisés dans 250 ml de MeOH; ajouter 40 ml d'eau, puis à TA 5,67 g de baryte (0,018 M), finement pulvérisée. Agiter 2h30 à TA. On contrôle la fin de la

réaction par CCM. Faire barboter $CO_2$ jusqu'à pH 6, filtrer. Evaporer le filtrat, le résidu est solubilisé dans l'alcool isopropylique et filtré. Concentrer l'alcool et précipiter le produit par l'éther.

Rendement: 12,31 g (98%)
CCM chloroforme-MeOH-AcOH 90/15/5
2-butanol-AcOH-eau 72/7/21
Contrôle RMN-HPLC-EPP 95,88.

Exemple VII: Boc-Gln-Leu-Ser-Ala-OMe (fragment $F_1$)

1°) Boc-Ser-Ala-OMe

Dissoudre 25 g de H-Ala-OMe, HCl et 66 g de Boc-Ser-ONb dans 500 ml de DMF. Refroidir au bain de glace et ajouter sous agitation magnétique 25 ml de NEM, 24 g de N-hydroxybenzotriazole et maintenir le pH à 6,5 ou 7 par addition de NEM. Agiter 18 h à TA. Après contrôle CCM, évaporer le milieu à 90% (0,1 mm Hg—35°C). L'huile obtenue est dissoute dans 1 500 ml de chloroforme. Laver avec une solution de chlorure de sodium saturée (2 fois), solution sulfate/bisulfate de potassium 5% 3 fois, bicarbonate de sodium saturée 5 fois. La solution est séchée sur $Na_2SO_4$ et évaporée à sec. On obtient une huile qui est séchée à poids constant (30°—0,1 mm/Hg).

Rendement: 53,78 g—Contrôle CCM—.

2°) H-Ser-Ala-OMe, TFA

Dissoudre 52,8 g de Boc-Ser-Ala-OMe dans 100 ml de chlorure de méthylène sous agitation magnétique en refroidissant au bain de glace. Ajouter 250 ml de TFA refroidi et agiter 20 min à TA. Filtrer. Evaporer le filtrat à sec (35° trompe à eau). Par addition de 2 l d'éther, un solide blanc précipite. Il est essoré, lavé à l'éther 3 fois. Sécher à poids constant (30°—0,1 mm Hg).

Rendement: 40,82 g.

3°) Boc-Leu-Ser-Ala-OMe

Dissoudre 40 g de H-Ser-Ala-OMe, TFA et 32,9 g de Boc-Leu-OH, $H_2O$ dans 400 ml de DMF. Refroidir au bain de glace sous agitation magnétique et ajouter 14,4 ml de NEM puis 65,8 g de BOP et assez de NEM pour maintenir le pH à 6,5—7. Agiter 2h à TA. Après contrôle CCM, évaporer à sec (0,1 mm—35°) et dissoudre l'huile obtenue dans 1 500 ml de chloroforme. Laver 2 fois avec une solution NaCl saturée, 3 fois avec sulfate/bisulfate de potassium 5%, 3 fois au bicarbonate de sodium saturé. Sécher/$Na_2SO_4$. Evaporer à sec (30°—trompe à eau). L'huile obtenue est solidifiée dans l'éther: Fraction A=29 g. Un deuxième jet B est obtenu par addition d'hexane, identique en CCM: B=13,7 g.

Rendement: 42,7 g—Contrôles RMN et CCM.

4°) H-Leu-Ser-Ala-OMe, TFA

Mettre en suspension 29 g de Boc-Leu-Ser-Ala-OMe dans 50 ml de chlorure de méthylène sous agitation magnétique en refroidissant au bain de glace. Ajouter 100 ml de TFA refroidi et, après dissolution, agiter 30 min à TA. Filtrer. Evaporer le filtrat à sec (35°—trompe à eau). L'huile obtenue est solidifiée dans l'éther (changer le solvant plusieurs fois). Sécher à poids constant (30°—0,1 mm Hg).

Rendement: 30,2 g.

5°) Boc-Gln-Leu-Ser-Ala-OMe

Dissoudre sous agitation magnétique 30 g de H-Leu-Ser-Ala-OMe, TFA et 25,5 g de Boc-Gln-ONp dans 300 ml de DMF. Neutraliser par 9,7 ml de NEM. Ajouter 10 g d'OHBt et agiter 2 h à TA en maintenant le pH à 6,5—7 par addition de NEM. Après contrôle CCM, le milieu est concentré à 90% (0,1 mm Hg—35°), repris dans 1 000 ml de chloroforme: un produit précipité en gel. Essorer, Laver en reprenant dans 500 ml de chloroforme 2 fois, puis dans l'éther 3 fois. Sécher à poids constant (0,1 mm Hg—30°).

Rendement: 32,1 g.
CCM: chloroforme/MeOH/AcOH: 9/2/0,5—Rf=0,81—HPLC—EPP=91%.
AAA: Ser: 0,86-Glu: 1,02-Ala: 0,99-Leu: 0,99—.

Exemple VIII: Boc-Tyr-Arg-Lys(Z)-Val-Leu-Gly-OH, HCl (fragment $G_1$)

1°) Z-Leu-Gly-$OCH_3$

On dissout 0,22 mole de Z-Leu-OH dans 500 ml de DMF. A cette solution, on ajoute successivement:

— 25,11 g de HCl-H-Gly-$OCH_3$ (0,2 mole)
— 28 ml de NEM
— 96 g de Bop (0,22 mole)

puis on ajuste le pH à 7 par addition de NEM.

On agite le mélange réactionnel à TA en maintenant le pH à 7 par addition de NEM si nécessaire. La réaction est suivie en CCM (chloroforme-methanol-acide acétique 95/5/9). Au bout de 2 h, la réaction est terminée. Le mélange réactionnel est évaporé à sec sous pression réduite (0,2 mm de mercure) et à

température inférieure à 30°C. Le résidu est repris dans l'acétate d'éthyle (1 litre). La solution est lavée successivement avec:

— solution aqueuse de bicarbonate (4×200 ml)
— solution aqueuse de $SO_4HK$-$SO_4K_2$ (4×200 ml)
— solution aqueuse saturée de chlorure de sodium (2×200 ml)

puis, elle est séchée sur sulfate de magnésium et évaporée à sec sous pression réduite. Le résidu est repris dans de l'éther (500 ml), titure, éssoré, séché.

Rendement: 56,7 g (84,28%).

CCM chloroforme-méthanol-acide acétique 95/5/3—Rf: 0,44—.

2°) HCl-H-Leu-Gly-$OCH_3$

On dissout 56 g (0,166 mole) de Z-Leu-Gly-$OCH_3$ dans 900 ml de méthanol chlorhydrique 0,23 N. A cette solution, on ajoute 6 g de Pd/C à 10% contenant 50% d'humidité. On hydrogène pendant 24 h, à TA et sous pression atmosphérique. On filtre le catalyseur et la solution est évaporée à sec sous pression résuite à température inférieure à 30°C. On reprend le résidue par de l'éther (2×50 ml), on décante et on séche le produit au dessiccateur en présence d'anhydride phosphorique. On obtient une poudre blanche.

Rendement: 33,53 g (84,4%).

CCM chloroforme-méthanol-acide acétique 90/20/9—Rf: 0,15.

Contrôle RMN.

3°) Boc-Val-Leu-Gly-$OCH_3$

28,24 g de Boc-Val-OH (0,13 mole) sont dissous dans 400 ml de tétrahydrofuranne refroidi à −10°C. On ajoute ensuite 14,45 ml (0,13 mole) de chloroformiate d'isobutyle. Le mélange est vigoureusement agité à −10°C pendant 15 min. A ce mélange, on ajoute une solution tétrahydrofurannique refroidie à −10°C contenant 31,5 g du chlorhydrate de H-Leu-Gly-$OCH_3$ (0,13 mole) préalablement neutralisé par 14,45 ml de N-méthylmorpholine. On poursuit l'agitation en refroidissant le mélange réactionnel dans un bain de glace (2 h) puis à TA pendant 2 h. Le mélange réactionnel est évaporé à sec sous pression réduite température inférieure à 30°C. Le résidu est repris dans un mélange acétate d'éthyle-eau (500 ml—500 ml). La phase aqueuse est décantée et la phase organique lavée successivement avec:

— une solution aqueuse de bicarbonate de soude (2×50 ml)
— une solution aqueuse de $SO_4HK$-$SO_4K_2$ (pH 2) (2×50 ml)
— une solution aqueuse de chlorure de sodium (50 ml),

puis séchée sur sulfate de magnésium et évaporée à sec sous pression réduite à température inférieure à 30°C. Le résidu est repris avec le péntane (50 ml), essoré et séché sous vide (0,1 mm de mercure).

Rendement: 39,7 g (76%).

CCM chloroforme-acétone 75/25—Rf: 0,46.

Contrôle RMN.

4°) TFA H-Val-Leu-Gly-$OCH_3$

On dissout 39 g de Boc-Val-Leu-Gly-$OCH_3$ (0,097 mole) dans 190 ml de TFA refroidi dans un bain de glace. Après dissolution, on enlève le bain de glace et on agite le mélange pendant 30 min à TA. On évapore à sec sous pression réduite et le résidu est repris dans l'éther (100 ml), trituré et l'éther décante. Cette opération est répétée 2 fois et les dernières traces de solvants sont éliminées sous pression réduite (0,1 mm de mercure).

Rendement: 44,8 g.

CCM chloroforme-méthanol-acide acétique 90/20/3—Rf: 0,36.

5°) Boc-Lys(Z)-Val-Leu-Gly-$OCH_3$

On dissout 25,55 g de TFA H-Val-Leu-Gly-$OCH_3$ (0,0615 mole) dans 200 ml de DMF. La solution est amenée à pH 7 par addition de NEM. On ajoute alors à cette solution 32,47 g de Boc-Lys(Z)-OTCP (0,058 mole) et 9 g de HOBT (0,059 mole). Le pH du mélange réactionnel est ramené à 7 part addition de NEM. On agite à TA en maintenant le pH à 7 par addition de NEM si nécessaire. La réaction est suivie en CCM: chloroforme-méthanol 90/10; chloroforme-méthanol-acide acétique (90/20/3 (2 milieux).

Au bout de 1 heure, on rajoute, 1,96 g de Boc-Lys(Z) OTCP (0,0035 mole) et 0,61 g de HOBT. Le pH de la solution est ramené à 7 par addition de NEM.

2 h après, la réaction est terminée en CCM. Le mélange réactionnel est alors évaporé à sec sous pression réduite (0,1 mm de mercure) à température inférieure à 30°C. Le résidu est repris avec 500 ml d'eau, trituré. Le solide formé est essoré puis lavé avec:

— 500 ml de solution aqueuse de $KHSO_4$—$K_2SO_4$ (pH 2)
— 500 ml d'une solution aqueuse saturée de bicarbonate de soude
— 200 ml d'eau

puis à l'éther (200 ml×2) et séché.

Le produit impur est chromatographié sur une colonne de gel de silice (5 cm×150 cm). Il est élué avec le mélange chloroforme-acétate d'éthyle 80/20 (débit 600 ml/h). Les bonnes fractions déterminées en CCM sont réunies, évaporées à sec sous pression réduite. Le résidu est repris dans l'éther, trituré et le solide obtenu essoré.

Poudre blanche 35,14 g (86%).
CCM chloroforme-méthanol 90/10—Rf: 0,62.
Chloroforme-méthanol-acide acétique 87, 7—9,4—2,8Rf: 0,52.
Contrôle RMN.
AAA: AC Gly 1,00-Val 0,96-Leu 1,06-Lys 0,98.

6°) TFA H-Lys(Z)-Val-Leu-Gly-$OCH_3$

On dissout 15 g de Boc-Lys(Z)-Val-Leu-Gly-$OCH_3$ (22,6 mM) dans un mélange de TFA-chlorure de methylène (75 ml/75 ml). On agite 40 min à TA, puis on évapore à sec sous pression réduite à température inférieure à 30°C. Le résidu est repris par de l'éther contenant 20% d'hexane (100 ml). Le solide formé est essoré et séché. On obtient un solide blanc de 14,51 g.

Rendement: 94,7%.
CCM chloroforme/méthanol/acide acétique 95/5/3—Rf: 0,12.

7°) Boc-Arg(HCl)-Lys(Z)-Val-Leu-Gly-$OCH_3$

On dissout 14,1 g de TFA H-Lys(Z)-Val-Leu-Gly-$OCH_3$ (21 mM) dans 50 ml de DMF. La solution est amenée à pH par addition de NEM. On ajoute alors à cette solution 6,57 g de Boc-Arg (HCl)-OH · $1H_2O$ (20 mM) et 10,6 g de BOP (24 mM). Le pH du mélange réactionnel est ramené à 6—7 par addition de NEM. On agite à TA en maintenant le pH à 6—7 par addition de NEM si nécessaire. La réaction est suivie en CCM: chloroforme/méthanol (80/20).

Au bout de 5 h, la réaction est terminée. On évapore à sec le mélange réactionnel sous pression réduite (0,1 mm de mercure) et à température inférieure à 30°C. L'huile obtenue est reprise dans 300 ml d'acétate d'éthyle. Le solide formé est essoré et lévé à l'éther (Ier jet). On rajoute de l'éther au filtrat précédent; un solide précipite qui est essoré (2ème jet): les 2 jets sont indentiques en CCM. Ils sont lavés à l'état solide par agitation pendant 1 h avec un mélange de 200 ml d'eau saturée en acétate d'éthyle et 60 ml d'eau. Le solide est essoré puis lavé avec 60 ml d'eau et 300 ml d'éther et séché.

Rendement: 78,7% (13,49 g).
CCM chloroforme/méthanol 20/20—Rf=0,32.
Contrôle RMN.

8°) TFA H-Arg(HCl)-Lys(Z)-Val-Leu-Gly-$OCH_3$

13,3 g de Boc-Arg(HCl)-Lys(Z)-Val-Leu-Gly-$OCH_3$ (15,5 mM) sont dissous dans un mélange TFA-chlorure de méthylène (60 ml/60 ml). On agite 40 min à TA, puis on évapore à sec sous pression réduite à température inférieure à 30°C. Le residu est repris par un mélange éther-hexane (80/20:100 ml). Le solide formé est essoré, lavé à l'hexane et séché (poids: 15,13 g).

CCM: chloroforme/méthanol (3/1)—Rf: 0,35.

9°) Boc-Tyr-Arg(HCl)-Lys(Z)-Val-Leu-Gly-$OCH_3$

On dissout 15,5 mM de TFA H-Arg(HCl)-Lys(Z)-Val-Leu-Gly-$OCH_3$ dans 50 ml de DMF. La solution est amenée à pH 6 par addition de NEM. On ajoute alors à cette solution 8,20 g de Boc-Tyr-OTCP (15,5×10 mM) et 2,41 g de HOBT (15,5×10 mM). On ramène le pH à 6 par addition de NEM. Le mélange réactionnel est agité à TA en maintenant le pH à 6 par addition de NEM si nécessaire. La réaction est suivie en CCM: chloroforme/méthanol (3/1). Au bout de 3 h, la réaction est terminée. On évapore à sec le mélange réactionnel sous pression réduite (0,1 mm de mercure) et à température inférieure à 30°C. L'huile résiduelle est reprise dans de l'acétate d'éthyle (300 ml). Le solide gélatineux formé est essoré, lavé avec de l'acétate d'éthyle (100 ml), avec de l'acétate d'éthyle-éther (1/1; 100 ml), puis à l'éther (100 ml), enfine à l'hexane (100 ml). Le produit est séché sous pression réduite jusqu'à poids constant.

Rendement: 96% (15,14 g).
CCM chloroforme/méthanol (3/1)—Rf: 0,47.
Contrôle RMN.
AA: AC: Gly: 0,95-Val: 0,98-Leu: 1,02-Lys, 1,01.
Arg: 1,01-Tyr: 1,05.
HPLC: 93,81% EPP.

10°) Boc-Tyr-Arg(HCl)-Lys(Z)-Val-Leu-Gly-OH

7 g de Boc-Tyr-Art(HCl)-Lys(Z)-Val-Leu-Gly-$OCH_3$ (6,86 mM) sont dissous dans un mélange de 70 ml de

dioxane et 35 ml d'eau. On ajoute à cette solution 6,4 ml de soude 4N (25,6 mM): 3,7 équivalents) et on agite pendant 30 min à TA. On dilue avec 200 ml d'eau et 800 ml d'acétate d'éthyle. Le mélange est alors acidifié à pH 3 par addition de HCl (N). On décante l'acétate d'éthyle et on essore le solide (ler jet). L'acétate d'éthyle est évaporé à sec: on obtient un 2ème jet. Les 2 jets identiques en CCM sont réunis, lavés à l'éther et séchés.

Rendement: 74,7% (5,16 g).
CCM: chloroforme/méthanol (2/1)—Rf: 0,45.
1H Contrôle RMN.
AAA: Ac: Gly: 1,00-Val: 0,97-Leu: 1,04-Tyr: 0,95.
Lys: 0,99-Arg: 1,05.
HPLC-EPP 91,52%.

Exemple IX: Boc-Ile-Phe-Thr-Asn-Ser-NH-$NH_2$ (fragment $H_1$)

1°) Boc-Asn-Ser-OMe

Une solution de 23,32 g de Boc-Asn-OH (0,1 M) et 19,7 g de NbOH (0,11 M) dans 250 ml de DMF, refroidie sur bain de glace est additionnée de 22,69 g de DCC. Après 3 h de réaction à TA, on filtre la DCU, on refroidit sur bain de glace et ajoute 15,56 g de HCl, H-Ser-OMe (chlorhydrate de H-Ser-OMe). Le pH est amené, puis maintenu à 7 par addition de NEM. Après 4 h TA et 20 h à +4°, la solution est concentrée sous pression réduite. La résidu est repris par le mélange AcOEt/n-butanol (50/50). La solution organique est lavée successivement par:

— une solution saturée de bicarbonate de sodium
— une solution saturée de chlorure de sodium
— une solution de $KHSO_4/K_2SO_4$ à 5%
— une solution saturée de chlorure de sodium.

Après séchage sur $MgSO_4$ et évaporation sous pression réduite, le résidu est cristallisé dans le mélange éther-hexane. Filtration, lavage à éther/hexane et séchage sous vide donnent 26,9 g de produit.

Rendement: 80,8%.
Contrôles RMN et CCM.

2°) TFA, H-Asn-Ser-OMe

Une solution refroidie sur bain de glace de 13,5 g de Boc-Asn-Ser-OMe (40,5 mM) dans 54 ml de dichlorométhane est additionnée de 81 ml de TFA. Après 1 h de réaction à TA, la solution est concentrée sous pression réduite, le résidu est repris à l'éther. On obtient une huile épaisse qui est utilisée dans la réaction suivante.

3°) Boc-Thr-Asn-Ser-OMe

Une solution refroidie sur bain de glace du produit brut précédemment obtenu dans 300 ml de DMF est additionnée de 14,09 g de Boc-Thr-ONSu (44,55 mM) 5,58 g d'HOBt (41,3 mM) et de NEM jusqu'à obtenir un pH de 7. Après 20 h de réaction à TA, la solution est concentrée sous pression réduite et le résidu est repris dans le mélange AcOEt/n butanol. La solution organique est lavée successivement par:

— une solution saturée de bicarbonate de sodium
— une solution saturée de chlorure de sodium
— une solution de $KHSO_4/K_2SO_4$ à 5%
— une solution saturée de chlorure de sodium.

Après séchage sur $MgSO_4$, la solution est concentrée sous pression réduite et le résidu cristallisé dans le mélange éther/hexane. On obtient 8,28 g de produit.

Rendement: 47%.
Contrôles RMN et CCM.

4°) TFA, H-Thr-Asn-Ser-OMe

Une solution refroidie sur bain de glace 8 g de Boc-Thr-Asn-Ser-OMe dans 32 ml de dichlorométhane est additionnée de 54 ml de TFA. Après 1 h de réaction à TA, la solution est concentrée sous pression réduite et le résidu précipité à l'éther. Après filtration, il est utilisé tel quel dans la réaction suivante.

5°) Boc-Phe-Thr-Asn-Ser-OMe

Une solution du précipité obtenu a l'étape précédente dans 100 ml de DMF est additionnée sous refroidissement par bain de glace de 7,82 g de Boc-Phe-ONp (20,24 mM), 2,53 g d'HOBt (18,71 mM) et de la N-éthylmorpholine jusqu'à obtenir un pH de 7. Après 4 h, la solution est concentrée sous pression réduite et le résidu est repris dans AcOEt. La solution organique est lavée par:

— une solution saturée de bicarbonate de sodium
— une solution saturée de chlorure de sodium
— une solution de $KHSO_4/K_2SO_4$ à 5%
— une solution saturée de chlorure de sodium.

Après séchage sur $MgSO_4$, concentration de la phase organique le résidu est cristallisé dans l'éther. On obtient 10 g de produit.
Rendement: 93,5%.
Contrôles CCM et RMN.

6°) TFA, H-Phe-Thr-Asn-Ser-OMe
Une solution de 10 g de Boc-Phe-Thr-Asn-Ser-OMe (17,19 mM) dans 40 ml de dichlorométhane est additionnée sous refroidissement par bain de glace de 60 ml de TFA. Après 1 h de réaction à TA, le produit est précipité à l'éther. La gomme obtenue après séchage s'organise pour donner 5,14 g de produit.
Rendement: 48,5%.

7°) Boc-Ile-Phe-Thr-Asn-Ser-OMe
Une solution refroidie sur bain de glace de 5,14 g de TFA, H-Phe-Thr-Asn-Ser-OMe (8,78 mM) dans 100 ml de DMF est additionnée de 3,17 g de Boc-Ile-OSu (9,66 mM), 1,30 g d'HOBt (9,62 mM) et de N-éthylmorpholine jusqu'à obtenir un pH de 7. Après 20 h de réaction à TA, la solution est concentrée sous pression réduite et le résidu repris à l'éther donne un précipité qui est filtré et séché sous vide. On obtient 5,24 g de produit.
Rendement: 85,9%.
Contrôles RMN et HPLC.

8°) Boc-Ile-Phe-Thr-Asn-Ser-NH-$NH_2$
Une solution de 5 g de Boc-Ile-Phe-Thr-Asn-Ser-OMe (7,2 mM) dans 40 ml de DMF et 200 ml de méthanol refroidie sur bain de glace est additionnée de 3,6 ml d'une solution à 80% d'hydrate d'hydrazine. Après 21 h de réaction à TA, on ajoute 0,9 ml de la même solution d'hydrate d'hydrazine. Après 3 h supplémentaires, le gel obtenu est filtré, lavé au méthanol et séché sous vide. On obtient 3,94 g de produit.
Rendement: 78,8%.
Contrôles RMN et CCM.

Exemple X: Z-Tyr-Ala-Asp(OBzl)-Ala-OH (fragment I)
1°) Boc-Asp(OBzl)-Ala-OBut
3,62 g (0,02 mole) de chlorhydrate d'alaninate de tertiobutyle sont mis en suspension dans 30 ml d'acétonitrile. 2,52 ml de NEM sont ajoutés puis 8,4 g (0,02 mole) de Boc-Asp(OBzl) ONSU. Le pH est maintenu à 6—6,5 par addition de NEM. L'agitation est poursuivie 18 h. Après contrôle CCM le milieu est évapore à sec, l'huile est reprise dans 50 ml AcOET, la solution lavée 2 fois par une solution $KHSO_4/K_2SO_4$, puis à l'eau salée, séchée sur $Na_2SO_4$ et évaporée à sec. L'huile obtenue est dissoute dans l'hexane, un peu de solide est filtré et le filtrat est évaporé à sec. L'huile obtenue est utilisée telle quelle.
CCM: $CHCl_3$/Acétone/ACOH 80/15/5—Rf=0,8.

2°) Boc-Ala-Asp(OBzl)-Ala-OH
L'huile brute obtenue précédemment est dissoute dans 50 ml TFA glacé et agitée 1h30 à TA. Le TFA est évaporé au maximum et l'huile résiduelle est reprise 3 fois dans l'éther anhydre et décantée. Elle est ensuite séchée au dessiccateur sous vide en présence de pentane. Il se forme une mousse solide qui est écrassée et remise à sécher.
On obtient 9,4 g de produit brut utilisé tel quel pour le couplage.
Le sel de TFA ci-dessus est dissous dans un mélange de 20 ml DMF et 18 ml d'eau distillée. Le NEM est ajoutée jusqu'à pH 7 (utilisation du pH métre). 4 g (0,014 mole) de Boc-Ala-ONSu en solution dans 16 ml de DMF sont introduits. Le pH est maintenu à 6,5—7 par addition de NEM. Après 5h30, la réaction est terminée (contrôle CCM). Le solvant est évaporé au maximum et le résidu est repris dans un mélange d'eau et d'acétate d'éthyle. La phase organique est lavée par une solution de $NaHCO_3$, à l'eau salée, séchée sur $NA_2SO_4$ et évaporée à sec. Le résidu est dissous dans l'éther et précipité par l'hexane pour donner une gomme qui cristallise après triturage. Le solide est essoré et séché à l'air.
Rendement: 5,05 g.
CCM $CHCl_3$-acétone-ACOH 80/15/5—Rf=0,28.

3°) Z-Tyr-Ala-Asp(OBzl)-Ala-OH
1 g (2 mmoles) de Boc-Ala-Asp(OBzl)-Ala-OH est introduit dans 10 ml TFA glacé agité. Après 30 min d'agitation à TA, le TFA est évaporé au maximum, le résidu repris 2 fois dans l'éther anhydre et décanté, puis séché au dessiccateur sous vide en présence de potasse. Il se forme une mousse solide. Le produit brut est mis en solution dans un mélange de 4 ml DMF et 2 ml d'eau distillée. NEM est ajouté jusqu'à pH 7—7,5. 1 g (2,4 mmoles) de Z-Tyr-ONSu est alors ajouté et le pH est maintenu à 7—7,5 par NEM (pH mètre).

Au bout de 2h30, après contrôle CCM, le solvant est évaporé au maximum à 40°C. Le résidu est repris dans l'acétate d'éthyle et lavé à l'eau, un peu de solide est éliminé et l'acétate d'éthyle est lavé au mélange sulfate/bisulfate et à l'eau salée. Après abandon quelques heures, il se forme un précipité qui est essoré, lavé à l'éther et séché à l'air. 500 mg sont ainsi obtenus. Un 2ème jet de 350 mg légérement moins pur est obtenu par concentration de l'acétate d'éthyle.

CCM chloroforme-ACOH 3/1 Rf: 0,4.
butanol-ACOH-$H_2O$ 72/7/2 Rf: 0,85.
chloroforme-acétone-ACOH 80/15/5 Rf: 0,1.
HPLC 90,9% pureté polypeptidique.
Contrôle RMN.

Exemple XI: synthèse de: H-Arg-Lys(Z)-Leu-Leu-Gln-Asp(Bzl)-Ile-Met-Ser-Arg-Gln-Gln-Gly-Glu(OBzl)-Ser-Asn-Gln-Glu(OBzl)-Arg-Gly-Ala-Arg-Ala-Arg-Leu-$NH_2$ (peptide K)

1°) Boc-Glu(OBzl)-Arg-Gly-Ala-Arg-Ala-Arg-Leu-$NH_2$, 3 HCl

34,47 g de H-Ala-Arg-Ala-Arg-Leu-$NH_2$, HCl (57 mM) sont dissous dans 230 ml de DMF. On ajoute 28 g de BOP (63 mM), puis en 1 h 33,5 g (57 mM) de Boc-Glu(OBzl)-Arg-Gly-OH en solution dans 190 ml de DMF. Le pH du milieu réactionnel est maintenu autour de 6 par addition de NEM. Après 4 h, le milieu réactionnel est concentré sous vide de moitié et le résidu versé sur 800 ml d'acétate d'acétate d'éthyle. Le précipité obtenu est filtré, lavé à l'acétate d'éthyle, à l'éther et séché sous vide. On obtient 56,8 g de produit avec un rendement de 80%.

Identification: 1H RMN et AAA: Glu: 0,98-Gly: 1,01-Ala: 1,97 Leu: 1,05-Arg: 2,97.
HPLC phase reverse EPP 95%.
CCM dans EPAW 40/20/10/6 Rf=0,55.

2°) H-Glu(OBzl)-Arg-Gly-Ala-Arg-Ala-Arg-Leu-$NH_2$, TFA, HCl

55,3 g du produit ci-dessus sont agités 30 min dans 250 ml de $CH_2Cl_2$+250 ml de TFA. Puis le milieu réactionnel est concentré sous vide de moitié et versé sur 1200 ml d'éther glacé. Le précipité obtenu est filtré, lavé à l'éther, séché sous vide sur potasse.

Rendement: 100%.

3°) Boc-Glu(OBzl)-Ser-Asn-Gln-Glu(OBzl)-Arg-Gly-Ala-Arg-Ala-Arg-Leu-$NH_2$, HCl

Au précédent sel de TFA (45 mM) dissous dans 400 ml de DMF et neutralisé par de la NEM, on ajoute 22,2 g de BOP (50 mM) puis, en 1 h, 30 g (45 mM) de Boc-Glu(OBzl)Ser-Asn-Gln-OH en solution dans 300 ml de DMF. Le pH du milieu réactionnel est maintenu à 6. Après 6 h, le milieu est concentré de moitié sous vide à 30°C puis versé sur 1 200 ml d'acétate d'éthyle. Le précipité formé est filtré, lavé à l'acétate d'éthyle, puis à l'éther et finalement séché sous vide.

Rendement: 60,5 g (75%).

Produit identifié par RMN et AA: Asp: 1,00-Ser: 0,81-Glu: 2,71-Gly: 0,89-Ala: 1,82-Leu: 0,96-Arg: 3,02-HPLC phase réverse.

EPP 70%.
CCM: EPAW 30/20/10/6 Rf=0,35.

4°) H-Glu(OBzl)-Ser-Asn-Gln-Glu(OBzl)-Arg-Gly-Ala-Arg-Ala-Arg-Leu-$NH_2$, TFA, HCl

53,3 g (30 mM) du produit ci-dessus sont agités 35 min dans 250 ml de dichlorométhane plus 250 ml de TFA. Après concentration sous vide de moitié, le résidu est versé sur 1 300 ml d'éther glacé; le précipité formé est filtré, lavé à l'éther, puis séché sous vide sur potasse.

5°) Boc-Ser-Arg-Gln-Gln-Gly-Glu(OBzl)-Ser-Asn-Gln-Glu(OBzl)-Arg-Gly-Ala-Arg-Ala-Arg-Leu-$NH_2$, HCl

Le précédent sel de TFA est dissous dans 300 ml de DMF et neutralisé par la NEM. On ajoute 14,8 g de BOP (33 mM) puis par petites portions en 1 h 21,4 g (30 mM) de Boc-Ser-Arg-Gln-Gln-Gly-OH. Le pH du milieu réactionnel est maintenu autour de 6 par la NEM. A Après 7 heures, le milieu est versé sur 2 l d'ACOEt. Le précipité obtenu est filtré, lavé à l'acétate d'éthyle, puis à l'éther et enfin séché sous vide.

Rendement 57 g (80%).

Produit identifié par RMN, AAA-Ser: 1,7-Arg: 4,1-Glu: 4,7-Gly: 2,05-Asp: 1,00-Ala: 1,91-Leu: 0,97-CCM BPWI-Rf: 0,35.

6°) H-Ser-Arg-Gln-Gln-Gly-Glu(OBzl)-Ser-Asn-Gln-Glu(OBzl)-Arg-Gly-Ala-Arg-Ala-Arg-Leu-$NH_2$, TFA, HCl

14,21 g (6 mM) du produit précédent en suspension dans 80 ml de dichlorométhane sont traités en 10 min sous azote par 120 ml d'acide trifluoroacétique. Après 45 min à TA sous agitation, on concentre sous vide au 1/3 du volume initial et coule sur 750 ml d'éther froid (+4°) agité. Le précipité est filtré et lavé à l'éther puis séché sous vide sur potasse puis anhydride phosphorique. On obtient ainsi 13,9 g (97%) de produit utilisé tel quel dans la réaction suivante.

7°) Boc-Asp-(OBzl)-Ile-Met-Ser-Arg-Gln-Gln-Gly-Glu(OBzl)-Ser-Asn-Gln-Glu(OBzl)-Arg-Gly-Ala-Arg-Ala-Arg-Leu-$NH_2$, HCl

5,82 g (10 mM) de Boc-Asp(OBzl)-Ile-Met-NH-$NH_2$ en solution dans 76,6 ml de DMF sont traités entre

−20° et −25° par 6,66 ml (40 mM) d'HCl gaz 6,0 N/dioxane, puis par 1,45 ml (12 mM) de nitrite de tertiobutyle en solution dans 5 ml de DMF. Après 1 h entre −20 et −25° on neutralise à pH apparent de 6—7 par addition d'environ 8 ml de diisopropyléthylamine (DIPEA) et ajuste le volume total à 100 ml par un peu de DMF, ce qui conduit à une solution d'azide à 0,1 molaire (qui est toujours conservée vers −20°). 13,84 g (environ 5,8 mM) du sel de TFA précédent dissous dans 58 ml de DMSO et 87 ml de DMF sont neutralisés à pH apparent de 6,7 par 1 ml de DIPEA et la solution ainsi obtenue est additionnée en 10 min environ à 72,5 ml (soit environ 1,25 équivalent) de la solution d'azide ci-dessus à température comprise entre −20 et −25°. Le pH est ajusté à 6—7 par un peu de DIPEA et la solution conservée à −15°. Après 16 h, 14,5 ml (0,25 équivalent) supplémentaires de la solution d'azide sont ajoutés. Un contrôle en CCM montre une réaction complète après 24 h, cependant la réaction n'est stoppée qu-après 40 heures par coulée de la solution sur 750 ml d'acétate d'éthyle glacé, agité. Le précipité ainsi formé est filtré et lavé abondamment par le même solvant et séché sous vide en présence d'anhydride phosphorique. On obtient ainsi 14,35 g utilisés tels quels dans l'étape suivante.

8°) H-Asp-(OBzl)-Ile-Met-Ser-Arg-Gln-Gln-Gly-Glu(OBzl)-Ser-Asn-Gln-Glu(OBzl)-Arg-Gly-Ala-Arg-Ala-Arg-Leu-$NH_2$, TFA, HCl

14 g du produit ci-dessus sont agités 35 min dans 80 ml de $CH_2Cl_2$+120 ml de TFA+10 ml $HS(CH_2)_2SH$. Après concentration sous vide de moitié, le milieu réactionnel est versé sur 800 ml d'éther glacé. Le précipité obtenu est filtré, lavé à l'éther, puis séché sous vide sur potasse.

Rendement: 100%.

9°) Boc-Gln-Asp(OBzl)-Ile-Met-Ser-Arg-Gln-Gln-Gly-Glu(OBzl)-Ser-Asn-Gln-Glu-(OBz)-Arg-Gly-Ala-Arg-Ala-Arg-Leu-$NH_2$, HCl

Au sel de TFA obtenu ci-dessus (4,9 mM) dissous dans 120 ml de DMF plus 30 ml de DMSO et neutralisé par de la NEM, on ajoute 740 mg d'HOBt (5,5 mM) puis par petites portions 2,02 g de Boc-Gln-ONp (5,5 mM), le pH du milieu réactionnel est maintenu vers 6 par addition de NEM. Après 6 h, le milieu réactionnel est versé dans 800 ml d'AcOEt. Le précipité obtenu est filtré, lavé à l'acétate d'éthyle, puis à l'éther, puis séché sous vide.

Poids: 14,8 g.

Rendement: 100%.

Le produit est identifié par RMN et AAA: Asp: 1,97-Ser: 2,0-Glu: 6,12-Gly: 2,06-Ala: 2,06-Met: 0,55-Ile: 0,93-Leu: 0,99-Arg: 3,81-CCM: Rf 0,45-$BPEW_1$.

10°) H-Gln-Asp(OBzl)-Ile-Met-Ser-Arg-Gln-Gln-Gly-Glu(OBzl)-Ser-Asn-Gln-Glu(OBzl)-Arg-Gly-Ala-Arg-Ala-Arg-Leu-$NH_2$, TFA, HCl

14,7 g du produit ci-dessus sont agités 35 min dans 80 ml de $CH_2Cl_2$+10 ml $HS(CH_2)_2SH$+120 ml de TFA. Après concentration sous vide de moitié, le milieu réactionnel est versé sur 600 ml d'éther glacé. Le précipité obtenu est filtré, lavé à l'éther puis séché sous vide sur potasse.

Poids: 14,5 g.

11°) Boc-Arg-Lys(Z)-Leu-Leu-Gln-Asp(OBzl)-Ile-Met-Ser-Arg-Gln-Gln-Gly-Glu(OBzl)-Ser-Asn-Gln-Glu(OBzl)-Arg-Gly-Ala-Arg-Ala-Arg-Leu-$NH_2$, HCl

Au sel de TFA obtenu ci-dessus (4,9 mM) dissous dans 120 ml de DMF+40 ml de DMSO et neutralisé par de la NEM, on ajoute 2,44 g de BOP (5,5 mM) et 4,2 g de Boc-Arg-Lys(Z)-Leu-Leu-OH, HCl (5,5 mM) par petites portions en 30 min. Le pH du milieu réactionnel est maintenu vers 6 par addition de NEM. Après 23 h, le milieu réactionnel est versé sur 1 litre d'AcOEt. Le précipité formé est filtré, lavé par AcOEt puis $Et_2O$, puis séché sous vide.

M: 16,5g-Rendement: 93%-Produit identifié par RMN et AAA: Asp: 2,10-Ser: 1,86-Glu: 6,2-Gly: 2,00-Ala: 2,00-Mét: 0,55-Ile: 0,80-Leu; 2,89-Lys: 0,87-Arg: 4,67-CCM Rf: 0,40 ($BPEW_1$).

Exemple XII: Synthèse de: Boc-Ile-Phe-Asn-Thr-Ser-Tyr-Arg-Lys(Z)-Val-Leu-Gly-Gln-Leu-Ser-Ala-OH, HCl (peptide J)

1°) H-Gln-Leu-Ser-Ala-$OCH_3$

10,3 g (18,8 mM) de Boc-Gln-Leu-Ser-Ala-$OCH_3$ sont agités 35 min dans 100 ml de dichlorométhane contenant 100 ml de TFA. Le milieu réactionnel est concentré sous vide à environ 50 ml et versé sur 300 ml d'éther. Le précipité est essoré, lavé à l'éther et séché sous vide.

2°) Boc-Tyr-Arg(Z)-Val-Leu-Gly-Gln-Leu-Ser-Ala-$OCH_3$, HCl

Au sel de TFA obtenu ci-dessus, dissous dans 200 ml de diméthylformamide et neutralisé par de la N-éthylmorpholine, on ajoute 18,9 g (18,8 mM) de Boc-Tyr-Arg-Lys(Z)-Val-Leu-Gly-OH, HCl puis 9,2 g de BOP (21 mM) puis de la NEM pour amener le pH du milieu réactionnel vers 6. Après 4 h d'agitation, le

milieu réactionnel est versé sur 1 l d'AcOEt. Le précipité obtenu est filtré, lavé 3 fois par 100 ml d'AcOEt, puis 3 fois par 100 ml d'$Et_2O$ puis il est séché sous vide.

On obtient 24,16 g de produit.

Rendement: 90%.

CCM Rf: 0,20 ($BEW_1$)-alpha D: −17,6°-(CC: 1-DMF à 5% d'AcOH).

Produit identifié par 1H RMN et AAA: Ser: 1,01-Glu: 0,99-Gly: 1,06-Ala: 1,01-Val: 0,98-Leu: 1,97-Tyr: 1,00-Lys: 0,98-Arg: 0,99-HPLC en phase reverse (EPP: 92%).

3°) H-Tyr-Arg-Lys(Z)-Val-Leu-Gly-Gln-Leu-Ser-Arg-$OCH_3$, HCl, TFA

8,66 g (6,1 mM) du produit ci-dessus sont agités 35 min dans 37 ml de dichlorométhane+3,5 ml d'anisole+40 ml de TFA. Après concentration sous vide de moitié, le résidu est versé sur 300 ml d'éther glacé; le précipité blanc obtenu est filtré, lavé à l'éther puis séché sous vide sur potasse.

Poids: 8,68 g.

Rendement: 100%.

4°) Boc-Ile-Phe-Thr-Asn-Ser-$N_3$

5,94 g (8,55 mM) de Boc-Ile-Phe-Thr-Asn-Ser-NH-$NH_2$ sont dissous dans 27 ml de DMSO+37 ml de DMF. Après refroidissement à −25°C, on ajoute 6,1 ml d'une solution HCl/dioxanne 5,6 N puis 1,23 ml de nitrite de tertiobutyl (1,2 équivalent) prédilué dans 5 ml de DMF prérefroidi à −20°C. Après 1h15 min d'agitation entre −20° et −25°C, on ajoute 6,1 ml de diisopropyléthylamine (DIPEA) pour amener le pH vers 6, puis on ajoute 3 ml de DMF pour amener le volume à 85 ml et on conserve cette solution à −25°C.

5°) Boc-Ile-Phe-Thr-Asn-Ser-Tyr-Arg-Lys(Z)-Val-Leu-Gly-Gln-Leu-Ser-Ala-$OCH_3$, HCl

Le sel de TFA obtenu à l'exemple XI-3°), dissous dans 70 ml de DMF, est neutralisé par de la DIPEA puis ajouté en 15 min à 70 ml de la solution d'azide obtenue ci-dessus à −20°C. Puis on ajoute de la DIPEA pour amener le pH vers 7, on agite 1 h à −20°C, puis on conserve le milieu à −12°C. Après 22 h, on ajoute 5 ml de la solution d'azide et remonte le pH vers 7 par DIPEA. Après 45 h, on rajoute le restant de la solution d'azide et remonte le pH vers 7 par DIPEA. Après 74 h, le milieu réactionnel est versé sur 1 200 ml d'acétate d'éthyle. Le précipité blanc obtenu est filtré, lavé à l'acétate d'éthyle puis à l'éther, puis séché sous vide.

Poids: 10,46 g.

Rendement: 86%.

Par concentration des eaux mères et reprécipitation par un mélange acétate d'éthyle-éther, on obtient un 2e jet qui permet d'avoir un rendement global de 95%. Le produit de couplage est identifié par 1H RMN et AAA: Asp: 1,02-Thr: 1,03-Ser: 1,91-Glu: 0,97-Gly: 1,06-Ala: 1,06-Val: 1,00-Ile: 0,94-Leu: 1,98-Tyr: 1,04-Phe: 1,02-Lys: 0,94-Arg: 1,02-CCM Rf: 0,20 ($BEW_1$)-HPLC en phase reverse (EPP: 90%).

6°) Boc-Ile-Phe-Thr-Asn-Ser-Tyr-Arg-Lys(Z)-Val-Gly-Gln-Leu-Gly-Gln-Leu-Ser-Ala-OH, HCl

5,3 g (2,7 mM) du produit obtenu ci-dessus sont dissous dans 40 ml de DMSO+6 ml d'eau; on ajoute goutte à goutte 10 ml de soude 1N, on agite 30 min puis neutralise par 10 ml d'acide chlorhydrique 1N. Le milieu réactionnel est versé sur un litre d'acétate d'éthyle. Le précipité obtenu est filtré, lavé 4 fois à l'acétate d'éthyle puis 2 fois à l'eau, puis 4 fois à l'éther, puis il est séché sous vide sur $P_2O_5$.

Poids: 4,56 g.

Rendement: 86%-Produit identifié par 1H RMN et AAA-CCM: Rf: 0,5 $BEW_1$.

Exemple XIII: Synthèse de: Z-Tyr-Ala-Asp-OBzl)-Ala-Ile-Phe-Thr-Asn-Ser-Tyr-Arg-Lys(Z)-Val-Leu-Gly-Gln-Leu-Ser-Ala-Arg-Lys(Z)-Leu-Leu-Gln-Asp(OBzl)-Ile-Met-Ser-Arg-Gln-Gln-Gly(OBzl)-Ser-Asn-Gln-Glu(OBzl)-Arg-Gly-Ala-Arg-Ala-Arg-Leu-$NH_2$, HCl

(GRF 1—44 protégé)

1°) H-Arg-Lys(Z)-Leu-Leu-Gln-Asp(OBzl)-Ile-Met-Ser-Arg-Gln-Gln-Gly-Glu(OBzl)-Ser-Asn-Gln-Glu(OBzl)-Arg-Gly-Ala-Arg-Ala-Arg-Leu-$NH_2$, HCl, TFA

1,4 g du produit de l'exemple XI-11°) sont agités 35 min dans 60 ml de $CH_2Cl_2$, +70 ml de TFA+7 ml d'éthanedithiol. Après concentration sous vide de moitié, le résidu est versé sur 500 ml d'éther glace. Le précipité obtenu est filtré, lavé à l'éther, puis séché sous vide sur potasse.

Poids: 7,44 g.

2°) Boc-Ile-Phe-Thr-Asn-Ser-Tyr-Arg-Lys(Z)-Val-Leu-Gly-Gln-Leu-Ser-Ala-Arg-Lys(Z)-Leu-Leu-Gln-Asp(OBzl)-Ile-Met-Ser-Arg-Gln-Gln-Gly-Glu(OBzl)-Ser-Asn-Gln-Glu(OBzl)-Arg-Gly-Ala-Arg-Ala-Arg-Leu-$NH_2$, HCl

A 6,6 g de sel de TFA ci-dessus (1,9 mM) dissous dans 36 ml de DMSO+90 ml de DMF et neutralisé par de la NEM, on ajoute 1,0 g de BOP (2,3 mM), puis 3,8 g du peptide de l'exemple XI-6°) (1,9 mM), le pH du milieu réactionnel est amené à 6 par de la NEM. Après 19 h, on ajoute 1,15 mM de BOP. Après 26 h, le milieu réactionnel est versé sur 600 ml d'AcOEt. Le précipité obtenu est filtré, lavé par AcOEt, puis filtré et séché sous vide.

Rendement: 88% (9,2 g).

Produit identifié par RMN et AAA.

3°) H-Ile-Phe-Thr-Asn-Ser-Tyr-Arg-Lys(Z)-Val-Leu-Gly-Gln-Leu-Ser-Ala-Arg-Lys(Z)-Leu-Leu-Gln-Asp(OBzl)-Ile-Met-Ser-Arg-Gln-Gln-Gly-Glu(OBzl)-Ser-Asn-Gln-Glu(OBzl)-Arg-Gly-Ala-Arg-Ala-Arg-Leu-$NH_2$, HCl, TFA

9,1 g du produit ci-dessus sont agités 35 min dans 100 ml de TFA+5 ml d'éthanedithiol. Puis le milieu réactionnel est versé sur 500 ml d'éther glacé. Le précipité obtenu est filtré, lavé par $Et_2O$, puis séché sous vide sur potasse.

Rendement: 100%.

4°) GRF-1—44 protégé

A 9,10 g du précédent sel de TFA (1,66 mM) en solution dans 50 ml de DMF et 50 ml de DMSO et neutralisé par la NEM, on ajoute 1,18 g du peptide décrit dans l'exemple X-3°) (1,78 mM) et 0,79 g de BOP (1,78 mM); le pH est maintenu vers 6 par de la NEM. Après 23 h, à TA, le milieu réactionnel est versé sur 600 ml d'AcOET. Le précipité formé est filtré, lavé à l'acétate d'éthyle, puis à l'éther et finalement séché sous vide.

Rendement: 8,54 g (84%).

Contrôle RMN et CMM: AAA: Asp: 4,2-Thr: 0,92-Ser: 4,02-Glu: 7,37 (pour 7)-Gly: 3,14 (pour 3)-Ala: 5,11-Val: 1,01-Met: 0,61-Ile: 1,84-Leu: 4,98-Tyr: 1,87-Phe: 0,95-Arg: 5,90.

Exemple XIV: Déprotection de GRF-1—44:

4,5 g du produit obtenu selon l'exemple XIII-4°) sont agités 1 h à 0° dans 180 ml de TFA contenant 19,8 ml de TFMSA, 27 ml de thioanisole et 11 ml de métacrésol. On ajoute ensuite 1 l d'éther. Le précipité obtenu est filtré, lavé à l'éther puis séché 1 h sous vide en présence de potasse. Le produit est redissous dans 200 ml d'eau et on ajoute de la résine échangeuse d'ions Amberlite IR 45 (sous forme acétate) afin de ramener le pH à 4,5. On agite le milieu 30 min à TA. La résine est filtrée, lavée à l'eau et les filtrats concentrès à 50 ml puis lyophilisés.

Rendement: 3,85 g.

Contrôle par RMN de la disparition des groupements protecteurs Z et OBzl. CCM (BEPW1): tache majoritaire Rf 0,38.

Exemple XV: Purification du GRF-1—44

La peptide déprotégé obtenu dans l'exemple précédent (XIV) est soumis à une chromatographie sur gel de Séphadex G 50 (fine) en utilisant l'acide acétique à 30% comme éluant. Les fractions contenant le peptide attendu sont réunies, évaporées et lyophilisées. Le lyophilisat ainsi obtenu est purifié à son tour par une chromatographie sur un échangeur de cations du type CM-32 carboxyméthylcellulose (Whatman) en utilisant un gradient linéaire d'acétate d'ammonium compris entre 0,1 M (pH 4,5) et 0,4 M (pH 6,5). Par exemple, pour 1 charge de 1 g de peptides à purifier, on utilisera une colonne à chromatographie ayant un volume de lit de 50 ml environ pour une hauteur de 20 cm. Les fractions contenant le peptide avec un degré de pureté ≥à 80% (HPLC) sont réunies et lyophilisées juqu'à poids constant (afin d'éliminer le tampon $CH_3CO_2NH_4$). Enfine, le précédent lyophilisat est soumis à une chromatographie de partition en utilisant comme support de la phase liquide stationaire le Séphadex G 50 fine et à l'aide du système de solvants suivant: n-butanol/éthanol/pyridine/acide acétique 0,2 N dans la proportion de: 4/1/1/7 (en volumes). Les fractions de chromatographie sont contrôlées par HPLC et celles dont le titre de pureté est ≥à 95% sont réunies et lyophilisées.

Le produit est finalement contrôlé par analyse des amino-acides:

| | |
|---|---|
| Tyr: 1,91 (2) | Arg: 5,89 (6) |
| Ala: 4,88 (5) | Lys: 1,98 (2) |
| Asn, Asp: 3,86 (4) | Val: 1,01 (1) |
| Ile: 1,96 (2) | Leu: 5,10 (5) |
| Phe: 0,93 (1) | Gly: 2,98 (3) |
| Thr: 1,02 (1) | Gln, Glu: 6,82 (7) |
| Ser: 3,88 (4) | Met: 0,91 (1) |

Exemple XVI—Synthèse de Boc-Glu-Ser-Asn-Gln-Glu-Arg-Gly (fragment B)

1°) Z-Arg($NO_2$)-Glu-OBzl

353 g de tosylate de glycine benzyl ester (H-Gly-OBzl, tosylate) sont dissous dans 2 l de DMF, on ajoute 115 g de NEM puis 353 g de Z-Arg($NO_2$)-OH et enfin 500 g de BOP. Le pH du milieu est ajusté à 7 à l'aide de NEM et en utilisant du papier indicateur de pH sur des prélèvements du milieu réactionnel dilués à l'eau. Le milieu réactionnel est agité et l'état d'avancement de la réaction suivi par C.C.M. Au bout de 4 h, la réaction est complète et ls milieu évaporé à sec sous vide à 35°. Le résidu d'évaporation est repris par 2 l d'acétate d'éthyle et 2 l d'eau. On obtient dans ces conditions un solide qui est essoré et lavé successivement en phase solide avec une solution aqueuse à 5% de $KHSO_4+K_2SO_4$, à l'eau pure, avec une solution aqueuse à 5% de $NaHCO_3$ et finalement à l'eau.

Le produit est séché à l'air et contrôle par R.M.N. et C.C.M.

Rendement: 410 g (82%).

2°) H-Glu(OBzl)-Arg-Gly-OH

500 g du précédent produit en solution dans 3 l de HClN dans le DMF et en présence de 100 g de Pd/C à 10% en Pd sont hydrogénés sous une pression de 1,5 bar d'hydrogène. Après 24 h, la réaction est terminée (contrôle par C.C.M.). Le catalyseur est filtré et le pH du milieu ajusté à 7 par addition de N-éthyl morpholine et en utilisant du papier indicateur de pH sur des prélèvements dilués à l'eau. On introduit 458 g de Boc-Glu(OBzl)-ONp en solution dans 1 litre de DMF. Le milieu réactionnel est dilué par addition de 1 litre d'eau et on ajoute progressivement 135 g d'OHBT et de la NEM en quantités suffisantes pour maintenir le pH à 7.

Le milieu est agité 4 h à la température ambiante. On s'assure que la réaction est complète et le solvant est évaporé sous vide à la température de 35°. Le résidu d'évaporation est repris dans l'acétate d'éthyle. Dans ces conditions, le produit s'organise en solide. Il est essoré et chromatographié sur gel de silice en utilisant comme éluant le chloroforme-méthanol (70—30 en volume). L'évaporation des fractions de chromatographie contenant le produit pur conduit à 412 g (75%) d'un composé pulvérulent blanc après lyophilisation. Il est contrôlé par C.C.M. et R.M.N.

Le précédent produit est dissous à la température ambiante dans 3 l d'un mélange (50/50) de TFA et de $CH_2Cl_2$. On maintient le milieu 15 min à la température ambiante, et on évapore à sec sous vide, en maintenant la température à 20°. Le résidu est repris dans l'éther éthylique et le solide obtenu est essoré et séché. Le rendement est de 423 g (100%) sous forme de trifluoroacétate.

3°) H-Gln-Glu(OBzl)-Arg-Gly-OH

564 g de trifluoroacétate de H-Glu(OBzl)-Arg-Gly-OH sont dissous dans 3 l de diméthylformamide. Le pH est ajusté à 7 par addition de NEM sur des prélèvement dilués à l'eau et à l'aide de papier indicateur de pH.

On introduit ensuite 367 g de Boc-Gln-ONp en solution dans 1 litre de DMF. Le milieu réactionnel est dilué par addition de 1 litre d'eau. On ajoute progressivement 135 g d'OHBT et la quantité suffisante de NEM pour que le pH se maintienne à 7.

Le mélange est agité 4 h à la température ambiante. On contrôle la fin de la réaction par C.C.M. et le solvant est évaporé sous vide poussé à 35°. Le résidu d'évaporation est trituré dans l'acétate d'éthyle. On obtient dans ces conditions un solide hygroscopique qui est utilisé tel quel pour le traitement à l'acide trifluoroacétique-$CH_2Cl_2$ (2 l du mélange 50—50 en volume).

Le milieu est conservé 10 min à la température ambiante et enfin évaporé sous vide à 20°. Le résidu d'évaporation est trituré dans l'éther éthylique pour donner 525 g (76%) de H-Gln-Glu(OBzl)-Arg-Gly-OH. (Contrôlé par R.M.N. et C.C.M.).

4°) H-Asn-Gln-Glu(OBz)-Arg-Gly-OH

En utilisant rigoureusement les conditions de l'exemple II-2 et à partir de H-Gln-Glu(OBzl)-Arg-Gly-OH, trifluoroacétate (691 g) et 393 g de Boc-Asn-ONb, on obtient 685 g (85%) d'un produit solide blanc contrôlé par R.M.N. et C.C.M.

5°) H-Ser-Asn-Gln-Glu(OBzl)-Arg-Gly-OH

Egalement dans les conditions de l'exemple II-2 et à partir de 806 g de H-Asn-Gln-Glu(OBzl)-Arg-Gly-OH et de 366 g de Boc-Ser-ONb, on obtient le produit contrôlé par R.M.N. et C.C.M. avec un rendement de 688 g (77%) et sous forme trifluoroacétate.

6°) Boc-Glu(OBzl)-Ser-Asn-Gln-Glu(OBzl)-Arg-Gly-OH (fragment B)

A partir de 894 g de H-Ser-Asn-Gln-Glu(OBzl)-Arg-Gly-OH trifluoroacétate et 458 g de Boc-Glu(OBzl)-ONp, on obtient 824 g (80%) du produit attendu après purification par cristallisation dans le milieu DMF-éther (50—50 en volume).

Le produit est contrôlé par R.M.N., C.C.M. et analyse d'amino acides:

| | |
|---|---|
| Gln—Glu: | 2,92 (3) |
| Ser: | 0,97 (1) |
| Asn: | 1,03 (1) |
| Arg: | 0,97 (1) |
| Gly: | 0,96 (1) |

En suivant les stratégies présentées dans les tableaux V, VI, VII et VIII et les techniques décrites dans les exemples I à IX et XVI, les composés suivants peuvent être obtenus:

| | |
|---|---|
| Boc-Lys(Z)-Leu-Leu-Gln-OH | (fragment E) |
| Boc-Gln-Leu-Ser-Ala-Arg-OH | (fragment F) |
| Boc-Lys(Z)-Val-Leu-Gly-OH | (fragment G) |
| Boc-Ile-Phe-Thr-Asn-Ser-Tyr-Arg-OH | (fragment H) |

Exemple XVII: Boc-Glu(OBzl)-Ser-Asn-Gln-Glu(OBzl)-Arg-Gly-Ala-Arg-Ala-Arg-Leu-$NH_2$ (fragment B-A)

69,2 g du fragment A chlorhydrate sont dissous dans 500 ml de DMF. On ajuste le pH à 7 sur des

prélèvements dilués à l'eau et à l'aide de papier indicateur de pH et de NEM. On ajoute ensuite 117 g du fragment B, 55 g de BOP, on ajuste à nouveau le pH à 7 à l'aide de NEM et le milieu est agité pendant 14 h à la température ambiante. Après contrôle de fin de réaction par C.C.M., on ajoute au milieu réactionnel 2 l d'éther éthylique. Un précipité pulvérulent blanc se dépose dans ces conditions. Il est purifié par recristallisation dans le DMF-acétate d'éthyle. Le produit essoré est ensuite lavé à l'acétate d'éthyle en phase solide et séché.

On obtient 149 g (81%) d'un produit blanc contrôlé par C.C.M. et R.M.N.

Analyse d'amino acides:

Gln-Glu: 2,92 (3)
Ser: 1,02 (1)
Asn: 0,97 (1)
Arg: 2,89 (3)
Gly: 1,01 (1)
Ala: 1,98 (2)
Leu: 0,93 (1)

Déprotection de l'azote N-terminal

Le produit décrit dans le précédent exemple (184 g) est introduit dans un mélange constitué par 750 ml de TFA et 750 ml de $CH_2Cl_2$ sous agitation et à la température ambiante. Après dissolution, le mélange est conservé 15 min à la même température et évaporé sous vide en maintenant le milieu d'évaporation à 25°. Le résidu d'évaporation est trituré dans l'éther et l'on obtient dans ces conditions un solide pulvérulent blanc qui est séche sous vide et contrôlé par C.C.M. et R.M.N.

Les composés suivants pouvant être obtenus selon les techniques de l'exemple XVII et représentant les différents stades de l'élongation du peptide.

Fragment: C-B-A

Boc-Ser-Arg-Gln-Gln-Gly-Glu(OBzl)-Ser-Asn-Gln-Glu-Arg-Gly-Ala-Arg-Ala-Arg-Leu-$NH_2$
(H)

Fragment: D-C-B-A

OBzl
|
Boc-Asp-Ile-Met-Ser-Arg-Gln-Gln-Gly-Glu(OBzl)-Ser-Asn-Gln-Glu(OBzl)-Arg-Gly-Ala-Arg-Ala-Arg-Leu-$NH_2$
(H)

Dans ce cas particulier, le fragment D est introduit en utilisant l'activation aux azides

$$(-\underset{\underset{O}{\|}}{C}N_3).$$

Dès que la méthionine est en séquence, un balayeur "scavenger" sera utilisé au cours des différents traitements par le TFA-$CH_2Cl_2$ pendant les phases de déprotection sélective (élimination du Boc). Nous préconisons le thioanisole comme agent de protection de la méthionine à l'oxydation dans la proportion de 5% dans le milieu TFA-$CH_2Cl_2$.

Fragment: E-D-C-B-A

Boc-Lys(Z)-Leu-Leu-Gln-Asp(OBzl)-Ile-Met-Ser-Arg-Gln-Gln-Gly-Glu(OBzl)-Ser-Asn-Gln-Glu(OBzl)-Arg-
(H)
Gly-Ala-Arg-Ala-Arg-Leu-$NH_2$

Fragment: F-E-D-C-B-A

Boc-Gln-Leu-Ser-Ala-Arg-Lys(Z)-Leu-Leu-Gln-Asp(OBzl)-Ile-Met-Ser-Arg-Gln-Gln-Gly-Glu(OBzl)-Ser-
(H)
Asn-Gln-Glu(OBzl)-Arg-Gly-Ala-Arg-Ala-Arg-Leu-$NH_2$

Fragment: G-F-E-D-C-B-A

Boc-Lys(Z)-Val-Leu-Gly-Gln-Leu-Ser-Ala-Arg-Lys(Z)-Leu-Leu-Ser-Asp(OBzl)-Ile-Met-Ser-Arg-Gln-Gln-Gly-Glu(OBzl)-Ser-Asn-Gln-Glu(OBzl)-Arg-Gly-Ala-Arg-Ala-Arg-Leu-$NH_2$

Fragment: H-G-F-E-D-C-B-A

Boc-Ile-Phe-Thr-Asn-Ser-Tyr-Arg-Lys(Z)-Val-Leu-Gly-Gln-Leu-Ser-Ala-Arg-Lys(Z)-Leu-Leu-Gln-
(H)
Asp(OBzl)-Ile-Met-Ser-Arg-Gln-Gln-Gly-Glu(OBzl)-Ser-Asn-Gln-Glu(OBzl)-Arg-Gly-Ala-Arg-Ala-Arg-Leu-$NH_2$

hpGRF protégé

Z-Tyr-Ala-Asp(OBzl)-Ala-Ile-Phe-Thr-Asn-Ser-Tyr-Arg-Lys(Z)-Val-Leu-Gly-Gln-Leu-Ser-Ala-Arg-Lys(Z)-Leu-Leu-Gln-Asp(OBzl)-Ile-Met-Ser-Arg-Gln-Gln-Gly-Glu(OBzl)-Ser-Asn-Gln-Glu(OBzl)-Arg-Gly-Ala-Arg-Ala-Arg-Leu-$NH_2$

La déprotection et la purification du hpGRF 1—44 sont ensuite effectuées comme indiqué dans les exemples XIV et XV.

**Revendications pour les Etats Contractants: BE, CH, DE, FR, GB, IT, LI, LU, NL, SE**

1. Procédé de synthèse, en phase liquide et par fragments, des hpGRF 1—44 et hpGRF 1—40, caractérisé en ce que l'on couple, l'un après l'autre et dans l'ordre de la séquence du GRF, les fragments dans lesquels:

a) les fonctions acides latérales des acides aspartique et glutamique et la fonction amine latérale de la lysine sont protégées par des groupes protecteurs stables dans les conditions de déprotection du groupement Boc,

b) la fonction guanidine de l'arginine est protégée par protonation, et

c) l'acide aminé N-terminal est protégé sur l'amine par le groupement Boc, en ce qu'on élimine sélectivement le groupement Boc de l'amine N-terminale du peptide en phase d'élongation par hydrolyse à l'acide trifluoroacétique, ledit couplage étant effectué dans un solvant polaire aprotique et en ce qu'on élimine en fin de séquence tous les groupes protecteurs par hydrolyse à l'aide d'une solution 0,1 à 1 M d'acide méthanesulfonique ou trifluorométhane-sulfonique dans l'acide trifluoroacétique.

2. Procédé selon la revendication 1, caractérisé en ce qu'après chaque couplage le produit obtenu est isolé du milieu réactionnel par précipitation à l'aide d'un solvant apolaire.

3. Procédé selon la revendication 1, caractérisé en ce que le produit brut de réaction est purifié par la distribution à contre-courant et la chromatographie de perméation sur gel.

4. Procédé selon l'une quelconque des revendications 1 à 3, caractérisé en ce qu'il consiste à coupler successivement et dans l'ordre de la séquence du GRF les neuf fragments peptidiques ci-après:

H-Ala-Arg-Ala-Arg-Leu-$NH_2$ (40—44) dit fragment A,
Boc-Glu(OBzl)-Ser-Asn-Gln-Glu(OBzl)-Arg-Gly-OH (33—39) dit fragment B,
Boc-Ser-Arg-Gln-Gln-Gly-OH (28—32) dit fragment C,
Boc-Asp(OBzl)-Ile-Met-NH-$NH_2$ (25—27) dit fragment D,
Boc-Lys(Z)-Leu-Leu-Gln-OH (21—24) dit fragment E,
Boc-Gln-Leu-Ser-Ala-Arg-OH (16—20) dit fragment F,
Boc-Lys(Z)-Val-Leu-Gly-OH (12—15) dit fragment G,
Boc-Ile-Phe-Thr-Asn-Ser-Tyr-Arg-OH (5—11) dit fragment H,
Z-Tyr-Ala-Asp(OBzl)-Ala-OH (1—4) dit fragment I,

et en ce que le peptide obtenu est le hpGRF 1—44.

5. Procédé selon l'une quelconque des revendications 1 à 4, caractérisé en ce que l'on utilise l'alaninamide à la place du fragment A et en ce que le peptide obtenu est le hpGRF 1—40.

6. Procédé selon l'une quelconque des revendications 1 à 3, caractérisé en ce que l'on couple successivement et dans l'ordre de la séquence du GRF les 3 fragments peptidiques:

H-Arg-Lys(Z)-Leu-Leu-Gln-Asp(OBzl)-Ile-Met-Ser-Arg-Gln-Gln-Gly-Glu(OBzl)-Ser-Asn-Gln-Glu(OBzl)-Arg-Gly-Ala-Arg-Ala-Arg-Leu-$NH_2$ (séquence 20—44 du GRF dit peptide K),

Boc-Ile-Phe-Thr-Asn-Ser-Tyr-Arg-Lys(Z)-Val-Leu-Gly-Gln-Leu-Ser-Ala-OH (séquence 5—19 du GRF dit peptide J),

Z-Tyr-Ala-Asp(OBzl)-Ala-OH (séquence 1—4 du GRF dit peptide I), ledit peptide obtenu étant le hpGRF 1—44.

7. Procédé selon la revendication 6, caractérisé en ce qu l'on utilise le fragment peptidique correspondant à la séquence 20—40 du GRF à la place du peptide K, ledit peptide obtenu étant le hpGRF 1—40.

8. Procédé selon l'une quelconque des revendications 1 à 7, caractérisé en ce que le couplage est réalisé à l'aide de l'hexafluorophosphate de benzotriazolyloxyphosphonium (BOP) ou la dicyclohexylcarbodiimide en présence d'hydroxy-1 benzotriazole (agent de couplage) ou par activation aux carboxyazides, dans un solvant approprié, tel que le diméthylformamide ou le diméthylsulfoxyde.

9. A titre de produits nouveaux, les peptides intermédiaires ou fragments peptidiques A à K mis en oeuvre dans le procédé selon l'une quelconque des revendications 1 à 8.

10. Peptides intermédiaires selon la revendication 9, caractérisé en ce qu'il est le peptide K dont la séquence correspond à la séquence 20—44 du GRF et dont certains amino-acides sont bloqués, obtenu par couplage successif en phase liquide des sous-fragments ci-après dans l'ordre de la séquence du GRF:

H-Ala-Arg-Ala-Arg-Leu-$NH_2$ (40—44) dit fragment A,
Boc-Glu(OBzl)-Arg-Gly-OH (37—39) dit fragment $B_1$,
Boc-Glu(OBzl)-Ser-Asn-Gln-OH (33—36) dit fragment $B_2$,
Boc-Ser-Arg-Gln-Gln-Gly-OH (28—32) dit fragment C,
Boc-Asp(OBzl)-Ile-Met-NH-$NH_2$ (25—27) dit fragment D,
Boc-Gln-ONp,
Boc-Arg-Lys(Z)-Leu-Leu-OH (20—23) dit fragment $E_1$ dans lesquels:

a) les fonctions acides latérales des acides aspartique et glutamique et la fonction amine latérale de la lysine sont protégées par des groupes protecteurs stables dans les conditions de déprotection du groupement Boc,

b) la fonction guanidine de l'arginine est protégée par la protonation, et

c) l'acide aminé N-terminal des fragments $B_1$, $B_2$, C, D, $E_1$ est protégé sur l'amine par le groupement Boc éliminable sélectivement en phase d'élongation du peptide par hydrolyse à l'acide trifluoroacétique, les couplages étant effectués dans un solvant polaire aprotique.

11. Peptide intermédiaire selon la revendication 8, caractérisé en ce qu'il est le fragment peptidique ayant la séquence 20—40 du GRF, obtenu par couplage successif en phase liquide des sous-fragments ci-après dans l'ordre de la séquence du GRF:

H-Ala-$NH_2$,
Boc-Glu(OBzl)-Arg-Gly-OH (37—39) dit fragment $B_1$,
Boc-Glu(OBzl)-Ser-Asn-Gln-OH (33—36) dit fragment $B_2$,
Boc-Ser-Arg-Gln-Gln-Gly-OH (28—32) dit fragment C,
Boc-Asp(OBzl)-Ile-Met-NH-$NH_2$ (25—27) dit fragment D,
Boc-Gln-ONp,
Boc-Arg-Lys(Z)-Leu-Leu-OH (20—23) dit fragment $E_1$ dans lesquels:

a) les fonctions acides latérales des acides aspartique et glutamique et la fonction amine latérale de la lysine sont protegées par des groupes protecteurs stables dans les conditions de déprotection du groupement Boc,

b) la fonction guanidine de l'arginine est protégé par la protonation, et

c) l'acide aminé N-terminal des fragments $B_1$, $B_2$, C, D, E est protégé sur l'amine par le groupement Boc éliminable sélectivement en phase d'élongation du peptide par hydrolyse à l'acide trifluoroacétique, les couplages étant effectués dans un solvant polaire aprotique.

12. Peptide intermédiaire selon la revendication 9, caractérisé en ce qu'il est le peptide J obtenu par couplage successif en phase liquide des sous-fragments suivants dans l'ordre de la séquence du GRF,

H-Gln-Leu-Ser-Ala-$OCH_3$ (16—19) dit fragment $F_1$,
Boc-Tyr-Arg-Lys(Z)-Val-Leu-Gln-OH (10—15) dit fragment $G_1$,
Boc-Ile-Phe-Thr-Asn-Ser-NH-$NH_2$ (5—9) dit fragment $H_1$,

dans lesquels:

a) la fonction amine latérale de la lysine du fragment G est protégée par un groupement protecteur stable dans les conditions de déprotection du groupement Boc,

b) la fonction guanidine du fragment 10—15 est protégé par protonation,

c) l'acide aminé N-terminal des fragments G et H est protégé sur l'amine par le groupement Boc, éliminable sélectivement en phase d'élongation du peptide par l'hydrolyse à l'acide trifluoroacétique, les couplages étant effectués dans un solvant polaire aprotique.

13. Peptide intermédiaire selon la revendication 9, caractérisé en ce qu'il est le peptide I obtenu par synthèse en phase liquide dans laquelle:

a) la fonction latérale acide de l'acide aspartique est protégée par un groupe protecteur stable dans les conditions de déprotection du groupement Boc,

b) l'acide alpha aminé N-terminal est protégé sur l'amine par un groupement clivable dans les conditions de déprotection des groupements protecteurs des chaînes latérales.

**Revendications pour l'état Contractant: AT**

1. Procédé de synthèse, en phase liquide et par fragments, des hpGRF 1—44 et hpGRF 1—40, caractérisé en ce que l'on couple, l'un après l'autre et dans l'ordre de la séquence du GRF, les fragments dans lesquels:

a) les fonctions acides latérales des acides aspartique et glutamique et la fonction amine latérale de la lysine sont protégées par des groupes protecteurs stables dans les conditions de déprotection du groupement Boc,

b) la fonction guanidine de l'arginine est protégée par protonation, et

c) l'acide aminé N-terminal est protégé sur l'amine par le groupement Boc, en ce qu'on élimine sélectivement le groupement Boc de l'amine N-terminale du peptide en phase d'élongation par hydrolyse à l'acide trifluoroacétique, ledit couplage étant effectué dans un solvant polaire aprotique et en ce qu'on élimine en fin de séquence tous les groupes protecteurs par hydrolyse à l'aide d'une solution 0,1 à 1 M d'acide méthanesulfonique ou trifluorométhanesulfonique dans l'acide trifluoroacétique.

2. Procédé selon la revendication 1, caractérisé en ce qu'après chaque couplage le produit obtenu est isolé du milieu réactionnel par précipitation à l'aide d'un solvant apolaire.

3. Procédé selon la revendication 1, caractérisé en ce que le produit brut de réaction est purifié par la distribution à contre-courant et la chromatographie de perméation sur gel.

4. Procédé selon l'une quelconque des revendications 1 à 3, caractérisé en ce qu'il consiste à coupler successivement et dans l'ordre de la séquence du GRF les neuf fragments peptidiques ci-après:

H-Ala-Arg-Ala-Arg-Leu-$NH_2$ (40—44) dit fragment A,
Boc-Glu(OBzl)-Ser-Asn-Gln-Glu(OBzl)-Arg-Gly-OH (33—39) dit fragment B,

Boc-Ser-Arg-Gln-Gln-Gly-OH (28—32) dit fragment C,
Boc-Asp(OBzl)-Ile-Met-NH-$NH_2$ (25—27) dit fragment D,
Boc-Lys(Z)-Leu-Leu-Gln-OH (21—24) dit fragment E,
Boc-Gln-Leu-Ser-Ala-Arg-OH (16—20) dit fragment F,
Boc-Lys(Z)-Val-Leu-Gly-OH (12—15) dit fragment G,
Boc-Ile-Phe-Thr-Asn-Ser-Tyr-Arg-OH (5—11) dit fragment H,
Z-Tyr-Ala-Asp(OBzl)-Ala-OH (1—4) dit fragment I,
et en ce que le peptide obtenu est le hpGRF 1—44.

5. Procédé selon l'une quelconque des revendications 1 à 4, caractérisé en ce que l'on utilise l'alaninamide à la place du fragment A et en ce que le peptide obtenu est le hpGRF 1—40.

6. Procédé selon l'une quelconque des revendications 1 à 3, caractérisé en ce que l'on couple successivement et dans l'ordre de la séquence du GRF les 3 fragments peptidiques:

H-Arg-Lys(Z)-Leu-Leu-Gln-Asp(OBzl)-Ile-Met-Ser-Arg-Gln-Gln-Gly-Glu(OBzl)-Ser-Asn-Gln-Glu(OBzl)-Arg-Gly-Ala-Arg-Ala-Arg-Leu-$NH_2$ (séquence 20—44 du GRF dit peptide K),

Boc-Ile-Phe-Thr-Asn-Ser-Tyr-Arg-Lys(Z)-Val-Leu-Gly-Gln-Leu-Ser-Ala-OH (séquence 5—19 du GRF dit peptide J),

Z-Tyr-Ala-Asp(OBz)-Ala-OH (séquence 1—4 du GRF dit peptide I), ledit peptide obtenu étant le hpGRF 1—44.

7. Procédé selon la revendication 6, caractérisé en ce que l'on utilise le fragment peptidique correspondant à la séquence 20—40 du GRF à la place du peptide K, ledit peptide obtenu étant le hpGRF 1—40.

8. Procédé selon l'une quelconque des revendications 1 à 7, caractérisé en ce que le couplage est réalisé à l'aide de l'hexafluorophosphate de benzotriazolyloxyphosphonium (BOP) ou la dicyclohexylcarbodiimide en présence d'hydroxy-1 benzotriazole (agent de couplage) ou par activation aux carboxyazides, dans un solvant approprié, tel que le diméthylformamide ou le diméthylsulfoxyde.

9. Procédé de synthèse du peptide intermédiaire K dont la séquence correspond à la séquence 20—44 du GRF et dont certains amino-acides sont bloqués, caractérisé en ce qu'il consiste à coupler successivement en phase liquide les sous-fragments ci-après dans l'ordre de la séquence du GRF:
H-Ala-Arg-Ala-Arg-Leu-$NH_2$ (40—44) dit fragment A,
Boc-Glu(OBzl)-Arg-Gly-OH (37—39) dit fragment $B_1$,
Boc-Glu(OBzl)-Ser-Asn-Gln-OH (33—36) dit fragment $B_2$,
Boc-Ser-Arg-Gln-Gln-Gly-OH (28—32) dit fragment C,
Boc-Asp(OBzl)-Ile-Met-NH-$NH_2$ (25—27) dit fragment D,
Boc-Gln-ONp,
Boc-Arg-Lys(Z)-Leu-Leu-OH (20—23) dit fragment $E_1$ dans lesquels:

a) les fonctions acides latérales des acides aspartique et glutamique et la fonction amine latérale de la lysine sont protégées par des groupes protecteurs stables dans les conditions de déprotection du groupement Boc,

b) la fonction guanidine de l'arginine est protégée par la protonation, et

c) l'acide aminé N-terminal des fragments $B_1$, $B_2$, C, D, $E_1$ est protégé sur l'amine par le groupement Boc éliminable sélectivement en phase d'élongation du peptide par hydrolyse à l'acide trifluoroacétique, les couplages étant effectués dans un solvant polaire aprotique.

10. Procédé de synthèse du peptide intermédiaire ayant la séquence 20—40 du GRF, caractérisé en ce qu'il consiste à coupler successivement en phase liquide les sous-fragments ci-après dans l'ordre de la séquence du GRF:
H-Ala-$NH_2$,
Boc-Glu(OBzl)-Arg-Gly-OH (37—39) dit fragment $B_1$,
Boc-Glu(OBzl)-Ser-Asn-Gln-OH (33—36) dit fragment $B_2$,
Boc-Ser-Arg-Gln-Gln-Gly-OH (28—32) dit fragment C,
Boc-Asp(OBzl)-Ile-Met-NH-$NH_2$ (25—27) dit fragment D,
Boc-Gln-ONp,
Boc-Arg-Lys(Z)-Leu-Leu-OH (20—23) dit fragment $E_1$ dans lesquels:

a) les fonctions acides latérales des acides aspartique et glutamique et la fonction amine latérale de la lysine sont protégées par des groupes protecteurs stables dans les conditions de déprotection du groupement Boc,

b) la fonction guanidine de l'arginine est protégée par la protonation, et

c) l'acide aminé N-terminal des fragments $B_1$, $B_2$, C, D, E est protégé sur l'amine par le groupement Boc éliminable sélectivement en phase d'élongation du peptide par hydrolyse à l'acide trifluoroacétique, les couplages étant effectués dans un solvant polaire aprotique.

11. Procédé de synthèse du peptide intermédiaire J, caractérisé en ce qu'il consiste à coupler successivement en phase liquide les sous-fragments suivants dans l'ordre de la séquence du GRF,
H-Gln-Leu-Ser-Ala-$OCH_3$ (15—19) dit fragment $F_1$,
Boc-Tyr-Arg-Lys(Z)-Val-Leu-Gly-OH (10—15) dit fragment $G_1$,
Boc-Ile-Phe-Thr-Asn-Ser-NH-$NH_2$ (5—9) dit fragment $H_1$, dans lesquels:

a) la fonction amine latérale de la lysine du fragment G est protégée par un groupement protecteur stable dans les conditions de déprotection du groupement Boc,

b) la fonction guanidine du fragment 10—15 est protégée sur protonation,

c) l'acide aminé N-terminal des fragments G et H est protégé sur l'amine par le groupement Boc, éliminable sélectivement en phase d'élongation du peptide par l'hydrolyse à l'acide trifluoroacétique, les couplages étant effectués dans un solvant polaire aprotique.

12. Procédé de synthèse du peptide intermédiaire I, caractérisé en ce qu'il consiste à coupler en phase liquide les acides aminés le constituant, dans lesquels:

a) la fonction latérale acide de l'acide aspartique est protégée par un groupe protecteur stable dans les conditions de déprotection du groupement Boc,

b) l'acide alpha aminé N-terminal est protégé sur l'amine par un groupement clivable dans les conditions de déprotection des groupements protecteurs des chaînes latérales.

**Patentansprüche für die Vertragsstaaten: BE, CH, DE, FR, GB, IT, LI, LU, NL, SE**

1. Syntheseverfahren für hpGRF 1—44 und hpGRF 1—40 in flüssiger Phase und mittels Fragmenten, dadurch gekennzeichnet, daß man nacheinander und in der Reihenfolge der Sequenz von GRF die Fragmente koppelt, in denen:

a) die seitlichen Säurefunktionen der Asparagin- und Glutaminsäure und die seitliche Aminfunktion von Lysin durch Schutzgruppen geschützt sind, die unter Entschützungsbedingungen für die Gruppierung Boc stabil sind,

b) die Guanidinfunktion von Arginin durch Protonierung geschützt ist, und

c) die N-terminale Aminosäure an der Aminogruppe durch die Gruppierung Boc geschützt ist, daß man selektiv die Gruppierung Boc der N-terminalen Aminogruppe des Peptids in der Verlängerungsphase durch Hydrolyse mit Trifluoressigsäure entfernt, wobei die Kopplung in einem aprotischen polaren Lösungsmittel vorgenommen wird, und daß man am Ende der Sequenz sämtliche Schutzgruppen durch Hydrolyse mit Hilfe einer 0,1 bis 1 M Methansulfon- oder Trifluormethansulfonsäurelösung in Trifluoressigsäure entfernt.

2. Verfahren nach Anspruch 1, dadurch gekennzeichnet, daß nach jeder Kopplung das erhaltene Produkt durch Ausfällen mit Hilfe eines apolaren Lösungsmittels aus dem Reaktionsmilieu isoliert wird.

3. Verfahren nach Anspruch 1, dadurch gekennzeichnet, daß das Reaktions-Rohprodukt durch Gegenstromverteilung und Gelpermeationschromatographie gereinigt wird.

4. Verfahren nach einem der Ansprüche 1 bis 3, dadurch gekennzeichnet, daß es aus der aufeinanderfolgenden Kopplung in der Reihenfolge der Sequenz von GRF folgender neun Peptidfragemente besteht:

H-Ala-Arg-Ala-Arg-Leu-$NH_2$ (40—44), genannt Fragment A,
Boc-Glu(OBzl)-Ser-Asn-Gln-Glu(OBzl)-Arg-Gly-OH (33—39), genannt Fragment B,
Boc-Ser-Arg-Gln-Gln-Gly-OH (28—32), genannt Fragment C,
Boc-Asp(OBzl)-Ile-Met-NH-$NH_2$ (25—27), genannt Fragment D,
Boc-Lys(Z)-Leu-Leu-Gln-OH (21—24), genannt Fragment E,
Boc-Gln-Leu-Ser-Ala-Arg-OH (16—20), genannt Fragment F,
Boc-Lys(Z)-Val-Leu-Gly-OH (12—15), genannt Fragment G,
Boc-Ile-Phe-Thr-Asn-Ser-Tyr-Arg-OH (5—11), genannt Fragment H,
Z-Tyr-Ala-Asp(OBzl)-Ala-OH (1—4), genannt Fragment I,

und daß das erhaltene Peptid hpGRF 1—44 ist.

5. Verfahren nach einem der Ansprüche 1 bis 4, dadurch gekennzeichnet, daß man das Alaninamid anstelle des Fragments A einsetzt und daß das erhaltene Peptid hpGRF 1—40 ist.

6. Verfahren nach einem der Ansprüche 1 bis 3, dadurch gekennzeichnet, daß man nacheinander und in der Reihenfolge der Sequenz von GRF die 3 Peptidfragemente koppelt:

H-Arg-Lys(Z)-Leu-Leu-Gln-Asp(OBzl)-Ile-Met-Ser-Arg-Gln-Gln-Gly-Glu(OBz)-Ser-Asn-Gln-Glu(OBzl)-Arg-Gly-Ala-Arg-Ala-Arg-Leu-$NH_2$ (Sequenz 20—44 von GRF, genannt Peptid K),

Boc-Ile-Phe-Thr-Asn-Ser-Tyr-Arg-Lys(Z)-Val-Leu-Gly-Gln-Leu-Ser-Ala-OH (Sequenz 5—19 von GRF, genannt Peptid J),

Z-Tyr-Ala-Asp(OBzl)-Ala-OH (Sequenz 1—4 von GRF, genannt Peptid I), wobei das erhaltene Peptid hpGRF 1—44 ist.

7. Verfahren nach Anspruch 6, dadurch gekennzeichnet, daß man anstelle des Peptids K das Peptidfragment einsetzt, das der Sequenz 20—40 von GRF entspricht, wobei das erhaltene Peptid hpGRF 1—40 ist.

8. Verfahren nach einem der Ansprüche 1 bis 7, dadurch gekennzeichnet, daß die Kopplung mit Hilfe von Benzotriazolyloxyphosphonium-hexafluorphosphat (BOP) oder Dicyclohexyl-carbodiimid in Gegenwart von 1-Hydroxybenzotriazol (Kopplungsmittel) oder durch Aktivierung mit Carboxyaziden in einem geeigneten Lösungsmittel, wie Dimethylformamid oder Dimethylsulfoxid durchgeführt wird.

9. Als neue Produkte die intermediären Peptide oder Peptidfragmente A bis K, die im Verfahren nach einem der Ansprüche 1 bis 8 verarbeitet werden.

10. Intermediäre Peptide nach Anspruch 9, dadurch gekennzeichnet, daß es das Peptid K ist, dessen

Sequenz der Sequenz 20—44 von GRF entspricht und von dem bestimmte Aminosäuren blockiert sind, welches durch aufeinanderfolgende Kopplung in flüssiger Phase der nachstehenden Subfragmente in der Reihenfolge der Sequenz von GRF erhalten wird:

H-Ala-Arg-Ala-Arg-Leu-$NH_2$ (40—44), genannt Fragment A,
Boc-Glu(OBzl)-Arg-Gly-OH (37—39), genannt Fragment $B_1$,
Boc-Glu(OBzl)-Ser-Asn-Gln-OH (33—36), genannt Fragment $B_2$,
Boc-Ser-Arg-Gln-Gln-Gly-OH (28—32), genannt Fragment C,
Boc-Asp(OBzl)-Ile-Met-NH-$NH_2$ (25—27), genannt Fragment D,
Boc-Gln-ONp,
Boc-Arg-Lys(Z)-Leu-Leu-OH (20—23), genannt Fragment $E_1$, worin:

a) die seitlichen Säurefunktionen der Asparagin- und Glutaminsäure und die seitliche Aminfunktion von Lysin durch Schutzgruppen geschützt sind, die unter Entschützungsbedingungen für die Gruppierung Boc stabil sind,

b) die Guanidinfunktion von Arginin durch Protonierung geschützt ist, und

c) die N-terminale Aminosäure der Fragmente $B_1$, $B_2$, C, D, $E_1$ an der Aminogruppe durch die Gruppierung Boc geschützt ist, die selektiv in der Verlängerungsphase des Peptids durch Hydrolyse mit Trifluoressigsäure entfernbar ist, wobei die Kopplungen in einem aprotischen polaren Lösungsmittel vorgenommen werden.

11. Intermediäres Peptid nach Anspruch 8, dadurch gekennzeichnet, daß es das Peptidfragment mit der Sequenz 20—40 von GRF ist, das durch aufeinanderfolgende Kopplung in flüssiger Phase der folgenden Subfragmente in der Reihenfolge der Sequenz von GRF erhalten wird:

H-Ala-$NH_2$,
Boc-Glu(OBzl)-Arg-Gly-OH (37—39), genannt Fragment $B_1$,
Boc-Glu(OBzl)-Ser-Asn-Gln-OH (33—36), genannt Fragment $B_2$,
Boc-Ser-Arg-Gln-Gln-Gly-OH (28—32), genannt Fragment C,
Boc-Asp(OBzl)-Ile-Met-NH-$NH_2$ (25—27), genannt Fragment D,
Boc-Gln-ONp,
Boc-Arg-Lys(Z)-Leu-Leu-OH (20—23), genannt Fragment $E_1$, worin:

a) die seitlichen Säurefunktionen der Asparagin- und Glutaminsäure und die seitliche Aminfunktion von Lysin durch Schutzgruppen geschützt sind, die unter Entschützungsbedingungen für die Gruppierung Boc stabil sind,

b) die Guanidinfunktion von Arginin durch Protonierung geschützt ist, und

c) die N-terminale Aminosäure der Fragmente $B_1$, $B_2$, C, D, E an der Aminogruppe durch die Gruppierung Boc geschützt ist, die selektiv in der Verlängerungsphase des Peptids durch Hydrolyse mit Trifluoressigsäure entfernbar ist, wobei die Kopplungen in einem aprotischen polaren Lösungsmittel vorgenommen werden.

12. Intermediäres Peptid nach Anspruch 9, dadurch gekennzeichnet, daß es das Peptid J ist, das durch aufeinanderfolgende Kopplung in flüssiger Phase der folgenden Subfragmente in der Reihenfolge der Sequenz von GRF erhalten wird:

H-Gln-Leu-Ser-Ala-$OCH_3$ (16—19), genannt Fragment $F_1$,
Boc-Tyr-Arg-Lys(Z)-Val-Leu-Gly-OH (10—15), genannt Fragment $G_1$,
Boc-Ile-Phe-Thr-Asn-Ser-NH-$NH_2$ (5—9), genannt Fragment $H_1$, worin:

a) die seitliche Aminfunktion von Lysin des Fragments G durch eine Schutzgruppe geschützt ist, die unter Entschützungsbedingungen für die Gruppierung Boc stabil ist,

b) die Guanidinfunktion des Fragments 10—15 durch Protonierung geschützt ist,

c) die N-terminale Aminosäure der Fragmente G und H an der Aminogruppe durch die Gruppierung Boc geschützt ist, die selektiv in der Verlängerungsphase des Peptids durch Hydrolyse mit Trifluoressigsäure entfernbar ist, wobei die Kopplungen in einem aprotischen polaren Lösungsmittel vorgenommen werden.

13. Intermediäres Peptid nach Anspruch 9, dadurch gekennzeichnet, daß es das Peptid I ist, das durch Synthese in flüssiger Phase erhalten wird, worin:

a) die seitliche Säurefunktion der Asparaginsäure durch eine Schutzgruppe geschützt ist, die unter Entschützungsbedingungen für die Gruppierung Boc stabil ist,

b) die N-terminale alpha-Aminosäure an der Aminogruppe durch eine Gruppierung geschützt ist, die unter Entschützungsbedingungen für die Schutzgruppe der seitlichen Ketten abspaltbar ist.

**Patentansprüche für den Vertragsstaat: AT**

1. Syntheseverfahren für hpGRF 1—44 und hpGRF 1—40 in flüssiger Phase und mittels Fragmenten, dadurch gekennzeichnet, daß man nacheinander und in der Reihenfolge der Sequenz von GRF die Fragmente koppelt, in denen:

a) die seitlichen Säurefunktionen der Asparagin- und Glutaminsäure und die seitliche Aminfunktion von Lysin durch Schutzgruppen geschützt sind, die unter Entschützungsbedingungen für die Gruppierung Boc stabil sind,

b) die Guanidinfunktion von Arginin durch Protonierung geschützt ist, und

c) die N-terminale Aminosäure an der Aminogruppe durch die Gruppierung Boc geschützt ist, daß man selektiv die Gruppierung Boc der N-terminalen Aminogruppe des Peptids in der Verlängerungsphase durch Hydrolyse mit Trifluoressigsäure entfernt, wobei die Kopplung in einem aprotischen polaren Lösungsmittel vorgenommen wird, und daß man am Ende der Sequenz sämtliche Schutzgruppen durch Hydrolyse mit Hilfe einer 0,1 bis 1 M Methansulfon- oder Trifluormethansulfonsäurelösung in Trifluoressigsäure entfernt.

2. Verfahren nach Anspruch 1, dadurch gekennzeichnet, daß nach jeder Kopplung das erhaltene Produkt durch Ausfällen mit Hilfe eines apolaren Lösungsmittels aus dem Reaktionsmilieu isoliert wird.

3. Verfahren nach Anspruch 1, dadurch gekennzeichnet, daß das Reaktions-Rohprodukt durch Gegenstromverteilung und Gelpermeationschromatographie gereinigt wird.

4. Verfahren nach einem der Ansprüche 1 bis 3, dadurch gekennzeichnet, daß es aus der aufeinanderfolgenden Kopplung in der Reihenfolge der Sequenz von GRF folgender neun Peptidfragemente besteht:

H-Ala-Arg-Ala-Arg-Leu-$NH_2$ (40—44), genannt Fragment A,
Boc-Glu(OBzl)-Ser-Asn-Gln-Glu(OBzl)-Arg-Gly-OH (33—39), genannt Fragment B,
Boc-Ser-Arg-Gln-Gln-Gly-OH (28—32), genannt Fragment C,
Boc-Asp(OBzl)-Ile-Met-NH-$NH_2$ (25—27), genannt Fragment D,
Boc-Lys(Z)-Leu-Leu-Gln-OH (21—24), genannt Fragment E,
Boc-Gln-Leu-Ser-Ala-Arg-OH (16—20), genannt Fragment F,
Boc-Lys(Z)-Val-Leu-Gly-OH (12—15), genannt Fragment G,
Boc-Ile-Phe-Thr-Asn-Ser-Tyr-Arg-OH (5—11), genannt Fragment H,
Z-Tyr-Ala-Asp(OBzl)-Ala-OH (1—4), genannt Fragment I,

und daß das erhaltene Peptid hpGRF 1—44 ist.

5. Verfahren nach einem der Ansprüche 1 bis 4, dadurch gekennzeichnet, daß man das Alaninamid anstelle des Fragments A einsetzt und daß das erhaltene Peptid hpGRF 1—40 ist.

6. Verfahren nach einem der Ansprüche 1 bis 3, dadurch gekennzeichnet, daß man nacheinander und in der Reihenfolge der Sequenz von GRF die 3 Peptidfragmente koppelt:

H-Arg-Lys(Z)-Leu-Leu-Gln-Asp(OBz)-Ile-Met-Ser-Arg-Gln-Gln-Gly-Glu(OBzl)-Ser-Asn-Gln-Glu(OBzl)-Arg-Gly-Ala-Arg-Ala-Arg-Leu-$NH_2$ (Sequenz 20—44 von GRF, genannt Peptid K),

Boc-Ile-Phe-Thr-Asn-Ser-Tyr-Arg-Lys(Z)-Val-Leu-Gly-Gln-Leu-Ser-Ala-OH (Sequenz 5—19 von GRF, genannt Peptid J),

Z-Tyr-Ala-Asp(OBzl)-Ala-OH (Sequenz 1—4 von GRF, genannt Peptid I), wobei das erhaltene Peptid hpGRF 1—44 ist.

7. Verfahren nach Anspruch 6, dadurch gekennzeichnet, daß man anstelle des Peptids K das Peptidfragment einsetzt, das der Sequenz 20—40 von GRF entspricht, wobei das erhaltene Peptid hpGRF 1—40 ist.

8. Verfahren nach einem der Ansprüche 1 bis 7, dadurch gekennzeichnet, daß die Kopplung mit Hilfe von Benzotriazolyloxyphosphonium-hexafluorphosphat (BOP) oder Dicyclohexyl-carbodiimid in Gegenwart von 1-Hydroxybenzotriazol (Kopplungsmittel) oder durch Aktivierung mit Carboxyaziden in einem geeigneten Lösungsmittel, wie Dimethylformamid oder Dimethylsulfoxid durchgeführt wird.

9. Syntheseverfahren für das intermediäre Peptid K, dessen Sequenz der Sequenz 20—44 von GRF entspricht und von dem bestimmte Aminosäuren blockiert sind, dadurch gekennzeichnet, daß es in der aufeinanderfolgenden Kopplung in flüssiger Phase der nachstehenden Subfragmente in der Reihenfolge der Sequenz von GRF besteht:

H-Ala-Arg-Ala-Arg-Leu-$NH_2$ (40—44), genannt Fragment A,
Boc-Glu(OBzl)-Arg-Gly-OH (37—39), genannt Fragment $B_1$,
Boc-Glu(OBzl)-Ser-Asn-Gln-OH (33—36), genannt Fragment $B_2$,
Boc-Ser-Arg-Gln-Gln-Gly-OH (28—32), genannt Fragment C,
Boc-Asp(OBzl)-Ile-Met-NH-$NH_2$ (25—27), genannt Fragment D,
Boc-Gln-ONp,
Boc-Arg-Lys(Z)-Leu-Leu-OH (20—23), genannt Fragment $E_1$, worin:

a) die seitlichen Säurefunktionen der Asparagin- und Glutaminsäure und die seitliche Aminfunktion von Lysin durch Schutzgruppen geschützt sind, die unter Entschützungsbedingungen für die Gruppierung Boc stabil sind,

b) die Guanidinfunktion von Arginin durch Protonierung geschützt ist, und

c) die N-terminale Aminosäure der Fragmente $B_1$, $B_2$, C, D, $E_1$ an der Aminogruppe durch die Gruppierung Boc geschützt ist, die selektiv in der Verlängerungsphase des Peptids durch Hydrolyse mit Trifluoressigsäure entfernbar ist, wobei die Kopplungen in einem aprotischen polaren Lösungsmittel vorgenommen werden.

10. Syntheseverfahren für das intermediäre Peptid mit der Sequenz 20—40 von GRF, dadurch gekennzeichnet, daß es in der aufeinanderfolgenden Kopplung in flüssiger Phase der folgenden Subfragmente in der Reihenfolge der Sequenz von GRF besteht:

H-Ala-$NH_2$,
Boc-Glu(OBzl)-Arg-Gly-OH (37—39), genannt Fragment $B_1$,
Boc-Glu(OBzl)-Ser-Asn-Gln-OH (33—36), genannt Fragment $B_2$,

Boc-Ser-Arg-Gln-Gln-Gly-OH (28—32), genannt Fragment C,
Boc-Asp(OBzl)-Ile-Met-NH-$NH_2$ (25—27), genannt Fragment D,
Boc-Gln-ONp,
Boc-Arg-Lys(Z)-Leu-Leu-OH (20—23), genannt Fragment $E_1$, worin:

a) die seitlichen Säurefunktionen der Asparagin- und Glutaminsäure und die seitliche Aminfunktion von Lysin durch Schutzgruppen geschützt sind, die unter Entschützungsbedingungen für die Gruppierung Boc stabil sind,

b) die Guanidinfunktion von Arginin durch Protonierung geschützt ist, und

c) die N-terminale Aminosäure der Fragmente $B_1$, $B_2$, C, D, E an der Aminogruppe durch die Gruppierung Boc geschützt ist, die selektiv in der Verlängerungsphase des Peptids durch Hydrolyse mit Trifluoressigsäure entfernbar ist, wobei die Kopplungen in einem aprotischen polaren Lösungsmittel vorgenommen werden.

11. Syntheseverfahren für das intermediäre Peptid J, dadurch gekennzeichnet, daß es in der aufeinanderfolgenden Kopplung in flüssiger Phase der folgenden Subfragmente in der Reihenfolge der Sequenz von GRF besteht:

H-Gln-Leu-Ser-Ala-$OCH_3$ (16—19), genannt Fragment $F_1$,
Boc-Tyr-Arg-Lys(Z)-Val-Leu-Gly-OH (10—15), genannt Fragment $G_1$,
Boc-Ile-Phe-Thr-Asn-Ser-NH-$NH_2$ (5—9), genannt Fragment $H_1$, worin:

a) die seitliche Aminfunktion von Lysin des Fragments G durch eine Schutzgruppe geschützt ist, die unter Entschützungsbedingungen für die Gruppierung Boc stabil ist,

b) die Guanidinfunktion des Fragments 10—15 durch Protonierung geschützt ist,

c) die N-terminale Aminosäure der Fragmente G und H an der Aminogruppe durch die Gruppierung Boc geschützt ist, die selektiv in der Verlängerungsphase des Peptids durch Hydrolyse mit Trifluoressigsäure entfernbar ist, wobei die Kopplungen in einem aprotischen polaren LÖsungsmittel vorgenommen werden.

12. Syntheseverfahren für das intermediäre Peptid I, dadurch gekennzeichnet, daß es in der Kopplung in flüssiger Phase der dieses bildenden Aminosäuren besteht, worin:

a) die seitliche Säurefunktion der Asparaginsäure durch eine Schutzgruppe geschützt ist, die unter Entschützungsbedingungen für die Gruppierung Boc stabil ist,

b) die N-terminale alpha-Aminosäure an der Aminogruppe durch eine Gruppierung geschützt ist, die unter Entschützungsbedingungen für die Schutzgruppen der seitlichen Ketten abspaltbar ist.

**Claims for the Contracting States: BE, CH, DE, FR, GB, IT, LI, LU, NL, SE**

1. A process for the synthesis, in liquid phase and by fragments, of hpGRF 1—44 and hpGRF 1—40, characterized in that it comprises the steps of:

— coupling, one after the other and in the order of the sequence of the GRF, the fragments in which:

a) the side acid functions of the aspartic and glutamic acids and the side amine function of the lysine are protected by protector groups stable in the conditions of deprotection of the group Boc,

b) the guanidine function of the arginine is protected by protonation, and

c) the N-terminal amino acid is protected on the amine by the Boc group;

— selectively eliminating the group Boc from the N-terminal amine of the peptide in phase of elongation by hydrolysis with trifluoroacetic acid, said coupling being effected in an aprotic polar solvent, and
— eliminating, at the end of sequence, all the protector groups by hydrolysis with the aid of a 0.1 to 1 M solution of methanesulfonic or trifluoromethanesulfonic acid in trifluoroacetic acid.

2. The process according to claim 1, characterized in that, after each coupling, the product obtained is isolated from the reaction medium by precipitation with the aid of an apolar solvent.

3. The process according to claim 1, characterized in that the crude product of reaction is purified by counter-current distribution and permeation chromatography over gel.

4. The process according to any one of claims 1 to 3, characterized in that it consists in coupling, successively and in the order of the sequence of the GRF, the nine peptide fragments hereinafter:

H-Ala-Arg-Ala-Arg-Leu-$NH_2$ (40—44) called fragment A,
Boc-Glu(OBzl)-Ser-Asn-Gln-Glu(OBzl)-Arg-Gly-OH (33—39) called fragment B,
Boc-Ser-Arg-Gln-Gln-Gly-OH (28—32) called fragment C,
Boc-Asp(OBzl)-Ile-Met-NH-$NH_2$ (25—27) called fragment D,
Boc-Lys(Z)-Leu-Leu-Gln-OH (21—24) called fragment E,
Boc-Gln-Leu-Ser-Ala-Arg-OH (16—20) called fragment F,
Boc-Lys(Z)-Val-Leu-Gly-OH (12—15) called fragment G,
Boc-Ile-Phe-Thr-Asn-Ser-Tyr-Arg-OH (5—11) called fragment H,
Z-Tyr-Ala-Asp(OBzl)-Ala-OH (1—4) called fragment I

and the peptide obtained is hpGRF 1—44.

5. The process according to any one of claims 1 to 4, characterized in that alaninamide is used in place of fragment A and the peptide obtained is hpGRF 1—40.

6. The process according to any one of claims 1 to 3, characterized in that the following three peptide fragments are coupled successively and in the order of the sequence of the GRF:

H-Arg-Lys(Z)-Leu-Leu-Gln-Asp(OBzl)-Ile-Met-Ser-Arg-Gln-Gln-Gly-Glu(OBzl)-Ser-Asn-Gln-Glu(OBzl)-Arg-Gly-Ala-Arg-Ala-Arg-Leu-$NH_2$ (sequence 20—44 of the GRF, called peptide K);

Boc-Ile-Phe-Thr-Asn-Ser-Tyr-Arg-Lys(Z)-Val-Leu-Gly-Gln-Leu-Ser-Ala-OH (sequence 5—19 of the GRF called peptide J);

Z-Tyr-Ala-Asp(OBzl)-Ala-OH (sequence 1—4 of the GRF called peptide I); said peptide obtained being hpGRF 1—44.

7. The process according to claim 6, characterized in that the peptide fragment corresponding to sequence 20—40 of the GRF is used in place of the peptide K, said peptide obtained being hpGRF 1—40.

8. The process according to any one of claims 1 to 7, characterized in that the coupling is carried out with the aid of hexafluorophosphate of benzotriazolyloxyphosphonium (BOP) or dicyclohexylcarbodiimide in the presence of 1-hydroxy benzotriazole (coupling agent) or by activation with carboxyazides, in an appropriate solvent such as dimethylformamide or dimethylsulfoxide.

9. By way of novel products, the intermediate peptides or peptide fragments A to K used in the process according to any one of claims 1 to 8.

10. The intermediate peptide according to claim 9, characterized in that it is the peptide K, the sequence of which corresponds to the sequence 20—44 of the GRF and certain amino-acids of which are blocked, obtained by successive coupling, in liquid phase, of the following sub-fragments in the order of the sequence of the GRF:

H-Ala-Arg-Ala-Arg-Leu-$NH_2$ (40—44) called fragment A,
Boc-Glu(OBzl)-Arg-Gly-OH (37—39) called fragment $B_1$,
Boc-Glu(OBzl)-Ser-Asn-Gln-OH (33—36) called fragment $B_2$,
Boc-Ser-Arg-Gln-Gln-Gly-OH (23—32) called fragment C,
Boc-Asp(OBzl)-Ile-Met-NH-$NH_2$ (25—27) called fragment D,
Boc-Gln-ONp,
Boc-Arg-Lys(Z)-Leu-Leu-OH (20—23) called Fragment $E_1$ in which:

a) the side acid functions of the aspartic and glutamic acids and the side amine function of the lysine are protected by protector groups stable in the conditions of deprotection of the group Boc;

b) the guanidine function of the arginine is protected by protonation; and

c) the N-terminal amino acid of the fragments $B_1$, $B_2$, C, D, $E_1$ is protected on the amine by the group Boc which may be eliminated selectively in phase of elongation of the peptide by hydrolysis with the aid of trifluoroacetic acid, the couplings being effected in an aprotic polar solvent.

11. The intermediate peptide according to claim 8, characterized in that it is the peptide fragment having the sequence 20—40 of the GRF, obtained by successive coupling in liquid phase of the following sub-fragments in the order of the sequence of the GRF:

H-Ala-$NH_2$,
Boc-Glu(OBzl)-Arg-Gly-OH (37—39) called fragment $B_1$,
Boc-Glu(OBzl)-Ser-Asn-Gln-OH (33—36) called fragment $B_2$,
Boc-Ser-Arg-Gln-Gln-Gly-OH (28—32) called fragment C,
Boc-Asp(OBzl)-Ile-Met-NH-$NH_2$ (25—27) called fragment D,
Boc-Gln-ONp,
Boc-Arg-Lys(Z)-Leu-Leu-OH (20—23) called fragment $E_1$ in which:

a) the side acid function of the aspartic and glutamic acids and the side amine function of the lysine are protected by protector groups stable in the conditions of deprotection of the group Boc;

b) the guanidine function of the arginine is protected by protonation; and

c) the N-terminal amino acid of fragments $B_1$, $B_2$, C, D, E is protected on the amine by the group Boc which may be eliminated selectively in phase of elongation of the peptide by hydrolysis with trifluoroacetic acid, the couplings being effected in an aprotic polar solvent.

12. The intermediate peptide according to claim 9, characterized in that it is peptide J obtained by successive coupling in liquid phase of the following sub-fragments in the order of the sequence of the GRF,

H-Gln-Leu-Ser-Ala-$OCH_3$ (15—19) called fragment $F_1$,
Boc-Tyr-Arg-Lys(Z)-Val-Leu-Gly-OH (10—15) called fragment $G_1$,
Boc-Ile-Phe-Thr-Asn-Ser-NH-$NH_2$ (5—9) called fragment $H_1$ in which:

a) the side amine fraction of the lysine of fragment G is protected by a protector group stable in the conditions of deprotection of the group Boc;

b) the guanidine function of the fragment 10—15 is protected by protonation;

c) the N-terminal amino acid of fragments G and H is protected on the amine by the group Boc, which may be eliminated selectively in phase of elongation of the peptide by hydrolysis with trifluoroacetic acid, the couplings being effected in an aprotic polar solvent.

13. The intermediate peptide according to claim 9, characterized in that it is the peptide I obtained by synthesis in liquid phase in which:

a) the side acid function of the aspartic acid is protected by a protector group stable in the conditions of deprotection of the group Boc;

b) the N-terminal alpha amino acid is protected on the amine by a group cleavable in the conditions of deprotection of the protector groups of the side chains.

**Claims for the Contracting State: AT**

1. A process for the synthesis, in liquid phase and by fragments, of hpGRF 1—44 and hpGRF 1—40, characterized in that it comprises the steps of:

— coupling, one after the other and in the order of the sequence of the GRF, the fragments in which:

a) the side acid functions of the aspartic and glutamic acids and the side amine function of the lysine are protected by protector groups stable in the conditions of deprotection of the group Boc,
b) the guanidine function of the arginine is protected by protonation, and
c) the N-terminal amino acid is protected on the amine by the Boc group;

— selectively eliminating the group Boc from the N-terminal amine of the peptide in phase of elongation by hydrolysis with trifluoroacetic acid, said coupling being effected in an aprotic polar solvent and
— eliminating, at the end of sequence, all the protector groups by hydrolysis with the aid of a 0.1 to 1 M solution of methanesulfonic or trifluoromethanesulfonic acid in trifluoroacetic acid.

2. The process according to claim 1, characterized in that, after each coupling, the product obtained is isolated from the reaction medium by precipitation with the aid of an apolar solvent.

3. The process according to claim 1, characterized in that the crude product of reaction is purified by counter-current distribution and permeation chromatography over gel.

4. The process according to any one of claims 1 to 3, characterized in that it consists in coupling, successively and in the order of the sequence of the GRF, the nine peptide fragments hereinafter:
H-Ala-Arg-Ala-Arg-Leu-$NH_2$ (40—44) called fragment A,
Boc-Glu(OBzl)-Ser-Asn-Gln-Glu(OBzl)-Arg-Gly-OH (33—39) called fragment B,
Boc-Ser-Arg-Gln-Gln-Gly-OH (28—32) called fragment C,
Boc-Asp(OBzl)-Ile-Met-NH-$NH_2$ (25—27) called fragment D,
Boc-Lys(Z)-Leu-Leu-Gln-OH (21—24) called fragment E,
Boc-Gln-Leu-Ser-Ala-Arg-OH (16—20) called fragment F,
Boc-Lys(Z)-Val-Leu-Gly-OH (12—15) called fragment G,
Boc-Ile-Phe-Thr-Asn-Ser-Tyr-Arg-OH (5—11) called fragment H,
Z-Tyr-Ala-Asp(OBzl)-Ala-OH (1—4) called fragment I and the peptide obtained is hpGRF 1—44.

5. The process according to any one of claims 1 to 4, characterized in that alaninamide is used in place of fragment A and the peptide obtained is hpGRF 1—40.

6. The process according to any one of claims 1 to 3, characterized in that the following three peptide fragments are coupled successively and in the order of the sequence of the GRF:
H-Arg-Lys(Z)-Leu-Leu-Gln-Asp(OBzl)-Ile-Met-Ser-Arg-Gln-Gln-Gly-Glu(OBzl)-Ser-Asn-Gln-Glu(OBzl)-Arg-Gly-Ala-Arg-Ala-Arg-Leu-$NH_2$ (sequence 20—44 of the GRF, called peptide K);
Boc-Ile-Phe-Thr-Asn-Ser-Tyr-Arg-Lys(Z)-Val-Leu-Gly-Gln-Leu-Ser-Ala-OH (sequence 5—19 of the GRF called peptide J);
Z-Tyr-Ala-Asp(OBzl)-Ala-OH (sequence 1—4 of the GRF called peptide I); said peptide obtained being hpGRF 1—44.

7. The process according to claim 6, characterized in that the peptide fragment corresponding to sequence 20—40 of the GRF is used in place of the peptide K, said peptide obtained being hpGRF 1—40.

8. The process according to any one of claims 1—7, characterized in that the coupling is carried out with the aid of hexafluorophosphate or benzotriazolyloxyphosphonium (BOP) or dicyclohexylcarbodiimide in the presence of 1-hydroxy benzotriazole (coupling agent) or by activation with carboxyazides, in an appropriate solvent such as dimethylformamide or dimethylsulfoxide.

9. A process for the synthesis of the intermediate peptide K of which the sequence corresponds to the sequence 20—44 of the GRF and of which certain amino acids are blocked, characterized in that it consists in: successively coupling, in liquid phase, the following sub-fragments in the order of the sequence of the GRF:
H-Ala-Arg-Ala-Arg-Leu-$NH_2$ (40—44) called fragment A,
Boc-Glu(OBzl)-Arg-Gly-OH (37—39) called fragment $B_1$,
Boc-Glu(OBzl)-Ser-Asn-Gln-OH (33—36) called fragment $B_2$,
Boc-Ser-Arg-Gln-Gln-Gly-OH (28—32) called fragment C,
Boc-Asp(OBzl)-Ile-Met-NH-$NH_2$ (25—27) called fragment D,
Boc-Gln-ONp,
Boc-Arg-Lys(Z)-Leu-Leu-OH (20—23) called fragment $E_1$ in which:

a) the side acid functions of the aspartic and glutamic acids and the side amine function of the lysine are protected by protector groups stable in the conditions of deprotection of the group Boc;

b) the guanidine function of the arginine is protected by protonation, and

c) the N-terminal amino acid of the fragment $B_1$, $B_2$, C, D, $E_1$ is protected on the amine by the group Boc which may be eliminated selectively in phase of elongation of the peptide by hydrolysis with the aid of trifluoroacetic acid, the couplings being effected in aprotic polar solvent.

10. A process for synthesis of the intermediate peptide having the sequence 20—40 of the GRF, characterized in that it consists in successively coupling in liquid phase, the following sub-fragments in the order of the sequence of the GRF:

H-Ala-$NH_2$,

Boc-Glu(OBzl)-Arg-Gly-OH (37—39) called fragment $B_1$,

Boc-Glu(OBzl)-Ser-Asn-Gln-OH (33—36) called fragment $B_2$,

Boc-Ser-Arg-Gln-Gln-Gly-OH (28—32) called fragment C,

Boc-Asp(OBzl)-Ile-Met-NH-$NH_2$ (25—27) called fragment D,

Boc-Gln-ONp,

Boc-Arg-Lys(Z)-Leu-Leu-OH (20—23) called fragment $E_1$ in which:

a) the side acid functions of the aspartic and glutamic acids and the side amine function of the lysine are protected by protector groups stable in the conditions of deprotection of the group Boc;

b) the guanidine function of the arginine is protected by protonation; and

c) the N-terminal amino acid of fragments $B_1$, $B_2$, C, D, E is protected on the amine by the group Boc which may be eliminated selectively in phase of elongation of the peptide by hydrolysis with trifluoroacetic acid, the couplings being effected in an aprotic polar solvent.

11. A process for synthesis of the intermediate peptide J, characterized in that it consists in successively coupling in liquid phase the following sub-fragments in the order of the sequence of the GRF,

H-Gln-Leu-Ser-Ala-$OCH_3$ (16—19) called fragment $F_1$,

Boc-Tyr-Arg-Lys(Z)-Val-Leu-Gly-OH (10—15) called fragment $G_1$,

Boc-Ile-Phe-Thr-Asn-Ser-NH-$NH_2$ (5—9) called fragment $H_1$ in which:

a) the side amine fraction of the lysine of fragment G is protected by a protector group stable in the conditions of deprotection of the group Boc;

b) the guanidine function of the fragment 10—15 is protected by protonation;

c) the N-terminal amino acid of fragments G and H is protected on the amine by the group Boc, which may be eliminated selectively in phase of elongation of the peptide by hydrolysis with trifluoroacetic acid, the couplings being effected in an aprotic polar solvent.

12. Process for synthesis of the intermediate peptide I, characterized in that it consists in coupling in liquid phase the amino acids constituting same, in which:

a) the side acid function of the aspartic acids is protected by a protector group stable in the conditions of deprotection of the group Boc;

b) the end terminal alpha amino acid is protected on the amine by a group cleavable in the conditions of deprotection of the protector groups of the side chains.